(19)

**Europäisches Patentamt**
**European Patent Office**
**Office européen des brevets**

(11) **EP 3 511 033 A1**

(12) **EUROPEAN PATENT APPLICATION**
published in accordance with Art. 153(4) EPC

(43) Date of publication:
**17.07.2019 Bulletin 2019/29**

(21) Application number: **17848894.6**

(22) Date of filing: **08.09.2017**

(51) Int Cl.:
*A61M 1/14* (2006.01)    *A61M 1/16* (2006.01)
*A61M 1/36* (2006.01)

(86) International application number:
**PCT/JP2017/032551**

(87) International publication number:
**WO 2018/047956 (15.03.2018 Gazette 2018/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **08.09.2016  JP 2016175295**

(71) Applicant: **Kabushiki Kaisya Advance
Tokyo 103-8354 (JP)**

(72) Inventor: **URAKABE, Nobuchika
Tokyo 103-8354 (JP)**

(74) Representative: **Santarelli
49, avenue des Champs-Elysées
75008 Paris (FR)**

(54) **SYSTEM FOR MANAGING INFORMATION RELATING TO DIFFERENCES BETWEEN INDIVIDUALS IN DIALYSIS TREATMENT**

(57)    To provide an optimized hemodialysis system such as capable of suppressing variations in a blood pressure according to the individual difference of a patient.

The system is configured to include: blood related information acquiring means configured to acquire blood flow information and patient biological information in a blood circuit for hemodialysis, the patient biological information being peripheral vascular resistance informa-

tion, blood pressure information, face information, body movement information, temperature information, and humidity information and the like; individual difference information managing means configured to acquire and manage patient individual difference information from the blood related information; and optimized dialysis means configured to perform optimized dialysis treatment for the patients on the basis of the patient individual difference information.

FIG. 1

EP 3 511 033 A1

**Description**

FIELD

The present invention relates to an individual difference information management system in dialysis treatment.

BACKGROUND

Hemodialysis treatment involves forming a dialyzer blood circuit outside the body, taking out blood out of the body, and circulating the blood while filtering, dehydrating and the like. This procedure is performed from 2 to 4 times a week for about 4 hours each time. Therefore, the burden on patients is significant, and hemodialysis has various risks for the patients. For example, dialysis patients are likely to develop arteriosclerosis, myocardial infarction, stroke, gangrene of distal portion of the extremities such as foot lesions, etc. due to the burden applied on the heart by hemodialysis. In addition, dialysis patients are more likely to be suffered from other diseases such as bone disorders, amyloidosis, infections, heart failure, malignant tumors, shunt disorders, curative peritonitis and the like. These risks add further burden and pain to patients.

As a cause of these risks, for example, when dehydration is carried out constantly as in the case of even dehydration, the blood pressure may suddenly be lowered due to due to an imbalance of a dehydration speed and a PRR (plasma refilling rate) in the body. In addition, the blood pressure may also be lowered due to excessive dosage of antihypertensive agent, advanced arteriosclerosis in chronic renal failure patients, an excessive amount of salt, or the like.

Although countermeasures are indicated for all these risks, it is not necessarily a gentle dialysis treatment depending on the individual differences of patients. For example, JP 2008-23269 A describes a configuration that measures blood flowing through a circuit with an ultrasonic wave type blood flow meter for detecting a blood removal failure during hemodialysis and regulates a dialysis duration.

JP 2004-357784 A discloses a configuration in which blood flows in the head and extremities are individually measured using a laser blood flow meter, and a pattern of blood flow value from 30 minutes to 1 hour after the start of dialysis and a pattern of degree of development of a blood flow waveform are recorded in advance as reference values, and based on a decreased amount of the blood flow value, a decreased amount of the degree of development of the blood flow waveform, and an increase in heart rate that can be derived from the reference values, serious situation is monitored.

JP 2012-526569 A discloses a configuration in which optimization of an extracorporeal blood circuit for hemodialysis is achieved by setting treatment parameters preset by a doctor as patient specific or machine specific treatment parameters and, from this treatment parameters, calculating a consumable supply cost and an energy cost in the hemodialysis, and processing information which helps for selection of a dialysis machine and a filter by a computer.

JP 2014-532475 A discloses a micro blood flow sensor, which can be mounted on a dialysis probe, provided with a microelectrode, and used in contact with blood, and processing sensor information by a computer. Also, JP 2015-29882 A discloses a configuration in which blood concentration sensors are disposed in an arterial blood circuit 21 and a venous blood circuit 2, respectively, and a blood flow amount is calculated from a blood concentration obtained through the sensor.

In addition, JP 2005-334527 A discloses a home medical apparatus in which an in-home dialysis apparatus is disposed in a house of each patient at home. Furthermore, JP 2003-190277 A discloses a dialysis apparatus provided with measuring means which can measure a blood velocity at any place in a dialysis machine using an ultrasonic wave.

[CITATION LIST]

[PATENT LITERATURE]

[PTL 1] JP 2008-23269 A
[PTL 2] JP 2004-357784 A
[PTL 3] JP 2012-526569 A
[PTL 4] JP 2014-532475 A
[PTL 5] JP 2015-29882 A
[PTL 6] JP 2005-334527 A
[PTL 7] JP 2003-190277 A

[NON-PATENT LITERATURE]

[NPL 1] Yu Dialysis Clinic- Material of 7th Study Session of Patients: (http://yutoseki.jp/swfu/d/study7.pdf)
[NPL 2] Annual Meeting of Japanese Society for Dialysis Therapy: Archived Abstracts of the General Assembly, Suguru Obunai and Satoaki Matoba, A case of finger gangrene that could be rescued by multidisciplinary treatment: Subject Number, P-1-181: (http:// /www.myschedule.jp/jsdt_archive/detail.php?sess_id=17048)
[NPL 3] Kei Eguchi et.al, Invention of a New Actual Blood Flow Amount Measurement Method (CRIT-2 point method), Journal of Japanese Society for Dialysis Therapy 41: 127 to 131, 2008.
[NPL 4] Kazuyuki Suzuki et al., Dialysis Conditions/Dialysis Volume and Life Prognosis, Journal of Japanese Society for Dialysis Therapy 45 (2): 143 to 155, 2012
[NPL 5] Kazuyuki Suzuki et al., Hemodialysis Condition /Dialysis Volume and Life Prognosis, Journal of Japanese Society for Dialysis Therapy 43 (7): 551 to 559, 2010
[NPL 6] Susumu Ookawara et al., Mathematical Analysis of the Change in Circulating Blood Volume during Hemodialysis by Continuous Hematocrit Measurement, Journal of Japanese Society for Dialysis Therapy 31 (6): 1001 to 1005, 1998

SUMMARY

[TECHNICAL PROBLEM]

As mentioned above, hemodialysis has uniform specifications and procedures. During even dehydration treatment, etc., in some cases, the blood pressure decreases several times during one dialysis treatment. Therefore, enough dialysis may not be performed because the patient does not feel well, and there are many risks of complications related to the heart and blood vessels, etc. Also, since the burden on the patients is large such as being subjected to a repeated blood pressure change during a certain dialysis treatment performed two or three times a week for four hours each time, it is hard to say that the dialysis is always performed in accordance with individual differences even though the hemodialysis plays a role of an artificial organ which performs the function of the kidney.

[SOLUTION TO PROBLEM]

In view of such circumstances, the present invention proposes an individual difference information management system that suppresses sudden variations in blood pressure and can perform so-called customized hemodialysis which is optimized based on individual differences while considering the patients' homeostasis in dialysis treatment.

That is, according to the present invention, with the provision of an information management system including: blood related information acquiring means for acquiring a combination of information including all or part of blood flow information, peripheral vascular resistance information, blood pressure information, face information of a patient, temperature information, and humidity information within a blood circuit for hemodialysis; change amount acquiring means for acquiring a change amount of the information acquired by the blood related information acquiring means; individual difference information managing means for acquiring and managing the patient's individual difference information from the blood related information and/or the change amount; and optimized dialysis means for performing optimized dialysis treatment for the patients on the basis of the patient individual difference information, variations in blood pressure is suppressed, and gentle dialysis treatment is achieved, extremities gangrene in association with the reduction of risks of the heart complications is prevented, and the life prognosis is improved, and gentle dialysis treatment is achieved.

The present invention makes it possible to improve dialysis efficiency and stably perform gentle dialysis at home in customized hemodialysis treatment.

Here, the "change amount" of the information in the present invention includes not only values which indicates, for example, the change amount of the blood pressure value, at a predetermined time interval, but also values of time during which predetermined changes occur.

Also, the "peripheral vascular resistance information" or the "peripheral vascular resistance related value" indicates, for example, a ratio of values of an ejection wave and a reflected wave obtained from a volumetric pulse wave signal, an interval value between the ejection wave and the reflected wave, and the like, and also indicates a peripheral vascular value such as a ratio referred to as Augmentation Index (AIx), as well as a variable part of a calculating formula for obtaining a peripheral vascular resistance.

The blood circuit for hemodialysis in the present invention indicates a part for circulating blood outside the biological body, and during the circulation, performs dialysis via a dialysis machine (dialyzer) configured to discharge waste matter or the like to the outside through a permselective film of a hollow fiber type and perform dehydration and a dialysate circulating device. In addition, the blood circuit for hemodialysis may use a dialysis system, which is normally used and configured to be additionally provided with a configuration for performing deaeration and filtration of an arteria chamber, a vein chamber, and the like. The blood circuit for hemodialysis of the present invention may also be provided with a configuration of hemodiafiltration (HDF) for forcedly filtering waste matter by applying a filtration pressure.

The blood related information acquiring means of the present invention includes, for example, components such as the dialysis machine and other components which form a blood circuit such as conduits, and has a configuration in which one or a plurality of blood flow detection sensors(if plural, mounted at different positions (four positions in total including a blood incoming side, a blood outgoing side, and two positions near a center, or a plurality of positions) are mounted on surfaces of positions where the blood flow can be detected from the outside for detecting change amount information of the blood flow in the hollow fiber.

Examples of the apparatus for detecting the change amount information include, for example, a non-contact type sensor which does not come into direct contact with bodily fluid such as a sensor for an ultrasonic blood flow meter and a laser blood flow meter sensors. However, a contact-type electrode sensor may also be applicable depending on the case. Further, means for monitoring a hematocrit value based on a light absorbency for near infrared light may be provided.

A hematocrit value monitor uses, for example, near infrared spectroscopy, and uses a combination of a light emitting diode and a light receiving semiconductor element as a sensor. The light emitting diode is configured to output near infrared light and the light receiving semiconductor element is configured to receive reflected return light, which is light emitted from the light emitting diode and reflected from blood tissues. The hematocrit value monitor is mounted on a surface of the conduit of the blood circuit, and used for measurement in a state out of contact with blood. An existing product may also be used.

A mounting site on a side surface of the dialysis machine of the blood flow sensor is, for example, near a bodily fluid (blood) incoming part and near a bodily fluid outgoing part. However, one or a plurality of sensors may be disposed on other parts in the dialysis machine where the blood flow state can be sensed or on an element which constitutes the blood circuit such as a conduit or the like in which blood flows.

For mounting the blood flow sensor, more preferably, measurement is performed at four positions including near the bodily fluid (blood) incoming part, near the bodily fluid outgoing part, and positions at a certain distance apart from the center, and from blood flow amounts at these four positions, a position where the blood flow amount, the blood flow velocity, and the filtration flow rate become maximum or minimum (which is referred to as state change point) is preferably calculated.

For the measurement of the blood flow amount, in addition to the measurement at four positions simultaneously, it is also possible to measure while changing the position chronologically so as to go the round. The filtration flow rate shows an envelope-like change which decreases over time by the positive filtration action based on the blood flow rate, and increases the flow rate value again by back filtration based on the flow rate of the dialysate. Since this is based on a change in blood concentration caused by dehydration, part of the blood state in the body may be obtained by comparing with the blood flow in the body, and thus a relationship with the variations of blood pressure may be implied.

The term "individual difference information" is patient specific information which is recognized also as an individual in the present invention. For example, biological body signals from the patients, such as average body temperatures for the individual patients, habits which appear especially when the blood pressure is decreased, such as a habit appearing in a face and a habit of the patient-specific state which can be recorded on an image are stored as image data.

The term "image data" is, for example, a movie data, and may be recorded as-is as digital data. In addition, specific parts such as mouth, arms, legs, body movement or the like, parts having depressions or projections, which are more likely to be detected by image processing, or predetermined portions are set as characteristic points in advance. The movements of these characteristic points are stored as a pattern. During dialysis, these patterns are extracted. For example, when the pattern of similar movement occurs repeatedly, that is, the symptom of at low blood pressure is detected from the image and is acquired as one item of information which indicates a decrease in blood pressure.

Information indicating the decrease in blood pressure or indicating a warning sign of the decrease in blood pressure is not necessarily determined from one item of information, but is determined by other items of information. For example, data such as a peripheral vascular resistance value, blood flow amount information of the dialysis machine, or the like is detected, and the possibility of the decrease in blood pressure is determined based on data in the past or data indicating the decrease in blood pressure, and based on the result of detection, whether it is a decrease in blood pressure which needs treatment is determined. It is because the facial color, expression, body movement, temperature, and moisture may indicate that the blood pressure value is not necessarily decreased, but a function for preventing the decrease in blood pressure (such as contraction of the peripheral blood vessel) is working.

In other words, the present invention is intended to prevent various complications such as gangrene of limbs by controlling the dialysis treatment system including the dialysis machine, which is an artificial kidney corresponding to the patients' own organs based on individual difference data including expression, body temperature, blood flow, blood pressure, peripheral vascular resistance, moisture of the skin, and the like, and improve life prognosis by gentle dialysis treatment.

The term "blood pressure related information" indicates the peripheral vascular resistance value, the blood flow value, the blood pressure value, and related value, and preferably, indicates the peripheral vascular resistance value, the blood flow value, the blood pressure value, and the change amount of the values related thereto over time. For example, in a state in which the sensor for measuring the blood pressure related information is mounted, the change amounts such as a derivative value and a difference value are further obtained by detecting a sensor value continuously or intermittently, and the change in expression of the face is input to a computer as data, the change is detected, and from this change amount, lowering of the blood pressure is estimated, so that the treatment is performed before the blood pressure is lowered.

For example, a pulse wave sensor is mounted on a joint portion from a big toe to a little toe on the bottom of the foot and the pulse wave information is measured continuously. From the pulse wave value, an AIx indicated by a difference, a ratio, or the like between the ejection wave amplitude value and the reflected wave amplitude value for obtaining the peripheral vascular resistance value is calculated, and from the change amount, a change in blood pressure is obtained predictively.

Also, for example, performing the dialysis operation manually or supplementally by a computer system provided with a learning function such as an artificial intelligence is enabled.

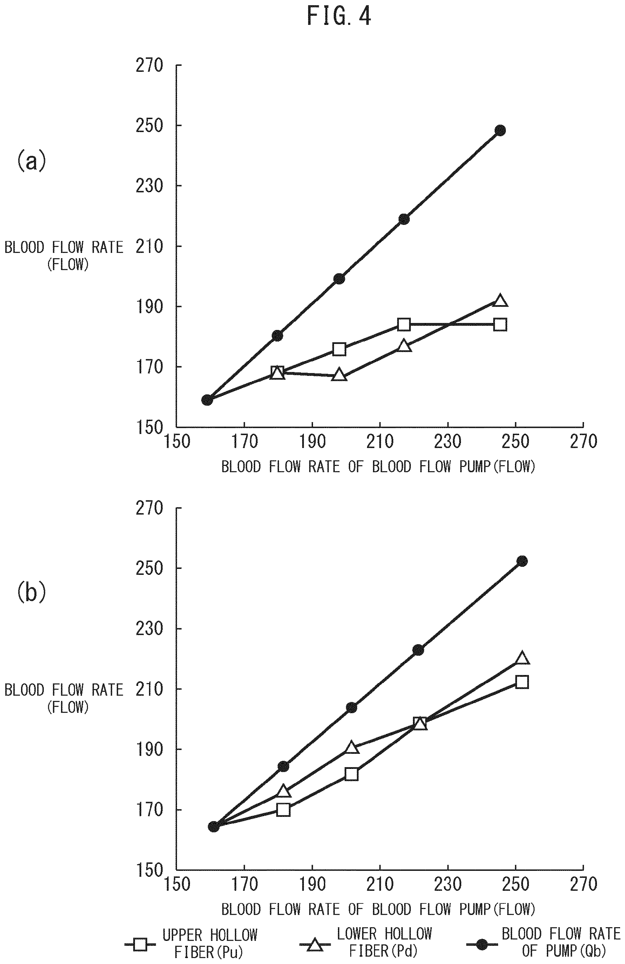

FIG. 4(a) and FIG. 4(b) are graphs plotting a difference in blood flow amount (FLOW) in the dialysis machines for two patients provided outside the bodies, respectively. Note that the blood flow amount was measured in a state in which probes of a laser blood flow meter (ALF21, manufactured by Advance Co., Ltd.) are adhered to an upper portion of a surface and a lower portion of the surface of a dialysis machine (dialyzer) (manufactured by Fresenius Medical Care Japan K.K.), respectively.

In addition, when the state change point obtained from blood flow information in the dialysis machine varies, a replacement fluid is administrated and driving means such as a blood pump is driven while varying the dosage of the replacement fluid for maintaining an optimum state and the driving amount of the blood pump.

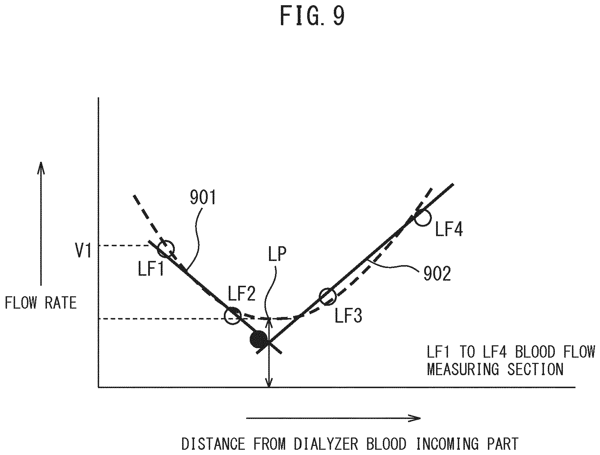

A point of change of the state in a blood flow distribution in the dialysis machine in the present invention indicates a minimum value or a maximum value in the blood flow information indicated by a change over time of the flow rate including the blood flow velocity and the blood flow amount in the dialyzer as illustrated in FIG. 9 for example.

Since the blood flow information at a center of the dialysis machine indicates a contact point of a blood flow information curve generated by the positive filtration and the back filtration, a point where this state changes varies due to the blood flow rate which is gradually changed in blood concentration by dehydration in contrast to a constant dialysate flow rate.

According to the present invention, the variations are measured and the blood pump is driven and the replacement fluid is administered so that the blood concentration is stabilized by suppressing the change due to dehydration. In this configuration, the need of estimation of an unstable PRR value from the circulating blood volume may be eliminated and the hemodialysis treatment in accordance with the individual difference may be enabled by enabling real time monitoring of the blood concentration.

It is regarded that a hollow fiber filter bundle constituting the dialysis machine serves as a load against movement of blood cells because of a pore diameter and a length, forms a rheological field even when it is diluted with physiological saline or the like, and forms blood flow difference as illustrated in Fig. 4 for a constant blood flow amount Qb generated by driving the blood pump in the blood circuit. Therefore, by applying statistical processing such as taking an average value to the blood flow value passing through the hollow fiber filter through the surface of the dialysis machine, effective blood flow information such as the actual blood flow, that is, an effective blood flow amount, an effective blood flow velocity may be used as individual difference information in individual difference information managing means.

The effective blood flow amount and blood flow velocity described above show different values from patient to patient, or depending on the patients' condition. Therefore, the individual difference information managing means records those values as the individual difference data for the individual patients, and the optimized dialysis means performs regulation to increase the driving amount of the blood pump as means for increasing the effective blood flow information.

At this time, considering the change in blood pressure due to the increase in dehydration speed, means for supplementing the blood concentration regulator such as saline water, glucose solution, and the like for regulating the PRR value in the direction of the blood returning part of the blood circuit by a predetermined amount may also be provided.

Referring again to Fig. 4. The vertical axis of the graphs represents the blood flow amount (FLOW) of respective parts per minute, and the abscissa axis represents the blood flow amount (FLOW) of the pump (the input flow rate to the dialyzer), and Qb represents the blood flow amount of the pump. The FLOW is a blood flow amount (blood flow rate), and is represented by Equation (3) described below. The term "blood flow amount" may be referred to as "blood flow rate".

In Fig. 4, Pu and Pd represent the blood flow amounts (FLOW) measured in an upper part of the dialyzer (hollow fiber) and a lower part of the dialyzer (hollow fiber), respectively. The blood pump (bodily fluid driving section) performs a rotation which achieves a constant flow rate, and thus assumes a proportional state. However, the blood flow amounts (FLOW) in the patients have different inclinations from each other, and shows different blood flow amounts from the blood flow amount achieved by driving he blood pump.

This difference may appear as difference from patient to patient. Actually, the dehydration amount set based on the flow rate of the blood pump may not be reached during the treatment time, or even when the dehydration amount is small, it may be a dehydration amount which induces the decrease in blood pressure.

Therefore, in the individual difference information managing means, when it is determined that the blood flow amount of the patient in the dialyzer is slower than a certain blood flow velocity or a blood flow amount in the blood circuit achieved by driving the blood flow pump, the optimized dialysis means performs an operation for increasing the driving amount of the blood flow pump. Preferably, the optimized dialysis means measures the blood flow amount and the blood flow velocity over time and provides a blood flow velocity equal to or higher than a normal 200 mL/min to form a blood flow optimal for dehydration for each patient. Accordingly, a sufficient dehydration amount may be ensured within the treatment time, so that an optimized dialysis may be achieved in accordance with the individual difference of the patients.

In addition, a point where the state of the blood flow changes in the dialyzer is obtained from a value obtained from the blood flow detection sensor disposed on the side surface of the dialysis machine by a curve or linear interpolation arithmetic processing as illustrated in FIG. 9, and the change of the point where the state changes is successively detected to inspect the variations. When the change amount of the variations is significant or the like, an increase in amount of the replacement fluid or an increase in rotating amount of the blood pump may be performed.

For measuring the above-described individual difference, the blood flow amounts at respective parts in the dialysis machine, the changes in faces of the patients, the changes in body movement, and the changes in body temperature are measured, and are converted into data. The data is stored in advance at the first dialysis treatment, and is used as a comparison data for treatment from the next time.

The expression "the blood flow in the blood circuit for hemodialysis is regulated based on the individual difference information of the individual difference information acquiring means" means, for example, that when the additional administration of the replacement fluid and the blood flow amount of the patient is small in the dialysis machine (dialyzer), the driving amount of the blood pump is increased and the flow rate of the pump is regulated to 200 mL/min or more. Alternatively, as means for regulating the blood flow, for example, the dehydration speed and the PRR are calculated, and the rotation speed of the blood pump is regulated such that a difference between the dehydration speed and the PRR falls within a range of approximately ±6 mL/min. It also includes replacing the dialysis machine with another one in which the pore diameter of the hollow fiber filter and void ratio of the porous portion are adjusted and performing dialysis, for example, in a case where the blood flow in the dialysis machine is low and an intended blood flow cannot be obtained even when the driving amount of the blood flow pump is increased.

In addition, it includes also a case where the dehydration amount is regulated by increasing the circulation amount by increasing the driving amount of the dialysate pump formed in the dialysate circulation circuit.

According to the present invention, the individual difference information managing means may form a combination of all or part of values obtained by measuring values based on the change of the peripheral vascular resistance related value over time, the blood pressure change, the change in temperature of limbs, face and other parts of the patient, the change in moisture of the face and the skin, the change in color of the face, the change in expression of the face, and the change in body movement, and the blood flow amount and blood flow velocity flowing in the hollow fiber bundle in the dialysis machine. When these changes are determined to affect the variations in blood pressure, the optimized dialysis means performs administration and regulation of the replacement fluid, regulation of the blood driving amount, regulation of the dialysate driving amount, regulation of dehydration amount, and administration of a replacement fluid newly adjusted in components, for example, by administering a concentration regulating agent for increasing the PRR value, so that an optimal dialysis is performed for each patient. This enables the patients to receive a gentle dialysis treatment, and thus elongation of the dialysis duration set for each patient to improve the life prognosis may be performed in parallel.

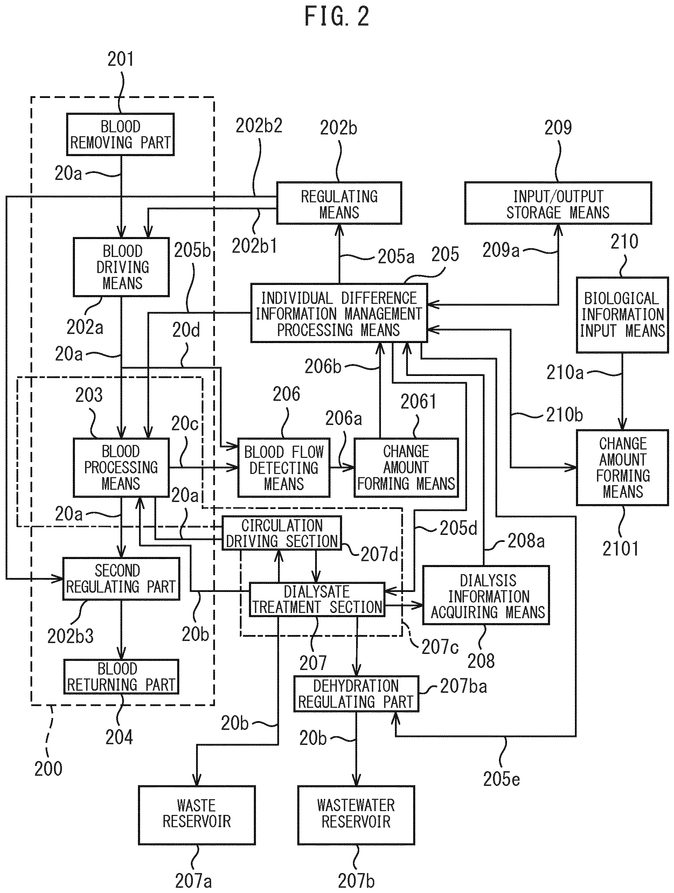

For regulation of the blood flow, there are illustrated a method of forming a situation in which the dehydration speed and the PRR value are balanced for each patient based on the method or the like disclosed in Susumu Ookawara, Mathematical Analysis of the Change in Circulating Blood Volume during Hemodialysis by Continuous Hematocrit Measurement, Journal of Japanese Society for Dialysis Therapy 31 (6): 1001 to 1005, 1998, Yu Dialysis Clinic-Material of 7th Study Session of Patients: (http://yu-toseki.jp/swfu/d/study7.pdf), Fig. 2, Annual Meeting of Japanese Society for Dialysis Therapy: Archived Abstracts of the General Assembly, Suguru Obunai and Satoaki Matoba, A case of finger gangrene that could be rescued by multidisciplinary treatment: Subject Number, P-1-181: (http:// /www.myschedule.jp/jsdt_archive/detail.php?sess_id=17048), and furthermore, depending on the case, a method of regulating the dialysis duration to four hours or more.

In the present invention, a gentle dialysis treatment with less risk of complications and improved life prognosis is performed for each patient by regulating a blood flow. In other words, in the present invention, the dehydration speed may be regulated in accordance with the above-described changes and the change amounts during the dialysis treatment for the patient.

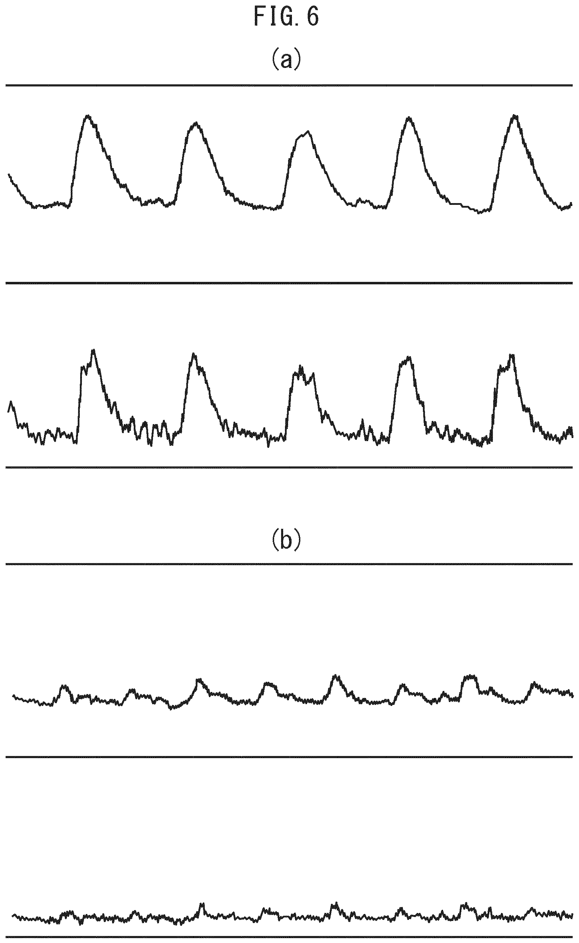

A decrease in blood flow in the peripheral blood vessel part may be expressed by a change in blood flow amount at two points on the skin of the lower leg portion, for example, for approximately one hour after the start of dialysis as illustrated in Fig. 6(a), and for approximately one hour before the end of the dialysis as illustrated in Fig. 6(b). In this manner, by detecting the consecutive decrease in blood flow, which may cause necrosis or the like, and performing regulation of the blood flow and circulation of the dialysate during dialysis, regulation of the fluid replacement volume, regulation of the dehydration speed, and regulation of other regulatable elements, resolving of sufferance of pain attacking at an end of the dialysis treatment, and preventing necrosis of the peripheral part such as fingers of the limbs are enabled. The measurement of the blood flow is performed, for example, by the laser blood flow meter (manufactured by Advance Co., Ltd.), so that a blood flow waveform may be obtained.

The change amount of the peripheral vascular resistance related value and the temperature change of the limbs enables determination of degree of progress of lower-limb ischemia before dialysis treatment or during dialysis treatment, determination of time for vascular treatment before an appearance of symptom of a serious lower-limb ischemia, determination of a relationship among the dehydration amount, the dehydration method, and circulatory disorder of the skin and the effect after the regenerative treatment, and the like. Based on this change information and biological information, the individual difference information managing means detects the tendency of decreasing blood pressure and the optimized dialysis means sometimes performs measures to raise the blood pressure value.

Preservation of homeostasis in the present invention indicates stabilization of blood properties, body temperature, etc. in dialysis treatment. With everyday life of the patient, a measuring device that measures consecutive data of body temperature, heart rate, calorie that the patient has spent, sleeping time, blood pressure is made as a portable carrier and is put on the patient all the time. Then we collect individual difference data of patients considering homeostasis. For example, temperature regulation of the blood flowing through the blood circuit, detection of possibility of changes in blood pressure during dialysis, regulation of the dehydration speed during dialysis treatment based on understanding of the physical and mental condition of the patient, and the like may be performed.

In addition, the type of music and information that patient likes are converted into data, and music or information is flowed through the earphone during the dialysis treatment, automatically or by selection by patient. Accordingly, it is also possible to alleviate the pain of during dialysis treatment. However, the timing may be output when it is determined as pain based on the information on the color of the face of the patient, expression information and body movement information.

[ADVANTAGEOUS EFFECTS OF INVENTION]

The present invention collects information based on the patient's individual difference from the patient and dialysis apparatus and performs optimization of so-called customized hemodialysis based on this collected individual difference information. By doing this, variations in blood pressure including abrupt decrease in blood pressure is suppressed, and a gentle treatment of patients is achieved. At the same time, it can reduce the complications related to the heart and blood vessels, prevent the necrosis of the extremities in the extremities, relieve the pain during dialysis, and improve the life prognosis.

BRIEF DESCRIPTION OF DRAWINGS

FIG. 1 is a diagram illustrating an example of the present invention.
FIG. 2 is a diagram illustrating an example of the present invention.
FIG. 3 is a diagram illustrating another example of the present invention.
FIG. 4 is a diagram for explaining an operation of another example of the present invention.
FIG. 5 is a diagram for explaining another example of the present invention.
FIG. 6 is a diagram illustrating an operation of another example of the present invention.
FIG. 7 is a diagram illustrating an operation of another example of the present invention.
FIG. 8 is a diagram illustrating a part of an example of the present invention.
FIG. 9 is a diagram illustrating an example of the present invention.
FIG. 10 is a diagram illustrating an example of the present invention.
FIG. 11 is a diagram for explaining another example of the present invention.
FIG. 12 is a drawing for explaining blood flow data measuring means of the present invention.
FIG. 13 is a diagram illustrating an operation at the time of measuring the blood flow data.
FIG. 14 is a diagram illustrating a relationship between dehydration speed and blood concentration (BV).

DESCRIPTION OF EMBODIMENTS

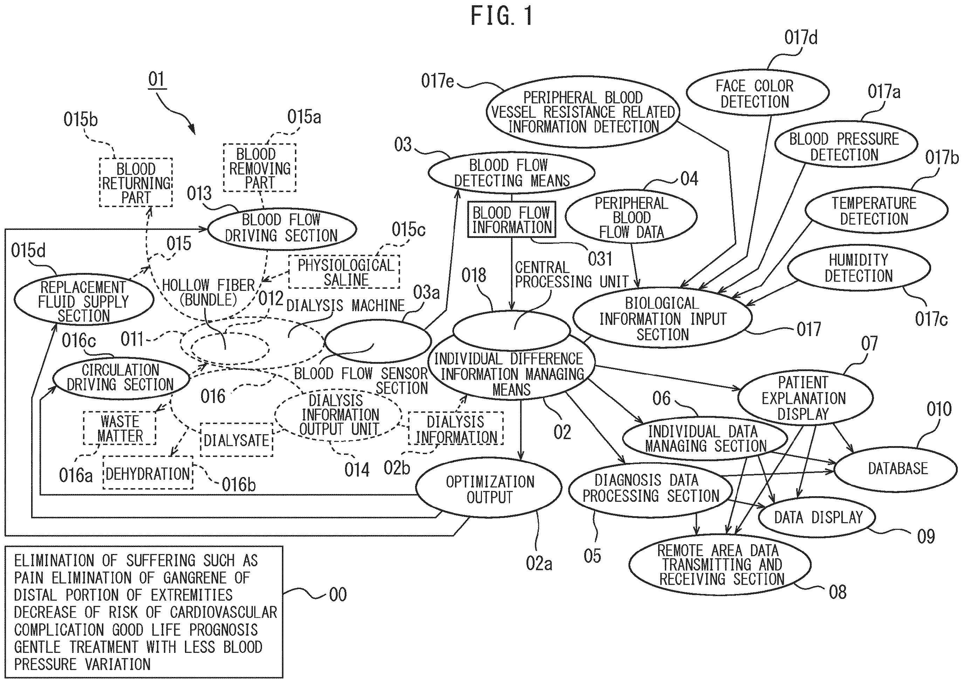

An operation of one form of present invention will be described with reference to the flowchart illustrated in FIG. 1. In FIG. 1, all of the configuration of the present invention is illustrated. However, a detection unit corresponding to the patient's individual difference may be selected from among the components illustrated in the figure. It is preferable to reduce the burden of the patients by minimize a measuring unit.

In FIG. 1, Reference sign 01 denotes a dialysis apparatus, which is illustrated by a simplified diagram to mainly show its operation. Parts indicated by the dotted line illustrate the configurations that the dialysis apparatus is equipped with. Although a conventional product is sufficient as the dialysis apparatus 01, it is preferable to incorporate driving means and a drive control unit, such as a replacement fluid supply section, a blood flow pump regulating section, etc., for performing at least a treatment for decrease in blood pressure.

Reference sign 011 denotes a dialysis machine (dialyzer), which is an existing product, and includes a hollow fiber filter 012, which is a bundle of hollow fibers made of a plurality of porous membranes. The hollow fiber filter 012 is incorporated in part of the dialysis machine 011. One or a plurality of blood flow sensor sections 03a for directly or indirectly detecting the blood flow information of the blood flowing inside the hollow fiber filter 012 from the dialysis machine 011 are mounted. It is preferable that sensors constituting the blood flow sensor section 03a be small and light enough to be attached and used. The number of the sensors is one or several on the upper part of the dialysis machine 011, that is, the part near the site where blood flows in, the middle part, and the bottom, and at least positions where a curve as illustrated in FIG. 9 is obtained are exemplified. Not that it may be fixed by using a mechanical jig without sticking.

Reference sign 012 denotes the hollow fiber filter, and includes, for example, a bundle of a plurality of filamentous hollow fibers formed of a hemodialysis membrane (porous membrane), for example, a PS membrane, a PES membrane, a PEPA membrane, a PMMA membrane, an EVAL membrane, a PAN membrane, and the like.

Reference sign 013 is a blood flow driving section, which is used for taking out blood from a blood removing part 015a to the outside, and for example, a roller type pump is used. Further, the blood flow driving section 013 is provided with regulating means including an electric control circuit for regulating the rotation speed of the impeller, which constitutes the roller portion. The regulating means supplies a regulated electrical output to the blood flow driven pump by means of an optimization signal output 02a from individual difference information managing means 02 and regulates the rotation speed so as to be optimum. The optimization includes outputting an regulating signal of the driving amount of the blood flow driving section 013 for eliminating the delay of the blood flow in the dialysis machine caused, for example, by individual differences, and a selecting operation to select a dialysis machine provided with another hollow fiber filter in which the pore diameter or the like of the hollow fiber filter in the dialysis machine is adjusted.

Reference sign 014 denotes a dialysis information output unit and outputs dialysis information 02b such as dehydration data, dialysate circulation rate, dialysate circulation amount, and the like. For example, the water removal data indicates dehydration amount, dehydration speed, and examples of other dialysis information 02b include waste matter component, a component that is an indispensable component as bodily fluid but is leaked out through voids of filter, and the like. The dialysate circulation rate and the like may be used as parameters for increasing the speed of dehydration speed, waste matter removal rate, and type thereof, for example, by increasing the speed. There is a case where the dialysis information output unit 014 may use the information obtained from the unit attached to the existing dialysis apparatus as-is.

Reference sign 015 denotes the blood circuit between the blood removing part 015a and a blood returning part 015b and indicates a configuration used or dialysis such as a physiological saline supply part 015c, an active ingredient to be lost by blood filtration, a replacement fluid supply section 015d for supplementing an effective bodily fluid component during dialysis.

In addition, the replacement fluid supply section 015d adds and supplies a blood concentration regulating agent such as saltwater, glucose solution etc. based on the control signal from the individual difference information managing means 02 or is a part for supplying a component which leaks out of the hole of the hollow fiber membrane, which is the replacement fluid containing essential ingredients.

The replacement fluid supply section 015d preferably has a configuration which can automatically administrate the replacement fluid quantitatively, and more preferably, it is performed by inputting an external electric signal at the time of administration.

Reference sign 016 denotes a dialysate circulation circuit, which make the dialysate circulates along an outer circumference of the hollow fiber filter (bundle) 012 in the dialysis machine 011 in an opposite direction from the direction of bodily fluid, and transports waste matter in the blood and extra water.

Reference sign 016a denotes a waste reservoir, which separates and stores waste matter transferred by dialysate including waste matter and the like, which passed through the holes of dialysis machine 011 by a filtration pressure or a concentration difference.

Reference sign 016b denotes a wastewater reservoir, which transports, separates and store the bodily fluid that passed through the filter holes of the dialysis machine 011 together with the dialysate. The bodily fluid obtained by dehydration is measured as, for example, an increment of dialysate.

Reference sign 016c denotes a circulation driving section, which includes a pump that drives the dialysate circulation amount of the dialysate circulation circuit 016, and a control electric circuit that regulates the driving amount of the pump. The circulation driving section 016c includes means for regulating the circulation amount based on the control electric signal from the individual difference information managing means 02.

The circulation driving section 016c regulates the driving amount of the circulation pump constituting the circulation driving section 016c based on the circulating driving electric signal within the optimization output from the individual difference information managing means 02.

Reference sign 017 denotes a biological information input section, includes input means indicated by 017a to 017e, for example, and inputs biological information such as blood pressure information, pulse wave information and the like. The biological information such as the blood pressure information and the pulse wave information may be input and used continuously or intermittently as data to be subjected to at least optimized dialysis, and may include a blood test information and the like in some cases.

Reference sign 017a denotes a blood pressure detection unit, and includes an invasive type and a non-invasive type for example, and a configuration that measures blood pressure continuously is preferable, and a non-invasive type with less risk, which may be caused by insertion into the body like an invasive type, is preferable.

Reference sign 017b denotes a temperature detection unit such as a body temperature sensor and a thermography camera and includes a contact type using a Peltier element or the like, a non-contact type using a thermography camera or the like for example, but it is preferable that continuous body temperature detection is possible. Temperature detection unit deep part 017b may be for measuring body temperature, and performs body temperature control that does not feel excessive cold at least during dialysis, and for example, an ear plug type that can always carry with stability is preferably used.

Reference sign 017c denotes a humidity detection unit, which is composed of a near infrared camera, a humidity sensor and the like, and it is preferable to have a configuration that can obtain sensor information that can detect an itching state, a dehydrating state, and the like by detecting the dry condition of the skin.

Reference sign 017d denotes a face color detection unit, which can photograph the facial color, continuously and intermittently with a WEB camera, a smartphone equipped camera, and other cameras, and replaces the data with the three primary colors such as RGB.

Reference sign 017e denotes a peripheral blood vessel resistance related information detection unit, and includes a combination of a pulse wave detection sensor and an arithmetic circuit for obtaining ejection wave time phase and amplitude value and reflected wave time phase and amplitude value, and obtaining the difference and ratio therebetween.

The peripheral vascular resistance related information includes not only the peripheral vascular resistance value but also the change amount of a resistance component parameter value indicated by a ratio or difference of the change amount of a resistance value, the ejection wave, and a reflection H.

These sensors may be integrated and formed, for example, like an earplug, or may be separated when the places suitable for individual sensing are apart from each other.

In the present invention, for the purpose of stability of blood pressure of during dialysis treatment, the optimized dialysis means controls regulation of dehydration amount and regulation of replacement fluid supply amount based on the change amount information of these sensors obtained by the individual difference information managing means.

Reference sign 018 denotes a central processing unit and includes a combination of personal computer, hard disk, monitor, mouse, keyboard etc., a combination of a server-computer and a server computer of other medical institution or a medical-related companies, and the like. The central processing unit may be part which constitutes the individual difference information managing means 02, and if plural, may represents a network configuration which allows data sharing.

The network may include, for example, an internet, an extra net, or a combination thereof.

In addition, the central processing unit 018 may be configured to perform a correct dialysis treatment management by being connected to a cloud server to constantly obtain the latest dialysis treatment information for allowing comparison with the biological information of the patient.

The central processing unit 018 may have a function for sharing data in which individual difference data for each patient to be managed by the individual difference information managing means 02, or a function for providing the individual difference information to a medical institution in a remote area for example. Also, the central processing unit 018 may have a function for monitoring a dialysis condition in the in-home hemodialysis, and collecting information such as a pulse wave of the patient, the blood pressure, the body temperature, the humidity information, the dialysis circulation information, and also has a function to issue an instruction regarding the operation of the dialysis treatment in case of emergency during dialysis at home through a both-direction communication with the patient by outputting images and voices from a computer monitor and a speaker provided on the individual difference information managing means 02 for example. This is not limited to the home use, but may be used in the dialysis treatment in the medical institution by forming the central processing unit 018 in a separate room, monitoring data of the patient, and displaying a corresponding signal depending on the condition.

In addition, the present invention may be provided with a cardiac electrogram detection sensor and a sensing unit for detecting breath sounds, sleeping, walking, calories that the patient has spent, or the like for collecting the individual difference information. In this case, if the individual difference information collecting device can be miniaturized, the miniaturized sensor may be made portable by forming into an earphone type sensor unit, which may be attached to ears or an ear, a ring shape or a finger loop shape which can be worn on the fingertip, the wrist, the instep, the heel, or the foot finger. The patient may wear such a sensor in a state of being free from a burden, and collect various items of sensor information in everyday life. During dialysis as well, the patient may wear the portable sensor in the same manner for recording data. Such an individual difference information collecting device may be provided.

Then, when a regular daily change in body temperature is different from that of the everyday life, homeostasis is maintained by regulating the blood temperature in the blood circuit, so that a gentle dialysis treatment can be performed.

When measuring the individual difference of the patients in a non-contact manner by a thermography camera or a camera for measuring a facial color, image following software may be used. The image following software tracks the movement of the face, the hand, and the legs, which are targets for measurement, in conformance with the rolling over or the change in face direction during the during dialysis treatment. In order to cover all directions of the patient as the tracking range, a plurality of cameras may be provided on the left, right, above and below of the bed. Alternatively a manipulator for moving the camera automatically may be used for tracking the target portions with a single camera. Accordingly, the target portions may be photographed.

The detection device used in the individual difference information managing means does not have to be the aforementioned sensors or the measuring devices, and may be selected for use. For example, the present invention may include a combination of means configured to measure the peripheral vascular resistance related value obtained by the pulse wave detector or the like and a change amount of the resistance value over time, image photographing and acquiring means configured to take still images or movies of the limbs, the head, and the face, a combination of body temperature measuring means configured to measure the temperature of the patient in a non-contact manner or a contact manner and detecting means configured to automatically read and detect the symptoms at low blood pressure from the obtained images, and a combination of facial color measuring means configured to measure the facial color of the patient.

The blood pressure is obtained by multiplying the peripheral vascular resistance by the blood flow as described in JP 2006-112277 A.

Detecting the change in the color of the face makes it possible to understand the condition of the patient, but for example, "the extraction and application of physiological information from the color change of the face, the 298th meeting of the Society of Instrument and Control Engineers, Tohoku Chapter (2015.11.18), Document No. 298-4" can be suitably used.

As a method of automatically detecting facial expressions, for example, facial expression recognition using Intel Perceptual Computing (PerC) SDK and Creative Intaractive Gesture Camera of made by Intel corporation, other kinect for Windows manufactured by Microsoft Corporation etc. are preferably available.

The measurement of the face, the humidity and the temperature of the skin can be carried out, for example, by photographing with a near infrared camera described in the document "Mariko EGAWA, Med Imag Tech Vol. 30, No. 1 January 2012", and photography method by a thermography camera or the like in the JP 2008- 534214 A is suitably used.

For example, a configuration in which the patient's chronological body temperature is measured by continuously or intermittently measuring the body temperature from a few days ago, and during dialysis, body temperature such as limb temperature etc. are measured continuously to perform dialysis treatment at least at the same temperature as he body temperature, a predetermined temperature such as 36.5 degrees, or at a temperature reduced by 0.5 degrees may be exemplified.

Likewise, the blood pressure sensor consecutively or intermittently measures the humidity of the skin and body temperature continuously or intermittently while measuring blood pressure consecutively and intermittently. At the same time, the blood sensor adds a temperature regulating or blood concentration regulating solution which stabilizes the blood pressure during the dialysis treatment as a replacement fluid, or regulates the blood flow pump speed or the dialysate circulating pump speed.

Reference sign 02 denotes individual difference information managing means, and includes for example, a personal computer, a storage medium such as and a hard disk, various interfaces such as a keyboard, a computer mouse, a liquid crystal monitor and the like, and stores a program for individual difference information processing, and reads out and executes the stored program on the computer.

The individual difference information managing means 02 receives patient's temperature data, humidity data, blood pressure data, facial color detection data, body movement, facial expression data, peripheral vascular resistance related value data, blood pressure data and heartbeat data from a biological information input section 017. Then, the individual difference information managing means 02 obtains its temporal change amount of these data. From these change amounts, the individual difference information managing means 02 predictively detects the change amounts of the blood pressure or the like and issues commands to a blood driving section 013, the replacement fluid supply section 015d, and the circulation driving section 016c, which are regulating units of the dialysis apparatus 01 in order to regulate the supply amount of the replacement fluid, the blood flow of the blood circuit, and the dialysate flow rate.

For example, the blood pressure can be expressed as the product of blood flow amount and peripheral vascular resistance, but it may adjust its component by homeostasis action. In that case, the change in measured blood pressure value and the action occurring in the body do not necessarily coincide with each other, and the blood pressure may not decrease as the dehydration amount increases. Therefore, it is necessary to measure the constituent elements of the blood pressure, and predictively measure the variation of the blood pressure value, not based on the variation of the blood pressure value, but based on the measurable value from outside the patient such as temperature, humidity, and facial color, which indicates the variation of the blood pressure.

As illustrated in FIG. 4, the blood flow flowing through the hollow fiber in the dialysis machine 011 has different blood flow rates at each part of the dialysis machine 011 from patient to patient, and there are individual differences between patients. The blood pump speed is regulated based on the blood flow amount for each patient, and the balance state of PRR value and dehydration speed (UFR) value is maintained in some cases.

This balance should indicate a state with little variation in blood pressure value, or a patient's state with mild facial color, facial expression, and motion. Increasing the driving amount of the blood flow pump often increases the dehydration amount. As a result, optimization dialysis drive may be performed when the dehydration speed becomes too fast with respect to the PRR value and exceeds the predetermined amount. The optimization dialysis drive is an operation of reducing the driving amount of the blood flow pump, for example, or supplying salt water or glucose solution from the replacement fluid supply section 015d, regulating the blood concentration to a dark direction and increasing the PRR value. Furthermore, while maintaining the difference between the dehydration speed and the PRR value within the predetermined amount, driving regulation may be performed to operate the driving amount of the blood pump based on the blood flow information obtained from the surface of the dialysis machine.

According to the present invention, the temperature data, humidity data, facial color data, data indicating body movement and facial expression of patient, blood pressure amount, blood flow amount, and change amount of peripheral vascular resistance related value are measured. Furthermore, associating the measured values with the situation of patient at that time, accumulation such as making it into a database is performed. Based on these change amount and patient's status during dialysis treatment, data to stabilize blood pressure is provided, and based on this data, regulation of the replacement fluid supply amount or the like is performed. However, it is also possible predictively determine a sign of blood pressure variation from the temperature data, humidity data, facial color data, data indicating body movement and facial expression of patient, blood pressure amount, blood flow amount, and change amount of peripheral vascular resistance related value by an expert-type computer having a learning capability, which is referred to as a patient's state artificial intelligence during dialysis treatment.

Artificial intelligence (AI) in the present invention performs, for example, a process of treatment such as dehydration speed control, supplying various replacement fluids and the like while predicting the blood pressure variation based on the information obtained from the dialysis patient and based on the individual difference. In addition, an appropriate amount regulation or the like of the dialysis volume depending on the individual difference is carried out.

Artificial intelligence is an example of means for appropriately regulating whether or not treatment can be accomplished gently by predicting the result of dialysis treatment by performing specific recognition, for example, based on input data. Further, for example, the AI predicts whether or not variations in blood pressure can occur suddenly based on the patient's individual difference information or predicts the same from generally available patient statistics data such as cloud data, and selects an optimum treatment means based on the predicted value.

The artificial intelligence also includes machine learning (machine learning), deep learning, etc.

The artificial intelligence includes a first generation (search and reasoning), a second generation (era of knowledge), a third generation (era of machine learning), a fourth generation (deep learning), and is one of a method of machine learning in which a system learns the data characteristics and recognize or classify the events (Yutaka Matsuo, Does Artificial Intelligence go beyond humans? kansai.main.jp/swfu/d/bookcafe20160312.pdf), and any method of those generation may be used. Among these methods, a boosting method in machine learning detects pattern data such as blood pressure change, blood pressure change, etc. with possibility of decrease in blood pressure every unit time, selects and adds weights from a pattern matching between a pattern of change per unit time and a pattern in the usual events, selects weights, and adds them over time. When matched with or approximated to the normal pattern, the weight is reduced. In contrast, if it is not an ordinary pattern, or a pattern not included in a pattern, the weights are changed so as to correspond to the degree of approximation. For example, if it is not an ordinary pattern and, as a result, a sudden change in blood pressure occurs, a sign to that effect is given and the weight is increased.

From the added weight and the change in weight, whether or not dialysis treatment is close to rest is predictively judged with time or at a predetermined time interval. When the weight exceeds a certain value, inference prediction of the possibility of variations in blood pressure is performed from the degree of excess. From the total amount of weight added over time and the change of the weight, regulation of the dehydration speed or regulation of the replacement fluid component automatically or prescription guidance is notified to a doctor, a nurse, a technician, etc. on the computer screen, voice, or printed matter or the like.

When the weight exceed is a predetermined value or greater but shows a normal state as a result, the light weight corresponding to the normal pattern is set and updated assuming that the state is normal.

The pattern forms a rule, and in the result after the trial, the magnitude of the weight is regulated and updated as described above.

Such a program based on the boosting method is described on a site (opencv.jp) which is a software library for computers such as Open CV or the like, that is, a side where the module is published. The example of the present invention may be implemented by downloading the program from this site.

A method of calculating the PRR value when performing dehydration speed operation based on the PRR value includes; for example, following documents: Yamanaka et al., Journal of Japanese Society for Dialysis Therapy, 35 (2): 97-107,2002, hematocrit value Investigation of usefulness and limits of various plasma refilling rate measurement method using continuous measurement device; Kihara et al., Journal of Japanese Society for Dialysis Therapy, 40 (3): 241-245, 2007, plasma in diabetic blood dialysis patient Study of refilling rate (PRR); Shibata et al., Journal of Japanese Society for Dialysis Therapy, 35 (10): 1337 to 1342, 2002, useful for the water removal control function based on BVM of personal dialysis apparatus 4008S made by Fresenius PRR value calculation function of dialysis apparatus indicated in sex etc.;

A method based on International Publication No. 2003/9888 is exemplified, and it may be performed by a computer storing a computer program including a calculation algorithm.

Note that, calculation of PRR value is not always necessary when it cannot be used for optimized dialysis treatment, such as when the calculation speed is slow, it may be enough to selectively use data indicating blood pressure variations.

For example, as an example of blood flow information in the dialysis machine, blood flow information directly affected by dehydrating operation can be obtained in the vicinity of a center of the dialysis machine in relation between positive filtration and back filtration. From this blood flow information, the blood flow amount, blood flow information near the boundary where the action of the positive filtration to the back filtration becomes large becomes the blood flow information at the time of dehydration. Therefore, on the premise that the fluid replacement volume such as dialysate supplied on the extracorporeal blood circuit is considered, determination information indicating whether the dehydrating state is appropriate or not may be obtained in some cases from the magnitude of the difference from the blood flow information after plasma refilling in the patient body.

If the size of the hollow fiber filter of the dialysis machine is not appropriate for the patient, the individual difference information managing means 02 may separately prepare dialysis machines with different pore sizes, lengths and the like of the hollow fiber filter, and perform switching operations.

Changing the size of the hollow fiber filter means, for example, at least, preparing the dialysis machine (dialyzer) 011 having the hollow fiber filter 012 having a different size and selecting the optimal dialysis machine 011 for patient.

Reference sign 03 designate a blood flow detecting means and includes a laser blood flow meter, an ultrasonic blood flow meter, and a contact type blood flow meter or the like, and detects (031) the blood flow information, such as the blood flow amount, the blood flow velocity, the hematocrit value and foreign substance information.

When a laser Doppler type blood flow meter is used, the blood flow amount (blood volume of flowing blood) (MASS) is obtained by the following Equation (1), the blood flow velocity (VELOCITY) is obtained from the following Equation (2), And the blood flow rate (FLOW) is expressed by the following Equation (3). Note that the blood flow rate is also called the blood flow amount as described above. [Equation 1]

$$\text{BLOOD AMOUNT (MASS)} = k_M \int_{f_1}^{f_2} P(f)\,df \,\big/\, \langle I^2 \rangle \qquad (1)$$

In the above equation and in the following equation, $k_M$, $k_V$ and $k_F$ are coefficients, $f_1$ and $f_2$ are cutoff frequencies of the bandpass filter, $P(f)$ is a power spectrum, and $\langle I^2 \rangle$ Is the total power of the light receiving signal.
[Equation 2]

$$\text{BLOOD FLOW VELOCITY (VELOCITY)} = k_V(f) = k_V \int_{f_1}^{f_2} f \cdot P(f) \, df \Big/ \int_{f_1}^{f_2} P(f) \, df$$

$$\cdots (2) \qquad f_1 = 2\,4\,Hz \quad f_2 = 2\,4\,KHz$$

[Equation 3]

$$\text{BLOOD FLOW RATE (FLOW)} = k_F \int_{f_1}^{f_2} f \cdot P(f) \, df \Big/ \langle I^2 \rangle \qquad \cdots (3)$$

$$k_F = k_V \cdot k_M$$

A blood flow detecting means 03 may detect erythrocytes, leukocytes and other thrombotic plugs and the like, and may also detect foreign matter information with large size particles contained in dialysate.

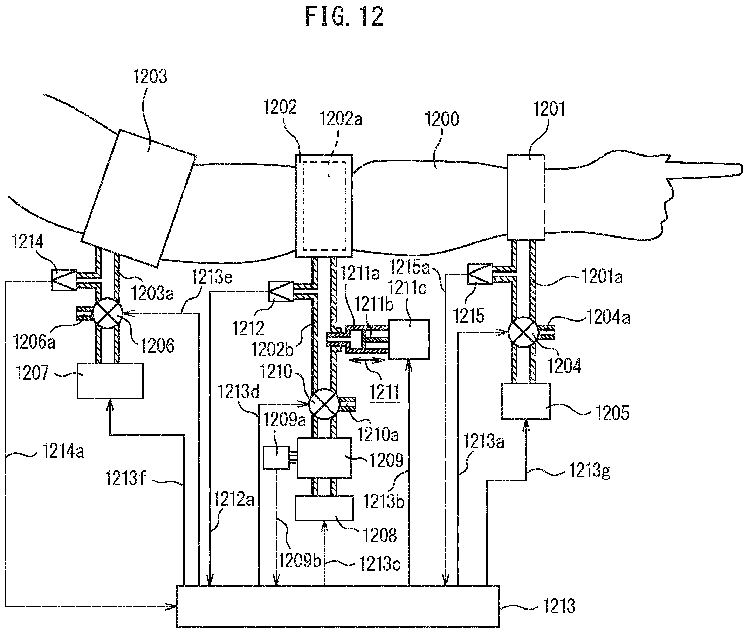

For example, the blood flow velocity value obtained in the example illustrated in FIG. 12 is an estimable value of the absolute amount. Therefore, even without obtaining blood flow velocity based on the example illustrated in FIG. 12 at constant intervals, a configuration may be made such that the value obtained from the blood flow meter is calibrated to obtain more accurate blood flow information, the dialysis treatment is monitored, and unstable blood flow values are detected.

Furthermore, a hematocrit value is obtained by separately connecting a hematocrit value monitor and the like. The hematocrit value may be detected by blood flow detecting means 03 from the blood flow information obtained by a blood flow sensor section 03a.

Note that a hematocrit value monitor may be incorporated into the blood flow detecting means 03. The hematocrit value monitor includes a light emitting element that outputs near infrared light and a light receiving element, and is preferably used for measuring light absorbency by being mounted on a surface of a flexible tube of a blood circuit, which is a tube having a wavelength of about 780 to 805 nm, for example, and is configured to allow passage of near infrared lights. From the necessity of error correction, it is preferable to arrange a plurality of hematocrit value monitors and use a value obtained by averaging obtained received electric conversion signal value.

A BV (Blood Volume) value used to calculate the PRR value and the UFR value can be detected by the blood flow meter unit equipped with the absorbance measurement function. Therefore, the blood flow sensor section 03a may be added to a blood circuit 015, which includes the blood removing part 015a, the dialysis machine 011 and the blood returning part 015b.

Reference sign 04 denotes blood flow data obtained from a blood flow measuring device configured to separately detect a blood flow in extremities and peripheral part of the head. The number of blood flow measuring devices is not limited as long as it is 1 or more, and it may not be necessary if comparison is not required.

FIG. 6 illustrating a specific example is an output chart obtained by measuring the peripheral blood flow in the skin of the lower leg portion after treatment and before the end of treatment by a laser blood flow meter during dialysis treatment. It indicates that blood flow decreases during dialysis treatment.

By monitoring peripheral blood flow, intermittently or continuously during dialysis treatment, the state of blood flow can be recognized, and possibility of gangrene can be judged in some cases.

Localized blood pressure value can be obtained by digitizing the blood flow data, digitizing the peripheral vascular resistance related value, and obtaining the product of both.

In some cases, the variation amount of the whole blood pressure may be obtained from the variation amount of this value.

Reference sign 05 denotes a diagnostic data processing section configured to store blood flow information or the like output from the individual difference information managing means 02 and the digital data for identifying the patient corresponding thereto in the patient data storage area of a server, and further store patient data such as the dehydration amount obtained from the dialysis information output unit 014, and perform processing for using it as data such as risk prediction during hemodialysis.

The diagnostic data processing section 05 may be built in the computer or computer server of the medical institution, and may be used as data of part of electronic medical records.

In addition, the diagnostic data processing section 05 may collect a blood flow information 031 and the dialysis information 02b during hemodialysis for the first time, then forms individual difference optimization information for performing optimal dialysis for each patient from these items of information, and may store the same in an individual data managing section 06 and database 010 in some cases.

Reference sign 06 denotes an individual data managing section which stores individual data of the patient formed in the individual difference information managing means 02, and dialysis data in a state of usable as data for dialysis such as speed regulating data of the dialysis information output unit 014, and time elapsed data of the PRR value and the Ultrafiltration Rage (UFR) of the dialysis information output unit 014, the value PRR and the Ultrafiltration Rate (UFR) value over time data, etc., available as data for dialysis, available as reference data for the next dialysis, and is made up of a computer that executes by means of a computer program and processes dialysis data.

Reference sign 07 denotes a patient explanation display, including a liquid crystal computer display and printer, and form data which make the patient's dialysis risk by optimizing the dialysis by individual difference information management displayable in an easy-to-understand manner together with individual data output from the individual difference information managing means 02.

Also, after the initial dialysis treatment, dialysis menu for performing optimized hemodialysis may be displayed from the patient-specific information for explanation to the patient.

Since dialysis is in accordance with individual differences, the dialysis duration may be prolonged. Therefore, when explaining to this patient, for example, the regulatable range of the blood flow amount is narrow due to the individual difference of bodily fluid. Therefore, if the longer time helps to enhance the dialysis efficiency, it may be preferable to give an explanation while displaying the blood flow state flowing in the dialysis machine per patient as statistical data.

It is also possible that a patient explanation display 07 may also execute a computer program and may include a computer having a storage for storing the data transmitted from the individual difference information managing means 02, a monitor, and a printer.

Since all of these diagnostic data processing section 05, the individual data managing section 06, and the patient explanation display 07 include a computer or a server, these members may be aggregated into one computer or server, or required data may be stored in an USB memory or the smartphone, or the like when the patient goes to a remote area.

Reference sign 08 denotes a remote area data transmitting and receiving section which includes a network connection such as internet and transmits and receives individual data processed and formed by the diagnostic data processing section 05 via a remote area terminal.

When dialysis is performed in a place remote from the dialysis medical institution specified by patient, hemodialysis constitution data based on individual difference data is provided so that dialysis suitable for patient can also be performed in the remote area. Furthermore, by sharing patient explanation data and sharing individual dialysis data such as blood flow data, dialysis suitable for patient and gentle hemodialysis according to the predisposition of patient can be performed always even in the remote area.

Since customized dialysis treatment performed for each patient is based on optimization data, it is preferable that optimization data for each patient is available for the dialysis medical institution. Therefore, it is preferable to make the dialysis treatment optimized for each patient possible even in a remote area or in a devastated district, for example, by storing the dialysis treatment data for optimization on a medium sharing state by internet or in a media-type medium with a storage medium such as IC card, USB memory, SD card, smartphone etc.

Reference sign 09 denotes a data display unit, including display via a computer monitor, or display via printed matter printed by a printer, and is used for explaining data to the patients or for sharing data display among physicians, and so on.

Reference sign 010 denotes a database which is exemplified by one configured as a server to accumulate patient data for each dialysis medical institution and a shared database server configured to share data between dialysis medical institutions. Particularly by sharing patient's optimized dialysis treatment data with dialysis medical institution, patients can receive dialysis treatment optimized for each patient at remote area, devastated districts, or the like.

The database 010 contains external databases both at home and abroad, and contains not only patient data but also databases such as document data relating to hemodialysis. The database 010 may contain so-called big data. In some cases, a database is created by using a server in the cloud.

Examples of configurations illustrated in the drawings above specifically include a combination of computers which constitute the dialysis apparatus, the blood flow measuring device of the dialysis machine, the biological information input section, and individual difference information processing section. In addition, a server, cloud server provided with computing capability that can perform hemodialysis in which an effective blood flow amount is secured based on the individual difference information of patients.

According to one embodiment including the example of the present invention illustrated in FIG. 1, an advantageous effects based on optimization dialysis treatment data indicated by 00 in the drawing can be expected.

In other words, examples of the advantageous effects of action include gentle dialysis treatment without pain owing to stabilization of blood pressure, elimination of suffer such as pain, elimination of gangrene of distal portion of the extremities, reduction of complications risk on cardiovascular, and good life prognosis.

EXAMPLES

Subsequently, the present invention will be described with reference to examples. Note that the present invention is not limited by these examples, but can be arbitrarily changed or improved in the spirit and within a scope of the present invention.

FIG. 2 illustrates an example of the present invention. FIG. 2 illustrates an example of a further specific configuration of the example in FIG. 1.

Reference sign 201 denotes a blood removing part, which corresponds to 015a in FIG. 1 and is a part for extracting blood from the artery of the upper arm to the outside of the body. Reference sign 204 denotes a blood returning part, which corresponds to 015b in FIG. 1 and is a part for returning cleansed blood from the vein near the blood removing part 201 into the living body.

The blood removing part 201 and the blood returning part 204 are formed, for example, in the vicinity of a shunt part formed in the extremities, the upper arm portion, or the like in advance.

A part which performs the external blood circulation between the blood removing part 201 and the blood returning part 204 is defined as a blood circuit 200.

Reference sign 202a denotes a blood driving means, which corresponds to 013 in FIG. 1 and includes, for example, a blood pump which rotates a roller to generate a blood flow.

Reference sign 202b denotes regulating means and includes an electric circuit or the like configured to regulates the rotation speed of the blood pump by an external regulating signal. The regulating means 202b forms one of the optimization signal outputs 02a illustrated in FIG. 1 and outputs a signal for regulating, for example, the rotation speed of the blood driving means 202a illustrated in FIG. 2 via an electrical lead wire 202b1.

Reference sign 202b3 denotes second regulating means composed of a combination of a reservoir for storing a concentration regulating solution formed of a solution such as salt or glucose and a solenoid valve configured to open and close by an external control signal, includes an automatic apparatus in which a concentration regulating solution in a reservoir is supplied to a conduit forming the blood circuit 200 by opening and closing of the solenoid valve attached to on outlet of the reservoir, or an apparatus configured to quantitatively provide the concentration regulating solution to the blood circuit 200 by a physician or a nurse, and corresponds to the replacement fluid supply section 015d in FIG. 1.

A second regulating means 202b3 supplies the concentration regulating solution to the blood circuit 200 so that the quantity can be regulated by an external control signal from the outside in the above-described configuration or manually.

Control of the electric signal via an electrical lead wire 202b2 from a regulating means 202b starts and stops the supply of the concentration regulating solution and regulates the supply amount.

Reference sign 203 denotes a blood processing means, which corresponds to the dialysis machine (dialyzer) 011 in FIG. 1, and includes the hollow fiber bundle (corresponding to 012 in FIG. 1) formed of a porous permeable membrane. In a periphery thereof, a circulation path in which the dialysate circulates is formed.

Reference sign 205 denotes individual difference information management processing means, which corresponds to the individual difference information managing means 02 in FIG. 1, and includes a computer interface such as a computer monitor, a keyboard, a mouse and a touchpad, and an internet and a router. The data processing operation, the optimization operation, the data accumulation operation to the input/output storage means 209, etc. of the individual difference information processing means 205 are performed by a doctor, a nurse, a person performing a medical office work, and the like. In addition, individual difference information management processing means performs display output to perform operation explanation etc., to patients for dialysis operation according to present invention, records data obtained from input blood flow information, dialysis information, or the patient, and detects and manages the individual differences.

Alternatively, dehydration speed and PRR value may be calculated by inputting blood flow information from change amount forming means A2061 and dehydration information from dialysis information acquiring means 208.

In addition, a possibility of lowering the blood pressure or forming an inclination of the blood pressure likely to lower is found from the change amount information of the biological information input from the biological information input means, the individual difference information management processing means 205 outputs an electric signal directing the supply of concentration regulating solution for regulates blood concentration to the regulating means 202b.

The inclination of the blood pressure likely to lower indicates a time point when the possibility that the inclination of adjacent measured values direct downward becomes high, for example, when the inclination implies descending property.

Reference sign 205c denotes an input end and is a part configured to input patient information such as patient's blood pressure value, pulse value, blood glucose level, peripheral blood flow value and the like.

The input end 205c performs data processing for diagnosis and the like, and based on these information, outputs a signal for regulating the driving amount of the blood driving means 202a to an optimized value to the regulating means 202b through via an electrical lead wire 205a.

Reference sign 206 denotes a blood flow detecting means, which measures the blood flow in an ultrasonic blood flow meter a laser blood flow meter or the like of the dialysis machine in a non-contact manner. The blood flow detecting means 206 is a conduit on the surface of the dialysis machine (dialyzer) including a hollow fiber filter and a dialysate circulation part constituting the blood processing means 203, and a conduit for other blood circuits, and connects sensors to a site where the blood flow can be measured, detects the blood flow amount, the blood flow velocity, and heterogeneous data and the like, and, for example, outputs as an electric signal data indicating the values processed by the above Equations (1) to (3), for example. The hematocrit value may be obtained by separately providing a hematocrit value detection device, mounting a sensor on a surface of a tubular body 20a having near infrared permeability, and receiving a photoelectric converted electric signal via an electrical lead wire 20d.

The dialysis machine is made of a material that allows transmission of at least laser light and ultrasonic wave used for blood flow detection and allows blood flowing in the hollow fiber to be irradiated therewith.

Note that the dialysis machine may be made a material having non permeability for the laser light or the ultrasonic wave in a case of a contact type blood flow meter using an electrode used in contact with blood flow as a probe instead of using a non-contact type blood flow meter such as the ultrasonic wave. Alternatively, a sensor for a hematocrit monitor may be attached to the same site as the sensor of blood flow meter to detect the hematocrit value at the same time.

Reference sign 2061 denotes change amount forming means A configured to calculates a change amount (for example, a difference value) during the predetermined time (for example, 1 msec or more) interval of the blood flow data input from the blood flow detecting means 206 via the electrical lead wire 206a, and output an electric signal converted from the change amount data and the blood flow data to the individual difference information management processing means 205 via an electrical lead wire 206b to the individual difference information management processing means 205.

Reference sign 207 denotes a dialysate treatment section, which is configured to circulate dialysate in the opposite direction of blood flow around the porous hollow fiber membrane in the dialysis machine. The dialysate treatment section 207 also includes a circulation driving section 207d such as a circulation driving pump, and transfers waste matter and moisture which have passed through the porous hole portion of the hollow fiber membrane and stores them in a waste reservoir 207a and a wastewater reservoir 207b which store them, respectively by being connected to the flow path through which the dialysate circulates.

Routes through which dialysate circulates are indicated by a dialysate circuit 207c (dashed line).

The circulation driving section 207d is regulated based on a regulating signal including the circulation rate of the dialysate and output via the electrical lead wire 205d from the individual difference information managing means 205. This allows the circulation driving section 207d to regulate the dehydration amount and dehydration speed by increasing or decreasing the negative pressure for the hollow fiber corresponding to the circulation rate.

Reference sign 207a denotes a waste reservoir. The dialysate transfers waste matter in the blood flowing in the interior of the hollow fiber and passed through the pores in a wall surface of the porous hollow fiber and stores the same in the waste reservoir 207a.

Waste matter stored in the waste reservoir 207a may contain substances required for the body. Therefore, means for analyzing waste matter and means for adding water to the replacement fluid supplied rom the replacement fluid supply section (indicated by 015d in FIG. 1) may be provided.

Reference sign 207b denotes a wastewater reservoir, which is configured to store excess moisture in the blood. The wastewater reservoir 207b is narrowed in an area near the exit of the hollow fiber bundle. This forms a state where moisture is encouraged to pass through the porous membrane. The wastewater reservoir 207b stores a moisture by an amount of increase of the dialysate by containing the dehydration amount, that is, by a predetermined dehydration amount, for example.

Reference sign 207ba denotes a dehydration regulating part, which includes a solenoid valve or the like, and is configured to regulate the dehydration amount from the bodily fluid flowing through the dialysate circulation circuit for dehydration based on a signal of a control input 205e.

Reference numeral 208 denotes a dialysis information acquiring means, and includes a computer, a specific apparatus, and the like. The dialysis information acquiring means 208 acquires dehydration information such as data for obtaining the dehydration speed from the dehydration amount and outputs the acquired information to the individual difference information management processing means 205 via an electrical lead wire 208a. Note that, the dehydration information such as dehydration speed may be detected using ultrasonic wave sensor or the like from a blood circuit, or if it is regulated in a known method, the regulated value may be used.

A reference sign 209 denotes an input/output storage means, which includes a high capacity storage medium such as a monitor, a computer mouse, a keyboard, a hard disk, etc., and a storage area such as a computer, a server or a cloud server. The input/output storage means 209 includes the database 010 in FIG. 1 and displays an image for explaining the treatment to the patient. The input/output storage means 209 displays and outputs patient information, and dialysis treatment information to a doctor, a nurse, a clinical engineering technician or the like, and allows them to input for operating this example. The input/output storage means 209 is connected to the individual difference information processing means 205 via an electrical lead wire 209a, and accumulates data processed or calculated by the individual difference information processing means 205 temporarily or continuously for being used as the patient specific dialysis information. Also, the input/output storage means 209 stores data used as data when performing dialysis for a patient common to other dialysis medical institution dialysis medical institution.

In this example, after detecting the blood flow information during dialysis for the first time, an optimal dialysis menu is formed based on the patient individual difference by analysis. Therefore, in addition to electronic medical records but also analytic data thereof, dialysis menu data based on the individual difference may be stored.

Reference sign 210 denotes a biological information input means, which is configured to input facial color data, peripheral vascular resistance related data, blood pressure data, temperature data, humidity data, facial color data, facial expression, fetal activity data and the like formed by the biological information input section 017 and the like illustrated in FIG. 1 as analogue or digital data output from the various sensor units.

The biological information input means 210 converts the biological information into computable data and outputs the same to change amount forming means B2101 via an electrical lead wire 210a.

In the case of image data, characteristic points of the image (the contact of the outline such as eyes, ears, mouth etc.) are extracted. The characteristic points have 2 dimensional or 3 dimensional coordinates, and are computable by change amount forming means B2101 downstream.

Reference numeral 2101 denotes change amount forming means B, configured to calculate input biological information data at a predetermined time interval (for example, 1 msec or more) to obtain a difference value and output the biological information data and the difference value data to the individual difference information management processing means 205 via an electrical lead wire 210b.

Reference sign 20a denotes a tubular body through which blood flows and reference sign 20b denotes a tubular body through which the dialysate can flow. Reference sign 20c denotes a connection line, which varies depending on the type of blood flow detecting device, but is formed by a combination of an optical fiber light guide and an electrical lead wire or an optical fiber light guide. Connections other than a conduit 20a, a conduit 20b and a connection line 20c are made by electrical lead wire.

The electrical lead wire indicates an electric connection, is used to distinguish the electrical connection from a conduit, light guide, etc. USB cable and LAN cable, Wi-Fi, Bluetooth (registered trademark), data communication by wireless such as infrared is also included in the electrical connection. Also, the conduit may be of any type for flowing blood and the dialysate. The light guide also includes an optical transfer member including laser light and other information.

Next, an operation of the example illustrated in FIG. 2 will be described.

Within the blood circuit 200, the blood removing part 201 and the blood returning part 204 are formed by, for example, bringing the artery and the vein of the upper arm into a shunt state or the like. The blood driving means 202a performs a constant driving so that, for example, 200ml of blood can be taken out from the blood removing part 201 per minute.

The blood removed from the blood removing part 201 is supplied to the blood processing means 203. The dialysate treatment section 207 forms the dialysate circuit 207c by driving and circulating dialysate so that dialysate flows along an outer surface of the hollow fiber filter of the blood processing means 203.

The dialysate circulating through the dialysate circuit 207c circulates with waste matter and the water passing obtained when the blood passes through porous hollow fiber of the blood processing means 203 and passing through the porous hole portion, and waste matter and the water are stored in the including the waste reservoir 207a, wastewater reservoir 207b respectively.

The biological information input means 210 receives patient's data from the blood pressure detection unit 017a, the temperature detection unit 017b, the humidity detection unit 017c, the face color detection unit 017d and the peripheral blood vessel resistance related information detection unit 017e illustrated in FIG. 1, and outputs to a change amount forming means B2101. The change amount forming means B2101 outputs a difference data of a biological information data for each predetermined time interval and outputs the difference data to the individual difference information management processing means 205 via the electrical lead wire 210b.

For example, in a case where the change amount data of the resistance related value obtained from the peripheral blood vessel resistance related information detection unit 017e illustrated in FIG. 1 input from the change amount forming means B2101 exceeds a predetermined threshold value, and the variation amount of the blood pressure value is small, and in addition, in a case where the change in the facial color image is directed toward low blood pressure, the individual difference information management processing means 205 detects that there is a possibility that sudden hypotension may occur, and inform this possibility to a monitor of the input/output storage means 209. At the same time, the individual difference information management processing means 205 regulates the driving amounts of regulating means 202b and circulation driving section 202a3, and outputs a display of a command to a doctor, a nurse, and a technician to reduce the dehydration amount manually by increasing the blood flow by the additional supply of the replacement fluid, and reducing the blood circulation amount.

Also, the blood flow information obtained from the sensor connected to the blood processing means 203 is converted into blood flow data such as blood flow amount, blood flow velocity, foreign body data, etc. with blood flow detecting means A206 and output to change amount forming means A2061.

The change amount forming means A2061 converts the input blood flow data into a blood flow change amount by differentiation at a predetermined time interval, and transmits the blood flow change amount to the individual difference information management processing means 205 together with the blood flow data.

The dialysis information acquiring means 208 analyzes waste matter, calculates its components and component quantities, data such as dehydration speed and dehydration amount, and change amount (difference), and transmits the calculated results to the individual difference information management processing means 205.

The individual difference information management processing means 205 receives a signal for optimizing the dialysis based on information input from the change amount forming means B2101, the change amount forming means 2061 and the dialysis information acquiring means 208 via the electrical lead wire 206b, the electrical lead wire 208a and the electrical lead wire 210B to the regulating means 202b via the electrical lead wire 205a.

For example, if the blood flow amount in the hollow fiber filter of the blood processing means 203 is smaller than the blood flow amount occurring by the blood driving means 202a and it cannot be said that the efficiency is in good state (FIG. 4), the individual difference information management processing means 205 outputs a signal to increase the blood driving amount to the regulating means 202b. Also, the individual difference information management processing means 205 outputs a signal to increase a circulation rate of the dialysate flowing outside the hollow fiber filter of the blood processing means 203 to the circulation driving section 207d of the dialysate treatment section 207, and the circulation driving section 207d increases the dialysate's circulation rate, for example, based on this signal. By an increase in dialysate's circulation rate, a negative pressure is formed against blood flowing in an opposite direction from the direction of a flow of the dialysate in the hollow fiber filter, which accelerates dehydration and thus may improve dialysis efficiency in some cases.

Furthermore, the changing dialysis information is monitored by this driving amount monitors, the individual difference information management processing means 205 outputs a regulation control signal to the regulating means 202b via the electrical lead wire 205a so that a state in which the difference between the dehydration speed value and the PRR value is equal to or smaller than the predetermined value can be maintained.

The individual difference information management processing means 205 outputs a regulating signal for regulating the driving amount of the dialysate treatment driving section pump of the dialysate treatment section 207 via the electrical lead wire 205d.

The regulating means 202b supplies electrical energy for causing the blood driving means 202a to perform driving corresponding to the regulation control signal.

The individual difference information management processing means 205 may further output an instruction to use an optimal dialysis machine for the patient as an electric signal to the blood processing means 203 via the electrical lead wire 205b. The individual difference information management processing means 205 sends and stores dialysis information and blood flow information and dialysis treatment information optimized for each patient to the input/output storage means 209 via the electrical lead wire 209a. These pieces of information may be data for electronic medical records.

The individual difference information management processing means 205 outputs blood flow information and blood flow state (PRR value, dehydration speed value, etc.) of the blood circuit 200 based on dialysis information to the input/output storage means 209 via the electrical lead wire 210a and displays the same to a doctor, a nurse, a medical staff or the patient. At that time, the blood pressure value and other biological signals may be separately measured continuously or intermittently, and blood pressure data may be acquired via the input unit 205c.

Furthermore, the individual difference information management processing means 205 transmits a regulation command signal to the regulating means 202b based on the input end 205c such as a blood pressure value, a signal indicating the patient's condition, and the like.

The regulating means 202b outputs to the second regulating part 202b3 or the blood driving means 202a based on the input regulation command signal.

For example, when blood pressure information is input from the input end 205c so that the blood pressure value is changed in the lowering direction, and when the dehydration amount increases and thus it is determined that the balance with the PRR value is about to be impaired, the individual difference information management processing means 205 regulates the driving amount of the blood driving means 202a or outputs a signal for supplying a concentration regulating solution to the blood circuit from the second regulating part 202b3.

With the supply of this concentration regulating solution, by increasing the blood concentration in blood vessels for example, it is possible to promote an inflow of an interstitial fluid into the blood vessel and regulates the change of the PRR value and the dehydration speed value subserviently.

In this example, since dialysis corresponding to the individual difference for each patient is enabled, the parameters necessary therefor may be transmitted to a smartphone or a mobile phone when the patient is in the dialysis medical institution in a remote area or when the patient carries the dialysis equipment, so that an optimized dialysis from patient to patient is enabled.

As described above, the individual difference information management processing means 205 outputs an electric signal to the regulating means 202b and the second regulating part 202b3 and performs an automatic driving based on the electric signal. However, the invention is not limited thereto, and for example, the individual difference information management processing means 205 may output a signal indicating that regulation is necessary, and based on this signal, a doctor or a nurse manually may regulates the blood driving means 202a, and second regulating part 202b3.

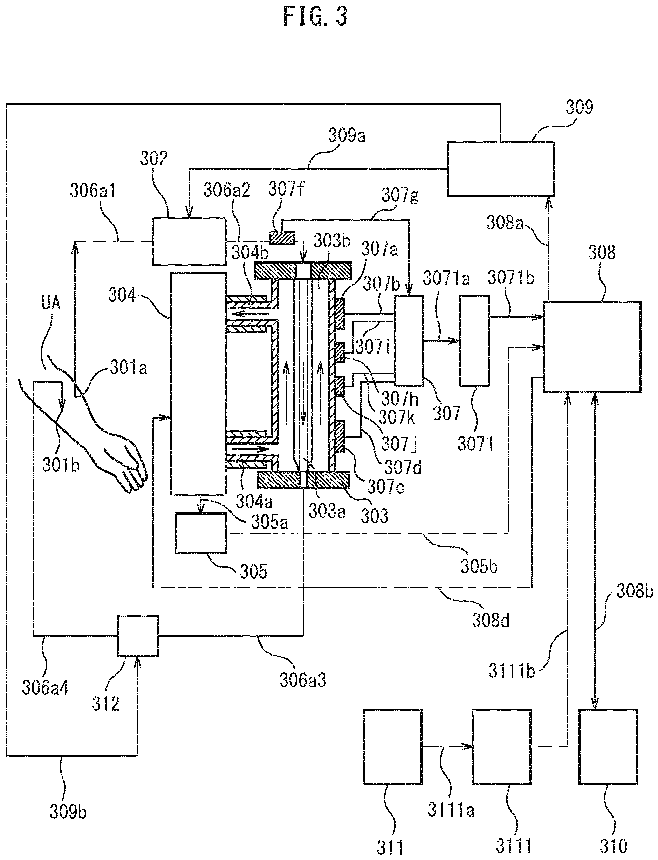

Next, another example will be described with reference to FIG. 3.

In FIG. 3, reference sign 301a denotes a blood removing part and forms an outlet for taking out blood from the artery of the upper arm (UA) to the outside of the living body. Reference sign 301b is a blood returning part and forms an entrance for returning the dialyzed blood to the vein. For example, it is a portion formed by puncturing a hollow needle into a blood vessel after a shunt state that joins arteries and veins together is formed.

Reference numeral 302 denotes a blood pump, and for example, a rotary member by electric input such as a roller type pump which forms a blood flow by the rotation of an impeller is exemplified. The rotating member is preferably in a state in which regulation such that the rotation speed can be varied by regulating the electricity input.

Reference signs 303 denotes a dialysis machine, which includes a hollow fiber filter 303a formed by bundling a plurality of hollow fibers formed of a porous member at the center of a cylindrical casing, and a dialysate flow path 303b, in which dialysate flows in an opposite direction from a direction of flow of the blood.

Reference numeral 304 denotes a dialysate circulating section, which includes a driving section for circulating the dialysate in the dialysate flow path 303b, and a dialysate circuit configured to remove waste matter and dehydrating component coming out from the hollow fiber filter 303a and returning the same back to the dialysis machine 303.

The dialysate circulating section 304 circulates the dialysate and includes a dialysate circulating drive pump (not illustrated) for regulatively performing dialysate circulation which may apply a negative pressure to the hollow fiber filter 303a, and may regulate the driving amount of the dialysate circulating drive pump and regulate the dehydration speed and dehydration amount in some cases.

Reference sign 304a denotes a conduit Ta, which connects the dialysis machine 303 with the dialysate circulating section 304 and forms a flow path for delivering dialysate from the dialysate circulating section 304 to the dialysis machine 303.

Reference sign 304b denotes a conduit Tb, which connects the dialysis machine 303 with the dialysate circulating section 304 and forms a flow path for returning dialysate from dialysis machine 303 to dialysate circulating section 304.

Reference sign 305 denotes a dialysis information detecting means, and is means for inputting sensor signals from a flow rate sensor disposed in the dialysate circulating section 304 and a sensor for component analysis via an electrical lead wire Ra305a and outputting data for obtaining the dehydration speed, dehydration amount, etc. to an individual difference information processing section 308 via an electrical lead wire Rb305b. Note that when collecting information from the dialysate circulating section 304, sensors that come into contact with dialysate and output an electric signal may be included.

Reference sign 306a1 denotes a conduit Ka, and is preferably includes an antithrombotic tube, and connects the blood removing part 301a with the blood pump 302. Reference numeral 306a2 denotes a conduit Kb, formed of the same material as the conduit Ka306a1 and is connected to the blood pump 302 and the hollow fiber filter 303a of the dialysis machine 303. Reference sign 306a3 denotes a conduit Kc1 and forms a blood flow path between the hollow fiber filter 303a and a second regulating part 312. Reference sign 306a4 is a conduit Kc2 and forms the blood flow path between the second regulating part 312 and the blood returning part 301b.

Reference sign 307 denotes a blood flow detecting means, which consists of a non-contact measurement blood flow meter such as a laser blood flow meter, an ultrasonic blood flow meter, a hematocrit meter, or a contact measurement blood flow meter. The obtained data is converted into computable data and outputs the data to the change amount detection means A3071 via an electrical lead wire 3071a.

Reference sign 3071 denotes a change amount detection means A, which is configured to perform a differential operation of blood flow data at a predetermined time interval (for example, 1 msec or more) to input blood flow data to form change amount data, and output the change amount data together with the original blood flow data, to the individual difference information processing section 308 via an electrical lead wire 3071b.

Reference sign 307a denotes an upper sensor and reference sign 307c denotes a lower sensor. The upper sensor 307a is connected to blood flow detecting means 307 via a combination of the electrical lead wire and an optical fiber or the connection line 307b formed by a light guide such as the optical fiber or the like. The lower sensor 307c is connected to the blood flow detecting means 307 via a connection line 307d formed by a combination of electrical lead wire and optical fiber or a light guide such as optical fiber.

In the upper sensor 307a and the lower sensor 307b, a laser light output part and a light receiving section are formed in pair, and when there is a light emitting laser in the blood flow meter device, the configuration near an end of the laser light output part is formed by an end portion of the optical fiber a polarizing plate, a prism, a lens and the like, and a configuration near and end of the sensor when the sensor for the light receiving section provided in the device is formed by the end portion of the optical fiber, a polarizing plate, a prism, a lens, and the like.

In addition, a configuration in which a laser light output semiconductor is disposed on the laser light output part, and the laser light receiving semiconductor is disposed on the laser light receiving part, respectively to function as a pair of semiconductor sensors is also applicable.

The upper sensor 307a and the lower sensor 307c preferably have a configuration for independently detecting the blood flow related signals, respectively, and has an irradiation port for irradiating the laser light and a light receiving part for receiving return light reflected from an object to be measured such as blood integrally. For example, a two channel type single laser blood flow meter which can measure blood flows, for example, at two locations simultaneously may be used.

The upper sensor 307a and the lower sensor 307c are connected by being adhered to the outside of the cylindrical portion of the dialysis machine 303 and are installed in two positions, that is, the upper part and the lower part of the dialysis machine 303. However, the invention is not limited thereto, and a sensor may be provided at a center to measure with three channels, and furthermore, the sensor or sensors may be provided on the outside of one or more of the conduit Ka306a1, a conduit Kb306a2, a conduit Kc1306a3, and a conduit Kc2306a4.

In any case, the sensor may be any position as long as at least the blood flow information can be measured accurately, and furthermore, it can be placed at a position where the dehydration amount or the like can be measured in some cases.

Reference sign 307f denotes a hematocrit sensor, which includes a combination of a light emitting diode configured to output near infrared light and a light receiving semiconductor element, is mounted on a surface of the conduit 30a. A light receiving semiconductor element receives a reflected return light which is output from the light emitting diode and reflected by a blood component in the conduit 30a, photoelectric converts and output the received light to blood flow detecting means 307 via an electrical lead wire Rh307g. The hematocrit sensor 307f may be mounted on the side surface of the dialysis machine 303 for use in the same manner as the upper sensor 307a and the lower sensor 307c.

Note that the hematocrit sensor 307f may be unnecessary when a value corresponding to the rate of change of the circulating blood volume can be obtained by the blood flow meter sensor mounted in the longitudinal direction of an outer periphery of the dialysis machine 303. The reasons are as follows. Values obtained by the blood flow meter sensors 307a to 307d are the blood flow velocity, and the object to be measured is the blood flow in the interior of the fixed hollow fiber. Therefore, the blood volume at the site measured by sensor is expressed as a product of the cross sectional area of the hollow fiber and the blood flow velocity. Accordingly, the rate of change of this blood volume roughly corresponds to the rate of change of circulating blood volume. Therefore, by obtaining the concentration from the blood flow information of the dialysis machine 303, the state of blood pressure can be detected in some cases.

Reference sign 307h denotes a central sensor 1, and is for example, a laser blood flow type sensor arranged at the center of the dialysis machine 303, which includes a pair of laser output part and light receiving part, and is connected to the blood flow detecting means 307 via a connection line 307i formed by a light guide such as an optical fiber.

Reference sign 307j denotes a central sensor 2, which is a sensor for laser blood flow type placed, for example, at the center of the dialysis machine 303, and includes a pair of laser output part and light receiving part, and is connected to the blood flow detecting means 307 via a connection line 307k formed by a light guide such as an optical fiber.

The blood flow sensor is not particularly limited as long as it is a site on the dialysis machine 303 where information for making blood flow efficiency regulatable is obtained. For example, the blood flow velocity, blood flow amount, and the like in the dialysis machine 303 may be acquired from an average of the blood flow information obtained from a plurality of blood flow sensors, in some cases. Also, four blood flow sensors are disposed at equal intervals or at predetermined intervals in this configuration. However, the invention is not limited thereto, any configuration is applicable as long as the blood flow in the dialysis machine can be measured using one or more sensors.

Note that by using a plurality of blood flow sensors to calculate the difference of the blood flow amount between the individual blood flow sensors, the change amount of the blood flow velocity and the blood flow amount are obtained, and further information related to the blood can be obtained.

Reference sign 308 denotes an individual difference information processing section, which is configured to output a signal for optimizing the rotation speed of the blood pump, for example, to the regulating means 309 based on input blood flow information and dialysis information.

In addition, the individual difference information processing section 308 outputs an optimizing electric signal for regulating the driving amount and the dehydration amount of the circulation driving section and the dehydration amount regulating section, which constitute the dialysate circulating section 304, to the dialysate circulating section 304 via an electrical lead wire Ri308d. By the regulation of the driving amount of the circulation driving section regulates the negative pressure amount for the hollow fiber filter inside the dialysis machine 303, and also regulates the dehydration amount. Also, the dehydration amount is regulated by regulating the amount of bodily fluid extracting from inside the circulation circuit to outside.

Also, the individual difference information processing section 308 inputs change amount data from change amount forming means A3071 and change amount forming means 3111. For example, when the change amount of the peripheral vascular resistance related value exceeds a predetermined threshold, or when the time exceeding the predetermined threshold is increased and furthermore, when a calculated value exceeds a predetermined threshold value as a result of referencing the value of the change amount of other biological information data and calculating the possibility of the blood pressure variation, the individual difference information processing section 308 outputs a regulating signal for regulating the blood circulation amount, the additional supply of a fluid replacement, the dialysate circulation amount, and the like to the regulating means 309 and the dialysate circulating section 304.

The circulation driving section illustrated in FIG. 3 corresponds to the circulation driving section 207d in FIG. 2.

Reference sign 309 denotes a regulating means, which is connected to the blood pump 302 via an electrical lead wire Re309a, and is connected to the individual difference information processing section 308 and an electrical lead wire Rd308a, and, upon reception of an input of digital or analogue electric signal for commanding regulation from the individual difference information processing section 308, for example, electrically outputs the rotation speed of the blood pump 302 based on the content of the electric signal for commanding regulation in a digital or analog form.

Reference numeral 310 denotes input/output storage means, which has the same configuration as the input/output storage means 209 illustrated in FIG. 2, and thus description will be omitted.

Reference sign 311 denotes biological information input means, which has the same structure as the biological information input means 210 illustrated in FIG. 2, and is configured to convert the input biological information into computable data and output the computable data to a change amount forming means B3111 via an electrical lead wire 3111a.

Reference sign 3111 denotes a change amount forming means B, which is configured to calculate a change amount (difference calculation) of the input biological information data at a predetermined time interval (1 msec -) to form a change amount data, and outputs the change amount data to an individual difference information processing section 308 via an electrical lead wire 3111b.

Reference sign 312 is a second regulating part, which includes a device for regulating blood concentration, for example, by supplying a concentration regulating solution such as saline water, glucose solution, physiological saline to circulating blood in the in vitro blood circuit. The second regulating part 312 is electrically connected to a regulating means 309 via an electrical lead wire 309b and is capable of supplying a concentration regulating solution to the blood circuit based on an electric signal from the regulating means 309 and increasing an inflow amount of the interstitial fluid into the blood vessel with blood at a high concentration which is returned to the blood vessel from a blood returning part 301b, so that regulation of the PRR (plasma refilling rate) value is achieved. The concentration regulating solution may further contain a blood component such as magnesium.

Next, an operation of the example illustrated in FIG. 3 will be described.

The blood removing part 301a for removing blood from the artery of the patient upper arm and the blood returning part 301b for returning the dialyzed blood to the living body are formed.

The blood pump performs a rotational drive to send blood from the blood removing part 301a to the dialysis machine 303 at a constant speed.

The blood arriving at the dialysis machine 303 by the driving of the blood pump 302 flows in the hollow fiber filter 303a, a waste matter or the like is taken into dialysate flowing in the dialysate flow path 303b from the hole on the wall surface and moves to the dialysate circulation means.

With the circulation of dialysate, the dialysate containing removal of waste matter and bodily fluid from blood including bodily fluid from the blood flowing in the hollow fiber filter 303a flow into the dialysate circulating section 304 via the dialysate flow path 303b via a conduit Tb304b.

The dialysate circulating section 304 separates waste matter and separates and eliminates extra bodily fluid. In some cases, if there is an ingredient necessary for the living body that has been separated and removed as waste matter, a replacement fluid supply section may be formed to replenish the ingredient, or the second regulating part 312 may be use in place of the replacement fluid supply section.

Furthermore, the dialysate circulating section 304 outputs bodily fluid information via the electrical lead wire Ra305a to the dialysis information detecting means 305, and which calculates the dehydration speed and the dehydration amount from the water component separated as extra bodily fluid.

The dialysis information detecting means 305 outputs data for calculating dialysate information such as the dehydration speed, the dehydration amount, the PRR value, the hematocrit value and the like to the individual difference information processing section 308 via the electrical lead wire Rb305b.

The upper sensor 307a and the lower sensor 307c disposed by being adhered to the outer side surface of the dialysis machine 303 transmit the blood flow information of the blood in the dialysis machine 303, mainly in the hollow fiber filter to the blood flow detecting means 307.

When the upper sensor 307a and the lower sensor 307c are sensors for a laser blood flow meter, for example, the blood flow detecting means 307 irradiate the blood flowing in the hollow fiber filter 303a of the dialysis machine 303 with laser light via the upper sensor 307a, detects reflected return light from the blood component by the upper sensor 307a and the central sensor 307h and transmits the result to the blood flow detecting means 307.

Further, the blood flow detecting means 307 irradiates the blood flow flowing through the hollow fiber filter 303a of the dialysis machine 303 with laser light via the lower sensor 307c, and detects and receives the reflected return light from the blood component by the lower sensor 307c. If the signal transmitted from the lower sensor 307c is light, the blood flow detecting means 307 converts the light into an electric signal by photoelectric conversion, obtains blood flow information, and sends it via the electrical lead wire Rc307e to a change amount forming means A3071.

Also, as regards the connection line A307b, when the upper sensor 307a is provided with a photoelectric conversion type sensor and light irradiation means, the reflected return light from the blood component is output from the sensor in a form of an electric signal by photoelectric conversion. Therefore, a connection line A307b is an electrical lead wire, and is formed by a combination of a light guide and an electrical lead wire. Similarly, as regards the connection line B307d, when the lower sensor 307c is provided with a photoelectric conversion type sensor and light irradiation means, the reflected return light from the blood component is output from the sensor in the form of an electric signal by photoelectric conversion. Therefore, a connection line B307d is an electrical lead wire, and it is formed by a combination of a light guide and an electrical lead wire.

The individual difference information processing section 308 converts data from a detection unit connected to a biological information input section 017 in Fig. 1 such as facial color data, temperature data, peripheral vascular resistance related data of the patient input from the change amount forming means B3111 via the electrical lead wire 3111b, and an electric signal input via the electrical lead wire Rc307e into an electric signal for information processing and analyzes the converted electric signal for information processing.

FIG. 9 is a chart indicating a blood flow amount for each distance from a blood inlet of the dialysis machine and indicating values of the flow rate including the blood flow velocity and blood flow rate detected from each probe of the laser blood flow meter probe 307a mounted on the side surface of the dialysis machine 303.

In FIG. 9, as blood pressure is input to the dialysis machine entrance by driving the blood flow pump, a bodily fluid in the blood leaks sequentially from the hollow fiber gap. Therefore, a positive filtration state in which the blood volume gradually decreases is achieved. On the other hand, the dialysate input from the direction of the blood outlet has a high pressure, and the pressure of the blood flow in the blood circuit is decreasing. Therefore, dialysate enters the blood circuit and flow rate increases (back filtration state). As a result, as shown in FIG. 9, an envelope curve having a minimum value in the downward direction is drawn. In this example, this minimum value LP is calculated.

In other words, from the blood flow value, an interpolation of the spline curve or the like is applied to achieve an envelope curve state. The flow rate such as the blood flow velocity and blood flow amount of the minimum value LP of this envelope is stored in temporary.

The intersection coordinates of a straight line 901 passing through LF1 and LF2 and a straight line 902 passing through LF3 and LF4 may be set as the coordinates of the minimum value LP without interpolation.

The change in the blood concentration value is detected by the change of the minimum value LP, and the fluid replacement volume and driving amounts of the blood pump and the dialysate pump are regulated while comparing with the preset proper concentration value, and regulation such that the minimum value LP fits in the appropriate range is performed.

As there are individual differences in proper concentration values, regulation is performed so that the blood concentration value becomes appropriate for patients while detecting changes in other biological information over time.

Storage is performed temporarily or over time (per predetermined time interval) in a computer memory (hard disk, USB memory, etc.) or the like.

This minimum value LP is the minimum positive filtration value of the internal filtration amount, and the value moves downward according to blood concentration. Since the change amount $\Delta LP$ of this movement corresponds to the magnitude of the dehydration amount, setting is made to suggest possibility of change in the blood pressure when the change amount becomes larger and exceeds the predetermined value, and an alarm display or the like is displayed, for example, on the computer monitor.

In addition, the individual difference information processing section 308 receives the electric signal regarding the dehydration speed and the PRR value from the dialysis information detecting means 305 via the electrical lead wire Rb305b. Therefore, when it is determined that the blood flow amount shown in FIG. 4 is lower than the flow velocity of the blood pump thus it is one of the factors lowering the dialysis efficiency from the change amount of the blood flow amount and the blood flow velocity in the dialysis machine 303 output from the change amount forming means A3071 via an electrical lead wire Rc3071b, the individual difference information processing section 308 outputs an electrical signal which increase or decrease the rotation speed of the blood pump 302 to the regulating means 309 signal via the electrical lead wire Rd308a. Further, in some cases, an electric signal for causing the dialysate circulating section 304 to further apply a negative pressure on the hollow fiber filter inside the dialysis machine 303 is output via the electrical lead wire Ri308d. The dialysate circulating section 304 increases the dehydration amount by driving to increase the negative pressure amount of the internal circulating fluid drive pump, and increases the dialysis efficiency.

Based on this regulating signal, the regulating means 309 outputs a signal for regulating the driving amount of the blood pump 302 to the blood pump 302 via the electrical lead wire Re309a.

When a signal for regulating the driving amount of the blood pump 302 is output the regulating means 309, the individual difference information processing section 308 monitors the dialysis rate information output from the dialysis information detecting means 305 via the electrical lead wire Rb305b and whether the difference from the PRR information is within a predetermined range. The individual difference information processing section 308 then suppresses the driving amount of the blood pump 302 when the difference between the dehydration speed and the PRR value exceeds in the direction in which the dehydration speed increases, and conversely outputs a signal for performing an operation for increasing the driving amount of the blood pump 302 to the regulating means 309 when the dehydration speed falls below the PRR value and falls further below the predetermined range. Accordingly, an optimal blood flow according to the individual difference is formed and dialysis efficiency according to individual difference is improved.

The individual difference information processing section 308 outputs and stores the optimization regulating signal data, the dialysate information, and the blood flow information to the regulating means 309 to the input/output storage means 310 via an electrical lead wire Rf308b, and causes to displays it based on the necessity.

Electrical lead wires indicated from an electrical lead wire Ra305a to an electrical lead wire Rg308b indicate electrical connection and include those formed by a plurality of conductive wires such as a multiconductor cord for a data bus, as well as wireless connection such as Wi-Fi, infrared, Bluetooth (registered trademark).

The upper sensor 307a and the lower sensor 307c and the central sensor 307h preferably perform sensor output to the blood flow detecting means 307 during dialysis treatment on a real time basis and measure the blood flow amount and the blood flow velocity over time. The diameter of the erythrocyte is around 8 $\mu$m, whereas the pore diameter of the hollow fiber filter 303a is around 200 $\mu$m to 150 $\mu$m. Since it can form a rheological field to some extent for erythrocyte, it may be possible to capture changes in blood concentration due to dehydration progression or the like with changes in blood flow amount. The individual difference information processing section 308 determines that the dehydration progress is too large when the inclination is directed in a decreasing direction in the change of the change amount data of the blood flow amount from change amount forming means A3071 over time, and outputs a signal issuing an alarm to a doctor, nurse, etc., or may output a signal for decreasing the driving amount of the blood pump 302 to the regulating means 309, or may output the signal which administers the concentration regulating agent to the second regulating part 312 in some cases.

Note that the rheological state of the blood of the hollow fiber filter 303a in the dialysis machine 303 may be diluted, but may differ depending on the patient, and furthermore, there can be individual differences in the change over time. Therefore, it may be preferable to store these state in the input/output storage means 310 as data for each patient, and cause the individual difference information processing means 308 to call input blood flow information and data for each patient from the input/output storage means 310 during dialysis treatment, and output a signal for performing an optimized regulation to the regulating means 309 in some cases.

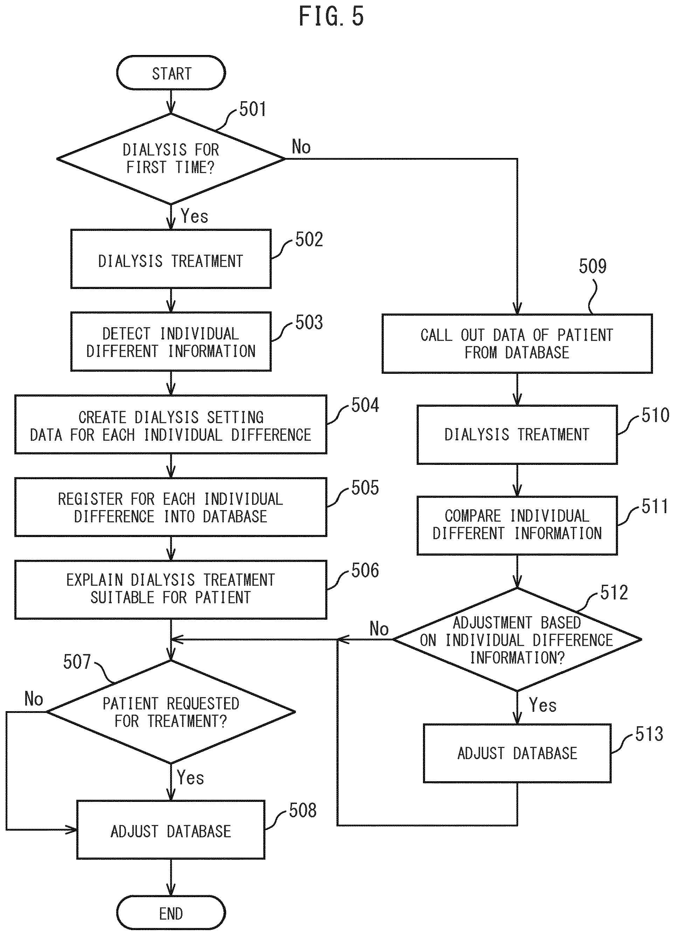

Next, another example of the present invention will be described with reference to FIG. 5.

In the flowchart of FIG. 5, Step 501 is a step of judging the initial dialysis treatment, and, for example, in the individual difference information management processing means 205 illustrated in FIG. 2, is a step of specifying an interface such as a selection screen or the like to be displayed on a monitor of the computer configuring the individual difference information management processing means.

Examples of the selection of step 501 include a case where a doctor, a nurse, a clinical engineering technician, etc. specifies and determines with a switch installed in the device or an icon on the computer monitor screen with a computer mouse, a touch pad, or the like.

Step 501, may include not only determining the first dialysis treatment, but also, in the case of the first dialysis treatment, inputting the patient data, and the input patient data may be formed by the electronic medical records specification and the like, and registered in the input/output storage means 209 as illustrated in Fig. 2.

In the case of the first dialysis treatment, inspections to a degree that can set parameters, such as inspection of rheology such as blood component examination, blood fluidity, viscosity, and deformability of erythrocyte, lifestyle examination by inquiry and the like, presence/absence of chronic diseases, anemia, and the like.

Also, facial color image of patient, temperature data, humidity data, body movement pattern, face expression pattern, peripheral vascular resistance related value, blood pressure value, blood flow value, heart rate and their change amount, and the like are also measured. Similarly, it is preferable to perform measurement of peripheral blood flow such as blood pressure, body temperature, limbs and the like in the second and subsequent times as well. These examination data are accumulated in, for example, database 010 illustrated in in FIG. 1 or input/output storage means 209 shown in FIG. 2, and is compared and collated with the inspection data of the registered patient. Then, dialysis treatment in which control is performed with various regulating parameters such as the dehydration speed and the dialysate circulation rate is preferably performed.

Examples of the regulating parameters include, for example, the rotation speed of the blood pump, the rotation speed of the dialysate circulation pump, the replacement fluid component, the amount of the replacement fluid component, the replacement fluid supply timing, the selection number of the dialysis machine, and the like and preferably also include the time series data.

Step 502 is a dialysis treatment step, indicating the dialysis treatment from the start to the end. During this dialysis treatment, dialysis treatment based on parameter values such as dehydration speed, dehydration amount and the like set in step 501 may be performed. The parameters during the initial treatment may be those set based on prior patient examination data.

In the dialysis treatment step 502, blood flow information is sequentially detected from four blood flow sensors 307a, 307c, 307h, and 307j connected to the dialysis machine 303 illustrated in FIG. 3, and in the blood flow detecting means 307, the blood flow amount, the blood flow velocity and the change amounts of their difference values are calculated and output to the individual difference information processing section 308. The individual difference information processing section 308 performs blood flow data processing as illustrated in FIG. 4 and FIG. 9, records in real time basis as the individual difference data of patient, and monitors whether the change is abnormal or not.

Preferably, the individual difference information at the initial dialysis searches the database for similar examination data based on the examination data and sets the parameters.

For example, when a predetermined threshold values are set and if the blood flow value and its difference change value fall below those threshold values, there may be a case where the blood concentration changes in a high direction depending on the individual difference of the patient, so that the possibility of the decrease of the blood pressure value becomes high. This state is referred to as abnormality of the change.

Further, it includes the change in the color of the face, decrease in temperature of the fingertips, and especially when the decrease is abrupt in a unit time or the change occurs upward and downward.

In addition, monitoring information on the change amount of peripheral vascular resistance related value by intermittent or consecutive pulse wave measurement by a pulse wave meter attached to the fingertip for measurement may be used together to detect the state in which the possibility of the decrease of the blood pressure value becomes high.

The pulse wave signal of the part close to the distal end such as the fingertip is the measurement far from the heart and in addition, it is the relative measurement. Further accurate pulse wave can be measured by sandwiching photoreceptor unit using an infrared LED unit provided with a plurality of infrared LEDs and a photoreceptor unit using a plurality of phototransistors with upper arm.

Note that when using a plurality of infrared light emitting diodes and infrared receiving photo sensors, infrared is irradiated in a wide range and light is received over a wide range. Therefore, connection is made in parallel, circumference, radiation state, and transmitted infrared light is received with respective light receiving signals while irradiating infrared at once. Alternatively, when converting the light receiving signal into the electric signal, the signal with this added thereto may be used.

By being able to detect the possibility of the decrease in blood pressure value, treatment for such decrease in blood pressure such as additional supply of replacement fluid can be done quickly, and thus a gentle dialysis treatment for the patient is achieved.

Step 503 is a step of detecting parameter values such as temperature data, humidity data, peripheral vascular resistance related value data, dehydration speed, PRR value, etc. corresponding to the patient's individual difference during dialysis treatment for the first time, creating individual setting data during dialysis treatment formed by, for example, and adding time data to the detected parameter values.

Step 504 is a step of creating dialysis setting data for each individual difference, and the parameters set in step 503 have the possibility of being rewritten for optimization during dialysis.

Parameters set in step 504 are optimal values of dialysis speed value and dialysate speed value in respective values such as patient's temperature values/humidity values, blood flow values, blood pressure values, peripheral vascular resistance values and minimum values illustrated in FIG. 9, and the step preferably includes a step of determining values sensitively corresponds to the blood pressure change with ranking attached thereto. This ranking is preferably performed based on the collected data in a plurality of dialysis treatments at the time of initial setting. However, in measurements other than dialysis treatment, patient database may be searched based on the measured values and estimated based on data of patient approximated to measured values, or may be corrected based on the results of treatment based on the estimate.

In other words, variations in blood pressure do not necessarily immediately correspond to a change in the change amount ΔBV value of the circulating blood volume, and from the compensation processing such as an error in the value of the Crit-Line monitor for obtaining the ΔBV value, the blood pressure which is immediately correspond to the blood pressure change cannot necessarily be obtained. Therefore, for each patient, the relationship between these parameters and the change in blood pressure value during dialysis may be recorded, or approximate data from patient database may be selected and set in some cases.

Step 505 is a step of registering and storing the set parameters as individual difference data of patients as database on continuous recording device of computer. This saved data may be stored in a state of being usable for parameter setting in other dialysis treatment occasions in a manner accessible from the outside such as server.

Step 506 is a step of explaining the dialysis treatment suitable for patients, in which explanation of the patient's individual difference is displayed on a computer monitor or on a paper with the obtained blood flow velocity, blood flow amount and the like. In addition, necessity of the dialysis treatment to explaining the necessity of dialysis treatment in situations which dialysis patient does not like so much such that the period of dialysis treatment, which is normally 4 hours or so will be prolonged, but the dialysis is gently carried out. Note that this step 506 may be performed not only for a new dialysis treatment patient but also for a patient who repeatedly performs dialysis treatment, if there is a significant change in parameters, or the like.

Step 507 is a step of request for treatment such as desire to ameliorate symptoms such as sagging remains or headaches appear after dialysis treatment, in which a doctor or the like listens directly from the patient, or the patient inputs and transmits a request for treatment through a computer terminal.

Step 508 is a database regulating step of recording the contents requested in Step 507 and calling and changing and recording again the parameters recorded for setting the parameters for the nest dialysis treatment.

For example, in the case of a request for improvement of headache after treatment, if it is a headache due to concentration of bodily fluid in the brain, which is caused because the blood circulation in the brain is slow, and hence blood with high concentration containing waste matter in the brain even after the blood in the body is purified, the cause is too fast dehydration speed in many cases, and thus the operation parameter values for regulating the dehydration speed is regulated.

Depending on the patient, even if the blood pressure value is constant, in a case where the peripheral vascular resistance related value changes, the temperature of the limbs changes, and the change exceeds a certain threshold value, and in a case where the patient yaws many times, and when the symptoms of low blood pressure is detected from the image, it is determined to be a state equal to the low blood pressure considering the individual difference, and the operating parameters for regulation of the dehydration speed and administration of the replacement fluid are adjusted.

In the case of dialysis treatment of the second and more times, Step 509 is a step of calling patient's operating parameters and other dialysis data from database.

In the second and more treatment, dialysis treatment may be performed based on patient-specific data stored in a database already formed in a storage device such as a computer or a server. However, the physical condition may change depending on a physical condition, disease, and the like, and blood may also change. Therefore, blood tests or the like may be performed in some cases. Depending on the result of the examination, it may regulate the parameter value used for dialysis treatment.

Step 510 is a step of performing dialysis treatment based on operating parameters for dialysis treatment called from database, and performs dialysis treatment suitable for patient.

Step 511 is a step of reading and comparing individual difference information formed from individual differences information measured during dialysis treatment in step 510, sensor information values of previous dialysis treatment and the like. In this step, individual difference information is collected during dialysis treatment as well, and when there is abnormality or the like, the parameters such as dehydration speed is changed and regulation of dehydration speed is performed. Furthermore, the whole individual difference information and the individual difference information of the previous dialysis stored in database are compared.

Step 512 is a step of adjusting the parameter values for improvement when the parameter values based on the individual difference information need to improve.

Step 513 is a step for adjusting the database, and is for rewriting and registering the parameter value etc. changed by the current dialysis treatment.

In FIG. 5, when a patient receives the dialysis treatment for the first time (yes at 501), the patient receives dialysis treatment based on the example in FIG. 3 (502).

From the blood flow information obtained from the upper sensor 307a and the lower sensor 307c set at the time of treatment, for example by being adhered to the dialysis machine 303 illustrated in FIG. 3, the blood flow information detecting means 307 calculates the blood flow velocity and the blood flow amount. From the electric signal obtained from the hematocrit sensor 307f, the hematocrit value and the like are calculated, then converted into data, and input to the individual difference information processing section 308. Further, the PRR data, the dehydration speed, and the other dehydration related data are input from the dialysis information detecting means 305 connected to the dialysate circulating means 304, and dialysis information such as peripheral vascular resistance related value data is input from the biological information input means 311, both into the individual difference information processing section 308 as individual difference information (503). Note that such dialysis information are not necessarily output from the dialysis information detecting means 305, but may be output from the blood flow information detecting means 307 in some cases.

The illustrated individual difference information processing section 308 illustrated in FIG. 3 creates another dialysis setting data for each individual difference (504).

Dialysis setting data for each Individual difference indicates information regarding the hollow fiber filter or the like depending on the state of blood of the patient based on, for example, the illustrated blood flow amount data, blood flow velocity data, hematocrit data in FIG. 4, the PRR value obtained from these data, dehydration speed value, and limb peripheral blood flow data measured therewith, peripheral vascular resistance related value data, blood pressure data, temperature data, humidity data, facial color image data, and other data effective as the individual difference information for dialysis.

These individual difference information is registered in a patient-specific database (505).

After that, explanation to the patient is made about dialysis treatment suitable for the patient, regarding relief of pain such as pair for each individual difference, healing of gangrene of distal portion of the extremities, reduction of risk of complications on cardiac blood vessels, dialysis treatment for desirable life prognosis. For example, explanation of dialysis treatment is based on individual difference information. Therefore, the explanation is for convince the passenger of elongation of dialysis duration, which is normally 3 hours to 4 hours, by a length suitable for the patient, optimization of the number of times of treatment, and the fact that the dialysis is optimum for the patient even though the dialysis treatment is different from those for other patients.

In this example, a configuration for realizing the dialysis treatment for optimization for each individual difference is provided based on the blood flow information, and based on dehydration related information, and thus a doctor, a nurse, or medical staff explain reference information for optimizing duration and the number of times of dialysis treatment to the patient for example, by displaying on the computer display (506).

By having an opportunity to discuss about whether the dialysis treatment can be performed according to the patient's desire by listening requests from the patient based on optimization of the dialysis treatment (507), and if the desire of the patient or information regarding other diseases can be used for the dialysis treatment, the data in the database is adjusted and registered based on such information (508).

In the next dialysis treatment (no in 501), data of patient is called from database in advance before dialysis (509). This is preferably used as data to be used for during dialysis together with electronic medical records including other patient information. For example, selection data of dialysis machine is displayed from the previous blood flow state, and dialysis treatment is performed based on the optimization information (510).

During dialysis treatment, the blood flow data and the dialysis data are collected over time as illustrated in Fig. 3 and detected as individual difference information, and is compared with the previously recorded individual difference information from the database (511). For example, if the blood flow amount in the dialysis machine is different and increasing the blood flow in the dialysis machine is desired, or if dehydration speed is decreased and a process of administration of a concentration regulating solution is performed because the change of the blood flow amount goes downwards over time and dehydration progresses too much (512) etc., information regarding the type of dialysis machine used, information regarding the changes in blood flow amount, information regarding other dialysis machines etc. to be used in the next dialysis treatment are adjusted and registered to the database (513).

Since the dialysis machine can be an illustrated indicator of blood rheological characteristics of each patient, blood flow data obtained from the dialysis machine surface is stored in database as data for dialysis treatment per patient.

Thus, in addition to using the illustrated dialysis system in FIGS. 1 and 2, 3 and 10, by collecting information for each during dialysis treatment, acquiring of requests or the like from the patient, and performing adjustment of the treatment method of the dialysis treatment together with usage of the dialysis system, dialysis treatment appropriate for each patient is enabled.

The patient's request also includes information from the medical institution when further performing diabetes treatment and other medical institution dialysis treatment.

By making it possible to acquire these information via network such as internet, it becomes easy to monitor and record lifestyle habit information after patient returns home, so that the degree of freedom of the patient may be improved by adjusting the dialysis treatment frequency and the like. In addition, by enabling collection of patient individual difference information via internet, dialysis treatment at the remote area is also facilitated, and travel for a long period of time or the like is enabled. In addition, alleviation of dietary limitation and adjustment of the number of times of dialysis and the like are also enabled.

Also, optimization treatment according to individual difference of a patient in dialysis treatment as illustrated to example of the present invention may include an advice for optimization of living environment including the dialysis treatment of the patient and adjustment of living environment.

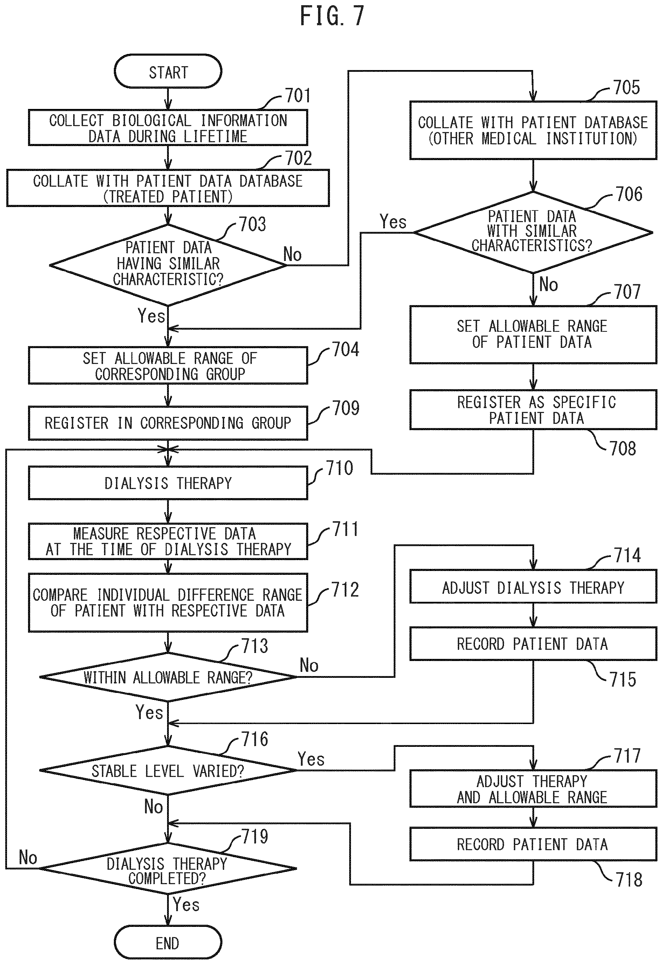

Next, another example of the present invention will be described in detail with reference to the flowchart of FIG. 7.

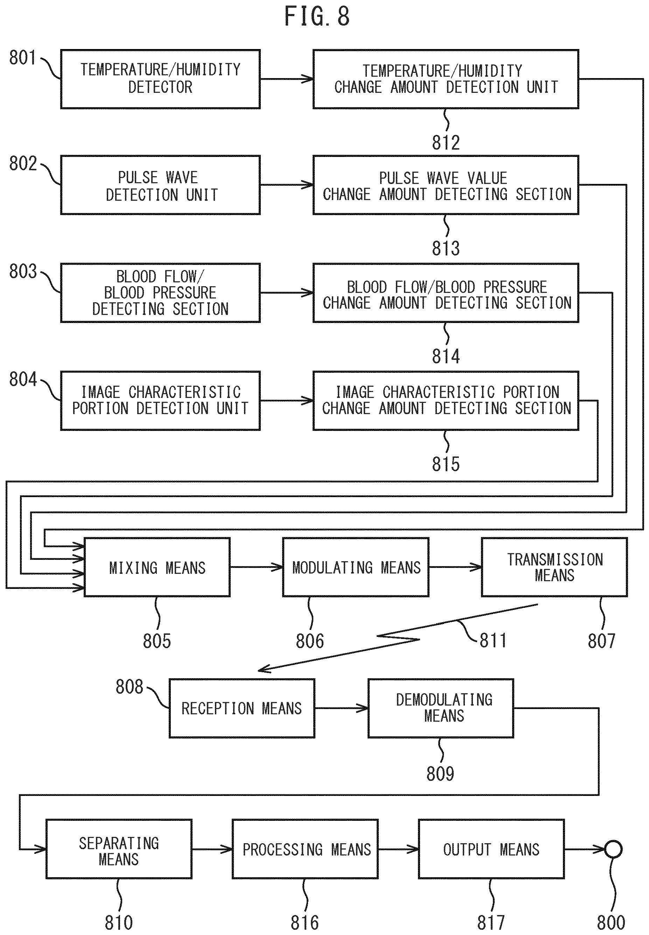

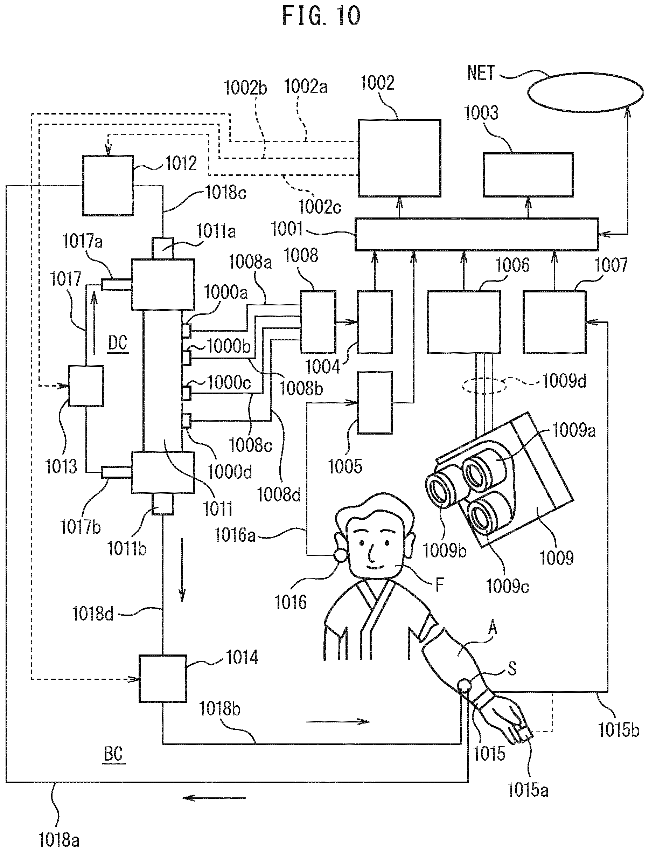

In FIG. 7, Step 701 is a step of collecting biological information data during life time and during dialysis treatment. Sensor information is collected from body temperature sensor, thermography camera photography data and the like illustrated in FIG. 8 or FIG. 10, converted into comparable data such as those capable of being digitized or statically processed, and is stored temporarily and consecutively as chronological data in computer memory.

Here, "comparable" means, for example, data that is not limited to the area but has commonality in form, formats, and the like and includes making it comparable with biological information data obtained from a similar dialysis treatment patient.

Step 702 is a step of collating with the patient data database, and it is compared with the biological information data of the patient mainly receiving the same dialysis treatment. Note that patient data is compared with accumulated data in one facility. This collation is a stage prior to dialysis treatment, and it is preferable to collate at timing such as before receiving dialysis treatment for the first time, before dialysis treatment is performed, or the like.

Step 703 is a step of determining whether there is similar data as a result of comparison with stored data in the patient database.

Step 704 is a step of setting the allowable range, and when there is similar data (Yes) and, for example, when group is divided for patients having similar biological information data, the allowable range of those groups are set.

"Acceptable range" is a range representatively indicated as a range within which a range of change in biological information occurring during dialysis treatment is acceptable, that is, a range in which a gentle dialysis treatment can be performed without distressing the patient, and for example, the range of variation of body temperature and the range of change of peripheral blood flow, and includes the range where gentle dialysis treatment is possible.

Note that the patients in that group do not all have the same tolerance range, average value may be set as a temporary tolerance range and further patient individual difference data (e.g., increase/decrease value) may be added.

Step 705 is a collation of data in patient database set and registered in domestic and overseas therapeutic organizations other than the therapeutic institution in step 702. What is required is only that at least database containing biological information data of numerically comparable patients is accessible and data with values similar to patient's biological information is searchable and detectable. Note that the determination of "similar" is made for example by changes in body temperature, the state of peripheral blood flow in the same life pattern, and the like in everyday life.

Step 706 is a comparison step with patient data called from the patient database of another medical institution.

Step 707 is a step for setting an allowable range of patient data, and when there is no data approximate to any of them, a preset permissible range is provisionally set.

In Step 708, since there is no patient data having another similar biological information, new group is set and registered in database.

Note that since at least several biological information is measured and compared and registered, if there is something that does not approximate as a whole but partly approximates, it is preferable to establish a relationship such that even a new group can be regarded as a relevant group, they are displayed in the patient data as a relevant group when searched.

Step 709 is a step of registering in the corresponding group, and patient data is grouped and registered, for example by appending the code of a group composed of patients having a similar biological information pattern.

Step 710 is a dialysis treatment step, which shows the dialysis treatment which the patients usually receive, and 3 to 4 hours of treatment including even dehydration is exemplified.

Step 711 is a step of measuring biological information during dialysis treatment and biological information is measured continuously or intermittently during dialysis treatment.

More specifically, data obtained by attaching sensor illustrated in FIG. 8 or FIG. 10 or photographing with a thermography camera, facial color detection camera and the like is recorded. For example, peripheral blood flow measurement is measured by a non-contact type laser blood flow meter, the body temperature measured by using a near infrared such as thermography camera, and the humidity of the skin surface is a humidity condition obtained from an image data in a state of being irradiated with an illumination using light sources of a specific wavelength are exemplified.

Also, as the skin dries, the skin temperature also increases, so that drying of the skin may be detected from the temperature change in the thermographic camera image.

In addition, changes in the humidity of the skin such as cold sweat are sometimes measured according to the condition occurring during dialysis treatment.

Note that blood pressure and the like may be a contact type sphygmomanometer using a fingertip or a method using an electrocardiogram.

In some cases, the blood flow information of the dialysis machine (dialyzer) illustrated in FIG. 10 is also used as biological information.

Step 712 is a step of continuing dialysis while monitoring whether data measured from a patient is in an allowable range, and compares whether or not being within the acceptable range continuously and intermittently.

Step 713 is a step of judging whether or not it is within an allowable range. Since it represents the change of the body condition of the during dialysis treatment, if it is not within the allowable range, it predicts the possibility of changing the blood pressure due to the possibility of impairment of the dehydration balance. Therefore, the procedure goes to a dialysis treatment implementation step 714.

A determination step 713 may be a display to the doctor, and Step 714 may be based on the prescription by a doctor or a technician.

Step 714 is a dialysis treatment performing step, and in addition to a step performed by prescription such as a doctor, treatment such as increase and decrease of the rotation speed of the blood pump, increase and decrease of the rotation speed of the dialysate pump, and automatic administration of a predetermined amount of replacement fluid may be automatically performed. The timing of stopping at step 714 is exemplified by, for example, monitoring the change of the point at which the state changes over time as illustrated in FIG. 9, performing control so as not to cause an abrupt change, continuous supply of replacement fluid, and the like.

Step 715 is a step of performing setting which allows widening the allowable range when the condition of the patient is gentle and there is no particular possibility of occurrence of the blood pressure or the like even if it exceeds the allowable range.

Step 716 is a step of confirming the variation of the stability level in which means for consecutively or intermittently measuring the blood pressure of the patient is used depending on blood pressure value or the like indicated by the stability index. For example, it is preferable that an instrument which has less contact point with the patient such as a fingertip sphygmomanometer, and provides less pressurized load is used.

"Stability level" indicates the degree of burden such as patient's blood pressure value, pain, itching, etc. during hemodialysis treatment, and may include the degree of possibility of risk of complications. The stability level indicates the variation rate of state change point illustrated in Fig. 9, the degree of change of sensor data illustrated in Fig. 9, for example.

Step 717 is a step of adjusting the permissible range of patient data and further performing dialysis treatment. It is a step of performing regulating treatment similar to step 714, and regulating the error of the allowable range.

Step 718 is a step of recording adjustment data of this allowable range in database as patient data.

Step 719 illustrates a step illustrating whether or not the dialysis treatment is complete.

Next, the operation of the example illustrated in FIG. 7 will be described.

At the time of daily activity, patient wears a sensor unit illustrated in Fig. 8 or Fig. 10 to collect body temperature data and the (701).

At that time, the face, the hands, the feet etc. are photographed by a camera. It is preferable to perform photographing in a healthy state in advance to obtain image data for a comparison reference.

Before the dialysis treatment, the obtained biological information of the patient is compared with the data group of the patient database (702). This comparison may be performed visually by a doctor, a nurse, a technician or the like, or may be performed by image processing, or computer processing if it is image collation or numerical comparison.

When patient data having similar characteristics are registered, an allowable range of various data at hemodialysis treatment is determined based on this patient data (704).

If approximate patient data cannot be detected in the patient database used for the first time, domestic or overseas database is used and patient data that approximates the range is collated (705).

When there is patient data having similar characteristics (706), from the patient data, setting is performed to belong to a group in which the patient data having an approximate allowable range of the change of the in-vivo information during hemodialysis treatment (707), (704), and is registered (708).

If there is no corresponding group, a group with a new code is set and registered as unique patient data to make a group referred to by a new dialysis patient.

Setting allowable range of variation from biological information detected during dialysis treatment in this manner is adjusted according to individual differences based on the timing and quantity of supply of dehydration, elimination of waste matter, and supply of replacement fluid according to individual differences during hemodialysis, and a temporary value of allowable range is provisionally set to keep the stability level high.

The provisional allowable range set above is adjusted according to the variation of the stability level in the actual during dialysis treatment, and it is set to a range corresponding to the individual difference of the patient.

Next, a dialysis treatment is carried out. In order to perform balanced dialysis treatment in terms of the relationship between the PRR value and the dehydration speed value based on the circulating blood volume, the driving amounts of a blood pump and a circulating fluid pump, and the supply amount of a replacement fluid are regulated while performing optimization (710). St that time, the level value and biological information data of the state change point illustrated in FIG. 9 is measured.

The circulating blood volume takes time to obtain the measured value error and the accuracy of the PRR value. Therefore, the state change point illustrated in FIG. 9 is further detected, and the variation rate per time is calculated. Next, the variation or the like of the dehydration amount within and outside the preset the acceptable range is detected. From this value, optimization of the blood pump, the circulating fluid pump, and replacement fluid supply amount is performed. Therefore, in a step (711) of measuring the respective data during dialysis treatment, the state change point illustrated in FIG. 9 may be detected.

During dialysis treatment also, the sensor information illustrated in Fig. 8 such as body temperature sensor is performed and the variation is monitored (711). If there is a change in the sensor value, whether it is within the acceptable range for each individual difference or not is compared (712).

If it is not within the range, dialysis treatment such as regulates such as blood pump, dialysate pump and driving amount of replacement fluid supply driving part is performed (714), the information at that time is additionally recorded as patient data (715), and regulates the setting of allowable range as needed.

Even if it is within the acceptable range, whether or not the blood pressure value and other sensor information illustrated in FIG. 8 is are in the stability level is determined (716). At that time, if there is a step of judging the patient's pain by monitor data, and it exceeds the stability level, adjustment such as widening allowable range is made (717), and adjustment data is recorded in patient data of database (718).

Up to the end of the dialysis treatment, regulation is performed to optimize driving of the blood pump, dialysate pump, and replacement fluid adjustment for dialysis treatment with the stability level within the acceptable range.

Next, a specific example of the sensor unit connected to the biological information input section 017 (205c in FIG. 2) illustrated in FIG. 1 will be described in detail with reference to FIG. 8.

Reference sign 801 denotes a temperature/humidity detector, and an infrared sensor type is exemplified. The infrared sensor type is configured to detect the temperature of a portion in the vicinity of the drum membrane inside the ear canals a constantly wearable portion. The infrared sensor type includes a non-contact type infrared detection sensor or the like when detecting the body temperature inside the ear canal by with contact type temperature sensor with Peltier element in a state in which it can be mounted portably in the inside the ear canal, arms and the like.

The humidity detector is formed by a chip-like humidity sensor (for example, manufactured by Sensirion AG) or the like. When subjected to dialysis treatment, it may cause itching and dermatitis due to decreased sweating, and decreased number of sweat glands because of drying of the skin. Therefore, measure is taken for the humidity of the skin and inside the ear canal over time and that effect is informed to the patient, or measures against drying is taken during dialysis treatment. In addition, because it is in a dry state, it does not reach a decrease in blood pressure. However, since dehydration may be performed in transient cases, dehydration amount may be regulated according to individual differences of patient.

When a thermography camera is used for patient temperature detection and a near infrared camera is used for humidity detection, a picture of the skin is taken over time by using a photographing camera for shooting videos, still images or burst captures such as CCD camera, C-MOS camera, etc. Then, the image captured at the beginning is compared with the images taken during dialysis treatment at predetermined intervals, and the temperature value and the humidity value are measured from the difference in color and shading.

In order to distinguish moisture such as perspiration on the image, for example, photographing with a near infrared camera described in the literature (Mariko EGAWA, Med Imag Tech Vol. 30, No. 1 January 2012), and photographing method with a camera described in JP 2008-534214 A or the like are preferably used.

Cameras used for humidity detection may record the face, gestures, etc. of the patient during dialysis treatment in the case of a digital camera, a camera attached to smartphone, and the like. The dialysis treatment gives patients a variety of sufferings, depending on the variation of blood pressure etc. Such sufferings can come across nurses or the like when it is a voice such as groaning, In the case of voice such as groaning. However, facial expressions are hard to be known unless seen. Accordingly, signs of lowering blood pressure and the like is made detectable by photographing facial expressions and physical conditions intermittently or continuously with moving images, or still images.

When temperature sensor and humidity sensor are used, data is one-dimension shape and is outputted as continuous numeric data.

When temperature and humidity are measured in a no-contact manner by using a camera, the output is data of two dimensions. However, it is also possible to set the measurement part in advance, detect only the data of that part, and convert it to a temperature value or a humidity value and convert it to data and then convert it to one dimension which is to be output continuously.

Preferably, as regards characteristic portions, for example, in the case of a finger or a hand in advance, being the finger or the hand is recognized by detecting pixels indicating a contour of the finger or the hand. In the case of a thermography camera, a color data portion corresponding to the temperature of the hand of the patient is detected from a screen. By using two types of data, that is, the contour data and the temperature color data, the characteristic portions of the finger or the hand are recognized, and the temperature color data is detected and output as one-dimensional data.

Reference numeral 802 denotes a pulse wave detection unit, which is formed by using a photo-coupling element including a combination of an infrared light emitting element and a light receiving element and outputs a volumetric pulse wave electric signal obtained by a light receiving element as one dimensional data. It is preferable that the pulse wave detection unit 802 is worn on the foot, arm, earlobe, or fingertip, and the waveform of an ejection wave, which is a peripheral vascular resistance related value, and a waveform which related to an amplitude value of a reflected wave but in a detectable state.

In the element for pulse wave detection, a plurality of infrared light emitting elements are collectively used as one light emitting element. Then, the plurality of infrared light receiving elements are used as one light receiving element, for example, in a form of being traversed inside the upper arm. This makes it possible to detect accurate pulse waves near the heart.

For example, by differentiating the volumetric pulse waveform, a characteristic spiked waveform is formed. When this is detected as a pulse by a comparator, an ejection wave time phase is obtained at a trailing edge. Then, a volumetric pulse wave amplitude value corresponding to that time phase is obtained as an ejection wave amplitude value.

When the reflected wave time phase is reversed after the second differentiation, the reflected wave time phase is obtained with the first spinous wave. When this is detected as a pulse by the comparator, the reflected wave time phase can be obtained at the trailing edge. The amplitude of the volumetric pulse waveform corresponding to this reflected wave time phase is a reflected wave amplitude value.

Note that a DC component may be superimposed on that that volumetric pulse wave. In that case, the rise of the pulse that has the ejection wave time phase corresponds to the minimum amplitude value of the volumetric pulse wave. Therefore, if the amplitude of the rise phase of the ejection wave pulse is detected from the volumetric pulse waveform, it is the lowest value of the volumetric pulse wave. Therefore, even if some drift occurs by taking the difference of the minimum amplitude value against the amplitude value of the ejection wave and the reflected wave, the amplitude values of the correct ejection wave and reflected wave are obtained. At the same time, a certain degree of reliable peripheral vascular resistance related value is obtained, and thus the change amount is also provided with certain certainty.

Reference sign 803 denotes a blood flow/blood pressure detecting section, and for example, a sensor module for a laser blood flow meter (manufactured by Pioneer Corporation), one dimension output of a microminiaturized laser blood flow meter or the like described in a NTT Technical Journal 2005. 11 (p. 24 to 27) is used to measure the blood flow on the skin surface. As the measurement data, for example, it is preferable to provide a miniaturized data than that from which the blood flow data as shown in FIG. 6 is obtained.

For blood pressure detection, an established blood pressure measurement device such as a fingertip sphygmomanometer is used, and it is possible to perform stable dialysis for each patient by continuously or intermittently measuring for a long time. The site of blood pressure detection is preferably measured by the upper arm which is not on the shunt-forming side, but in some cases the blood pressure detection may be done at an earlobe, a toe, a fingertip, or the like.

There are generally two types of blood pressure detection, invasive type and non-invasive type, and both of these measurement methods are included. However, an invasive method is suitable as a continuous, stable blood pressure measurement method. Methods such as Manchette pressurization measurement, volume pressure pulse wave method, etc. are preferred because there is risks, such that surgery may be required to implant into the body in order to place the sensor inside or outside the blood vessel.

Note that It is preferable to measure blood pressure in the vicinity of the blood flow and the part where the peripheral vascular resistance value is to be measured. In addition, measuring the blood pressure at the upper arm or the like may also be preferable in some cases in that it corresponds to the peripheral vascular resistance.

Next, referring to FIG. 12, the means for measuring the blood flow data that can be used to estimate the absolute value used in the present invention will be described in detail.

In FIG. 12, reference numeral 1201 denotes a first pressure cuff, which is attached near the wrist of the arm portion 1200 and is used to press the mounting site by the expansion of the air pressure, and it may include a cuff for a blood pressure gauge.

Reference numeral 1201a denotes a first pressure cuff conduit for mechanically connecting a pressure sensor 1215 for wrist cuff and the first pressure cuff 1201.

Reference numeral 1202 denotes a cuff for detecting volume change, which is for measuring a pressure amount when a suck-like member 1202a disposed inside the cover member is pressurized by circumference expansion caused by avascularization block of venous circulation due to expansion of the second pressure cuff 1203. An example of the specific configuration is shown in FIG. 1.

Reference sign 1202b denotes a conduit for suck-like member, which is preferably formed of rubber, plastics, resin or the like and has a hardness to an extent that is not deformed by the air pressure force in terms of improving the accuracy.

Reference sign 1203 denotes a second pressure cuff, which is attached to the upper arm of the arm portion 1200 and presses the mounting site by an expansion of the air pressure and may include a cuff for a blood pressure gauge or the like.

Reference sign 1203a denotes a second cuff conduit for mechanically connecting the second pressure cuff 1203 and a second switching body 1206, and a branch path for mounting the pressure sensor 1214 for the upper arm cuff is provided midway.

The first pressure cuff 1201 and the second pressure cuff 1203 may have the same configuration and may have a configuration of a cuff used for a general-purpose blood pressure gauge.

Reference sign 1204 denotes a first switching body which includes a solenoid valve or the like and performs an opening and closing switching operation by inputting an electric signal. The term "switching" indicates switching of the connection from a connection of tubes between a first air driving member 1205 and a first pressure cuff 1201 to a connection between the first pressure cuff 1201 and a third opening conduit 1204a. The third opening conduit 1204a causes pressurization of the first pressure cuff 1201. Therefore, it is performed by a mechanical connection between the first pressure cuff 1201 and the third opening conduit 1204a by switching of the first switching body 1204.

Reference sign 1205 denotes a first air driving member, and configured to output an air pressure for performing expansion to the extent of blocking avascularization of the venous circulation with respect to the first pressure cuff 1201.

Reference sign 1206 denotes a second switching body, which includes a solenoid valve and the like, and is configured to perform an opening and closing operation by inputting an electric signal. The term "switching" indicates switching a connection between a second air driving member 1207 and the second pressure cuff 1203 to a connection between the second pressure cuff 1203 and a first opening conduit 1206a. This is to release the air pressure of the second pressure cuff 1203 after completion of measurement or abnormal pressurization.

Reference numeral 1207 denotes a second air driving member, which is configured to output an air pressure for expanding to the extent of blocking avascularization of the venous circulation with respect to the second pressure cuff 1203.

Reference sign 1208 denotes a regulating air driving member for supplying a predetermined air to the suck-like member 1202a.

Reference sign 1209 denotes a quantitative air reservoir for storing a predetermined amount of air for supplying a predetermined air to the suck-like member 1202a.

The quantitative air reservoir 1209 is connected with a regulating air driving member 1208 by conduit and stores an amount of air sent by the regulating air driving member 1208 by a predetermined amount.

Reference sign 1209a denotes a quantitative air adjustment pressure sensor, which detects the air pressure in the quantitative air reservoir 1209 and outputs a signal for storing a constant air pressure.

Reference sign 1209b denotes a quantitative air pressure signal transmission path, which transmits an output signal of the quantitative air adjustment pressure sensor 1209a in a wired or wireless manner.

Reference sign 1210 denotes a switching body for regulation which performs opening and closing and switching operations by inputting an electric signal such as a solenoid valve. Reference sign 1210a denotes a second opening conduit for releasing the air pressure in the suck-like member to the atmosphere by the switching operation of the switching body for regulation 1210. This is done, for example, when opening the air pressure in the suck-like member 1202a at timing other than the time of measurement.

Reference sign 1211 denotes a calibration air input/output unit including a combination 1211b of a gasket body and a plunger body, which slides in the interior of a barrel-shaped body 1211a, and a slide driving section for sliding the barrel-shaped body 1211a to the left and right is denoted by 1211c.

Reference sign 1212 denotes a pressure sensor, which has the same configuration as the pressure sensor 111 shown in FIG. 1. Reference sign 1212a denotes a second transmission path, which is a transmission path for transmitting pressure data output from the pressure sensor 1212 by wire or wireless.

Reference sign 1213 denotes a control unit, which has a built-in computer with consecutively memorable memory such as SDD, SD card, USB memory etc., Wi-Fi, wired LAN, etc., and is configured to process and store an air pump driving signal, a solenoid valve opening/closing signal, a pressure data of a pressure sensor 1215 for the wrist cuff.

Reference sign 1213a designates a first transmission body, formed of an electrical lead wire or the like, and is configured to transmit an electric signal for causing the first switching body 1204 to perform switching operation and opening/closing operation.

Reference sign 1213b designates a second transmission body, which is formed of an electrical lead wire or the like, for performing a driving electric output and a control electric signal to the slide driving section 1211c.

Reference sign 1213c denotes a third transmission body, which is formed of an electrical lead wire or the like for performing a driving electric output and a controlling electric output to the regulating air driving member 1208.

Reference sign 1213d denotes a fourth transmission body, which is formed of an electrical lead wire for transmitting an output from a driving opening/closing electricity to the switching body for regulation 1210.

Reference sign 1213e denotes a fifth transmission body, formed of an electrical lead wire or the like for transmitting an electric signal for performing an opening/closing operation and a switching operation of a second switching body 1206.

Reference sign 1213f denotes a sixth transmission body, formed of an electrical lead wire or the like, for transmitting an electric output and a control output for driving the second air driving member 1207.

Reference sign 1213g denotes a seventh transmitter, formed of an electrical lead wire, for transmitting the driving electric output and the control signal of the second air driving member 1205.

Reference sign 1214 denotes a pressure sensor for upper arm cuff, for detecting a pressure amount when pressurizing the upper arm and outputting the detected result to the control unit 1213.

Reference sign 1214a denotes a first transmission path, which forms a transmission path of an electric signal by wired or wireless. For example, when the pressure sensor 1214 for upper arm cuff is provided with wireless output means, constitutes a transmitting body, and the first transmission path 1214a constitutes a transmission body, and in the case of the wired output, it is constituted made of an electrical lead wire.

Reference sign 1215 denotes a pressure sensor for wrist cuff, which detects a pressure amount when pressurizing the upper arm and outputs it to the control unit 1213.

Reference sign 1215a denotes a third transmission path, which forms a transmission path by wire or wireless. For example, the third transmission path 1215a constitutes a wireless transmission body when the pressure sensor 1215 for wrist cuff has wireless output means, and in the case of a wired output, it is made of an electrical lead wire.

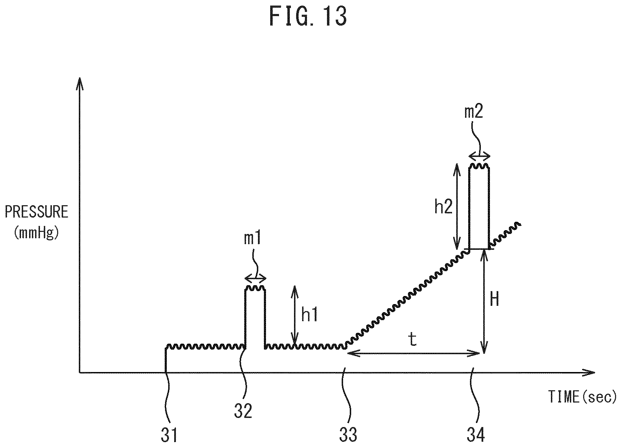

Next, an operation of an example shown in FIG. 12 will be described in detail with reference to FIG. 13. FIG. 13 is a chart illustrating a change in pressure value of the pressure sensor 1212.

The control unit 1213 outputs air to the regulating air driving member 1208 via the third transmission body 1213c to the quantitative air reservoir 1209 until the pressure becomes equal to the predetermined amount (3 mmHg to 10 mmHg).

When the quantitative air reservoir 1209 stores a predetermined air pressure, a quantitative air adjustment pressure sensor 1209a is provided to measure and monitor the pressure value.

Alternatively, means for driving the regulating air driving member 1208 based on the time until the regulating air driving member 1208 obtained from the supply rate value of the air amount supplied from the regulating air driving member 1208 and the volume value of the quantitative air reservoir 1209 reaches a predetermined pressure is also applicable.

The quantitative air adjustment pressure sensor 1209a outputs a signal capable of monitoring the air pressure of the quantitative air reservoir 1209. When the control unit 1213 reaches a predetermined pressure from this pressure sensor output signal value, the regulating air driving member 1208 Is stopped.

Since the air pressure in the quantitative air reservoir 1209 decreases over time, it is sometimes necessary to drive the intermittent regulating air driving member 1208 in order to maintain a constant amount of air pressure at all times.

With the amount of air in the quantitative air reservoir 1209 being a predetermined amount, the control unit 1213 outputs a signal that opens the switching body for regulation 1210 via the fourth transmission body 1213d. When switching body for regulation 1210 mechanically connects conduit for suck-like member 1202b and quantitative air reservoir 1209, quantitative air is supplied to suck-like member 1202a via conduit for suck-like member 1202b. When the suck-like member 1202a expands and makes contact with the skin, the output voltage of the pressure sensor 1212 in FIG. 13 becomes a state of 31, and the pressure pulse wave superimposes as the pressure rises.

At the timing 32 in FIG. 13, the control unit 1213 drives the calibration air input/output unit 1211 and outputs a DC voltage to supply quantitative air (0.5 mL to 1.5 mL). After the predetermined time (m1) (0.5 sec to 2 sec), the control unit 1213 operates to recover the air supplied to the slide driving section 1211c via the second transmission body 1213b.

Next, the control unit 1213 transmits a signal for starting driving to the first air driving member 1205 via the seventh transmitting body 1213g.

Further, the control unit 1213 outputs a signal for mechanically connecting the first pressure cuff conduit 1201a and the first air driving member 1205 to the first switching body 1204 via the first transmission body 1213a.

The first air driving member 1205 drives to output the air pressure, and outputs the air pressure of a predetermined amount from the first air driving member 1205 to the first pressure cuff 1201.

Next, the control unit 1213 transmits a signal for starting driving to the second air driving member 1207 via the sixth transmitting body 1213f.

Further, the control unit 1213 outputs a signal for mechanically connecting the second cuff conduit 1203a and the second air driving member 1207 to the second switching body 1206 via a fifth transmission body 1213e.

The second air driving member 1207 drives to output the air pressure and outputs the air pressure of a predetermined amount from the second air driving member 1207 to the second pressure cuff 1203.

Pressure from the second pressure cuff 1203 caused the venous circulation to become an avascularization block-like condition, so that an outward pressure is applied to the suck-like member 1202a in the measuring part by the circumference expansion of the arm 1200. Therefore, a pressure value increases as indicated by 33 to 34 in FIG. 13.

At timing 35 of FIG. 13, the control unit 1213 drives the calibration air input/output unit 1211 again by outputting the starting voltage V (V) to the slide driving section 307 via the second transmission body 1213b, and supplies quantitative air to the suck-like member 1202a.

The control unit 1213 stops an electric output to the slide driving section 307 with respect to the calibration air input/output unit 1211 after the treatment time m2 (0.5 sec to 2 sec), and recovers the supplied air, so that the pressure falls as indicated at 36 in FIG. 13.

After the above operation is completed, the control unit 1213 outputs a signal to switch so as to release the connection direction of the second switching body 1206 and the first switching body 1204 to lower cuff pressures of the first pressure cuff 1201 and the second pressure cuff 1203.

The pressure sensor 1212 transmits pressure data to the control unit 1213 via the second transmission path 1212a and the control unit 1213 calculates and detects parameters for calculating the blood flow velocity from change over time of this pressure data.

The control unit 1213 performs an operation of substituting the parameters shown in FIG. 13 obtained by the above operation into the following Equation (4). Here, the control unit 1213 stores the result in the storage device, outputs numeric data. Based on the following Equations (4), (5) and (6), a blood flow amount (Q60) per minute per unit volume (100 ml) which allows estimation of an absolute value is obtained and the blood flow information of dialysis patients can be obtained through the intermittent measurement.

[Equation 4]

$$\Delta V = h0 / h \times H \qquad \cdots (4)$$

[Equation 5]

$$Q60\ (V0\ \text{WITHOUT CORRECTION}) = (h0/h \times H/t) \times 60\ \text{mL/min(UNIT)} \qquad \cdots (5)$$

[Equation 6]

$$Q60\ (V0\ \text{WITH CORRECTION}) = Q60 \times (100/\ V0)\ \text{mL/min/100mL(UNIT)} \qquad \cdots (6)$$

In the above equation, the units of ΔV and h0 are mL respectively, ΔV is the volume change amount h in the suck-like member according to the circumference expansion of the measuring part, the unit of H is mmHg or the voltage mV, h is the midpoint air h = (h1 + h2)/2 of each pressure value when supplying and recovering calibration air, H/t is the rising speed in the vicinity of the midpoint, V0 is a value obtained by measuring the volume of the measuring part (measuring the circumference C of the measuring part in advance, and using V0 = (the width of the cuff for detecting the volume change) × C2/4π), h0 is the volume of the calibration air, and H is the change amount of cuff internal pressure due to circumference expansion.

Referring again to FIG. 8, reference sign 804 denotes an image characteristic portion detection unit which includes a combination of a WEB camera, a smartphone camera capable of shooting moving images and still images, and a combination of a computer, a CPLD, and an FPGA and the like activating image processing software.

The image characteristic portion detection unit 804 recognizes a predetermined part of the obtained image, for example, in the case of a face, recognizes a region of tail regions of the eyes and part of the nose hole by detecting the image outline, sets a portion at a predetermined distance as the portion to be detected, and extracts the color data. This color data is further decomposed into primary colors of RGB or CMYK, and the density value of each primary color is output as one dimensional data.

Reference sign 812 denotes a temperature/humidity value change amount detection unit, which takes a difference between the data outputted from the temperature/humidity detector 801 before predetermined time (1 msec or more) and the subsequent data, and outputs the difference data.

Reference numeral 813 denotes a pulse wave value change amount detecting means, which calculates a difference between the pulse wave data outputted from the pulse wave detection unit 802 at a predetermined time (1 msec or more) before and after the subsequent data, and outputs the difference data.

Reference sign 814 denotes a blood flow/blood pressure value change amount detecting section, and form a difference data as described above between data outputted from the blood flow/blood pressure detecting section 803 before and after predetermined time (for example, 1 msec or more).

Reference sign 815 denotes an image characteristic portion change amount detecting section, and form a difference data as described above between data outputted from the image characteristic portion detection unit 804 before and after predetermined time (for example, 1 msec or more).

Reference sign 805 designates a mixing means, which is composed of a multiplexer and the like, and is means for time-dividing each signal into one or more signal strings. It is unnecessary to output each signal, for example in the case of modulating and outputting in different frequency bands, instead of mixing them one by one.

Reference sign 806 is a modulating mean. An FM, an AM, a pulse code modulation (PCM), a pulse amplitude modulation (PAM), a pulse frequency modulation (PFM), a pulse width modulation (PWM), a pulse frequency deviation modulation (FSK), the modulating means 806 modulate with each signal and in some cases is combined into one signal and sent to transmission means 807 for each patient.

Reference sign 807 denotes a transmission means, which transmits and outputs the modulation signal outward wirelessly or by wire. The term "outside" means from the patient or the bed side of patient, for example to the center that manages the patient's bed. The configuration up to transmission means 807 is located around patient and bed as a patient terminal.

Reference sign 808 denotes a reception means which is disposed at the center, receives a signal wirelessly or wired from the transmission means 807, converts it into an electric signal, and outputs it to the demodulating means 809.

Reference sign 809 denotes a demodulating means demodulates the modulated temperature modulation signal, humidity modulation signal, and state value modulation signal, respectively, and outputs the demodulated signals to the processing means 816, respectively.

In the case of bidirectional communication, the signal to be sent may be, for example, a signal for remote control. For example, a case where only one of the detection units is used to stop the others, and a case where a signal for turning on and off the operation is transmitted to a terminal on the ear side are exemplified.

Reference sign 810 denotes a separating means, which is configured to separate a detection signal grouped in one signal. When the mixing means 805 is unnecessary, this is not necessary.

Reference numeral 811 denotes a transmission medium, which includes a wireless transmission and a cable transmission such as a radio wave and a cable, but here, it indicates a wireless medium.

Reference numeral 816 denotes a processing means configured to add numeric data which can be processed by the individual difference information management processing means 218 illustrated in FIG. 2, identification data which can be identified for each detecting unit, and detection time data, and convert it into a digital signal format.

Reference sign 817 denotes output means, in which an output end 800 is connected to the biological information input section shown in FIG. 2, and configured to adjust data to be recognizable as data by individual difference information management processing means 218, and output the same.

The processing means 816 and the output means 817 may be formed as a part of the biological information input section and the individual difference information management processing means 218 illustrated in FIG. 2.

Note that that output means 817 may be connected to the biological information input section 205C in a wireless specification and may include a transmitting antenna, a light emitting diode, or the like. All of these are executed by program processing by computer, and may include an infrared transmission module, a Bluetooth module, a Wi-Fi module, or the like.

Next, an operation of the example illustrated in FIG. 8 will be described.

The temperature/humidity detector 801, the pulse wave detection unit 802, the blood flow/blood pressure detecting section 803, the image characteristic portion detection unit 804 are configured in such a manner that when detecting data with sensor, a dialysis patient wears and operates an ear sensor used in the ear canal or a bracelet and a ring type sensor unit to be used by being inserted and attached to the arm or the finger during dialysis treatment and other life time.

In the case of using the cameras, a plurality of cameras are set to allow the patient to be measured in a position other than the spine position, and if possible, are set to be capable of recognizing and tracking measuring parts.

The temperature detector of the temperature/humidity detector 801 converts the infrared signal into an electric signal from, for example, an infrared sensor.

In the case of a humidity sensor for measuring the humidity of the skin, the humidity detector converts the humidity of the skin into electric signal in contact with the skin or in a non-contact state.

The pulse wave detection unit 802 detects an ejection wave amplitude value, a reflected wave amplitude value, and the like from a pulse wave sensor pulse wave signal worn on the toe, the knee, the fingertip, the earlobe or the like, obtains the ratio or difference, and, in some cases, outputs to the pulse wave value change amount detecting section 813 as a peripheral vascular resistance related value together with the heart rate value.

The blood pressure detecting section 803 measures the blood flow data and the blood pressure data using the blood flow meter and blood pressure gauge at the part near the site where the fingertip, upper arm, pulse wave detection sensor is attached, and outputs the measured value to the blood flow/blood pressure value change amount detecting section 814.

The image characteristic portion detection unit 804 detects the color, the expression, and the movement of the patient's face or body, or outputs one-dimensional, or two-dimensional portion data changed in color, expression, and movement by a predetermined value or more to the image characteristic portion change amount detecting section 815.

Each of the temperature/humidity change amount detection unit 812, the pulse wave change amount detecting section 813, the blood flow/blood pressure change amount detecting section 814, and the image characteristic portion change amount detecting section 815 takes a difference from values before and after a predetermined time of input data and outputs the value to the mixing means 805.

In the case of comparison with two dimensional data, the image characteristic portion change amount detecting section 815 may output a change in color of an entire image of the face of the patient as a two-dimensional image, and may output a change data of a portion which has changed into a color other than the predetermined color to be changed depending on the position of irradiation.

The predetermined time interval may be the same time interval, but it may be different time intervals, and at least a change in the patient's during dialysis condition and time intervals to which the change amounts output from the temperature/humidity change amount detection unit 812, the pulse wave change amount detecting section 813, the blood flow/blood pressure change amount detecting section 814, and the image characteristic portion change amount detecting section 815 may be taken separately.

The mixing means 805 time-divides, phase-divides, and the like into one signal, and outputs it to the modulating means 806. The modulating means 806 modulates the mixed signal by a method such as AM, FM, PCM, PM etc., and outputs it to the transmission means 807. The transmission means 807 wirelessly outputs the modulated signals.

The signal transmitted from the transmission means 807 is received by the reception means 808 at the center, where the signal is subjected to amplification and filter processing, demodulated by the demodulating means 809, and the original signal is taken out.

The separating means 810 is means for separating signals from sensors mixed in the mixing means 805 and converted into one signal. The signal indicating the separated change amounts are output the processing means 816. The processing means 816 combines the time when the change has occurred, the change amount (one-dimensional or two-dimensional difference data), and the detection target as one block of data and output the same to the output means 817.

The processing means 816 temporarily records these biological information signal data in the digital memory and forms a state in which it can be outputted to the biological information input section 215C illustrated in FIG. 2. If individual difference information management processing means 205 requests data, or while requesting, the processing means 816 provides data.

An individual difference information management processing means 205 discriminates whether the inputted biological information belonging to dialysis treatment or living activity by time information attached to biological information data and the like, monitors the body temperature, the blood pressure, the skin humidity condition and its change amount on real time basis, and records and registers the data in an input/output storage means 209 as patient data.

Note that when the individual difference information managing means 02 illustrated in FIG. 1 detects that data the change amount data outputted from the temperature/humidity change amount detection unit 812, the pulse wave change amount detecting section 813, the blood flow/blood pressure change amount detecting section 814, the image characteristic portion change amount detecting section 815 exceed or falls below a predetermined value occurs during dialysis treatment, even if the blood pressure value has not changed, there is possibility that the blood pressure will change, and thus a signal for performing optimization output 02a such as administration of replacement fluid, or adjustment for decreasing the driving amount of the blood pump, and the like are output.

The biological information input section illustrated in FIG. 8 is configured to directly modulate the change amount data from signals obtained from various sensors and transmit to the data processing terminal. Wireless transmission may be transmitted over wireless LAN using Bluetooth (registered trademark), Wi-Fi, direct Wi-Fi and the like. In addition, it is also possible to make a connection in a wired format instead of a wireless format.

Also, for face color, expression, body movement, temperature measurement, and humidity measurement, a camera capable of creating digital moving images such as WEB camera is used. However, at that time, the patient may change the direction such as turning over.

In such a case, by preparing a plurality of WEB cameras to be tracked with the face or the like in advance as a tracking target and arranging it on the bed side in a plurality of orientations, a state which can be tracked with any one camera except when the face is hidden or the like may be created.

Also, if the face, hands and feet are hidden and cannot be measured, alert may be issued to the patient to place them in a situation which can be taken by the camera.

The biological information input section collects data by contacting the patient. The biological information input section may be configured to be an ear sensor type in which all the sensors are accommodated in a single carrier as a portable carrier having an earplug type ear sensor form, a bracelet form, ring form and the like, or may be configured separately in a handheld carrier such as a bracelet, a ring, a smartphone or the like.

Next, another example illustrated in FIG. 10 will be described.

Reference sign 1001 denotes an individual difference information management unit, which includes a computer system such as a CPU, a system memory, a hard disk, etc., a hardware operation circuit such as Field Programmable Gate Array (FPGA), and Complex Programmable Logic Device (CPLD), etc., in which an optimized dialysis application and associated software are stored. It is configured to be executed when necessary.

The individual difference information management unit 1001 further is connected to network NET such as internet, intranet, etc., and makes it possible to obtain dialysis related information such as patient's dialysis treatment data.

Reference sign 1002 denotes a drive control unit, which receives a drive command from individual difference information managing means and outputs drive commands to the driving units of the blood circuit and the dialysate circuit in accordance with the command.

The drive control unit 1002 and the blood driving pump 1012 are electrically connected by a wireless or wired connecting body 1002c and the drive control unit 1002 and the dialysate driving section 1013 are electrically connected by a wireless or wired connecting body 1002b, and the drive control unit 1002 and the replacement fluid supply section 1014 are electrically connected by a wireless or wired connecting body 1002a.

Reference sign 1003 denotes a display unit, which includes a monitor display, a mouse for computer, a keyboard, an I/O unit such as a microphone, etc., and a clinical technicians, a doctor, and a nurse are allows to perform input operations such as input of individual difference information. In addition, it is means for communicating to doctors or the like by alert sound, indication or the like when emergency measures are necessary due to the drop in blood pressure etc.

Reference sign 1004 denotes an information processing unit for dialyzer, which is a unit for forming dialysis machine information based on information from the dialyzer information input unit 1008 and outputting dialysis machine information to individual difference information management unit 1001. The information processing unit for dialyzer 1004 may constitute a signal processor in combination with the dialyzer information input unit 1008.

Reference sign 1005 denotes a pulse wave information processing unit, which is a signal processing unit for forming a blood pressure related signal from pulse wave information and includes a filter, an amplifier, a differentiating circuit, a secondary differentiating circuit, and a blood pressure related signal forming circuit, or software.

A blood pressure related signal forming circuit has a circuit for obtaining an ejection wave time phase and a reflected wave time phase from a pulse wave signal, for example, and obtaining an amplitude value in that phase analogue or digital.

As body portion for detecting pulse waves, the earlobe, the fingertip, the wrist and other parts of the fingertip of the foot are appropriate to increase the degree of freedom of the patient dialysis treatment.

Reference sign 1006 denotes an image processing unit for obtaining preset information, for example, a computer configuration for obtaining facial color data, facial expression data, facial blood flow data, temperature data, and humidity related data from the obtained image, and a hardware configuration such as the FPGA.

Reference sign 1007 denotes a blood flow information processing section, which comprises a hardware processing circuit for extracting blood pressure related data from an input signal from the blood flow input unit 1015, or a processing circuit activated by software, and the like.

Reference sign 1008 denotes a dialyzer information input unit, which includes a blood flow measuring unit. The dialyzer information input unit 1008 has a combination of a blood flow information detection unit with a laser including, for example, a light collecting unit, a light receiving unit, an optical multiplexer, an FFT and the like.

Reference numeral 1009 denotes a photographing unit, which includes one or more cameras. In FIG. 10, three cameras are provided, and there are a case where the number of cameras according to the purpose of photographing and a case where one camera is used.

Reference sign 1009a denotes a patient photographing camera, which may be a CCD camera a CMOS camera, etc., for taking still images or moving images, and includes so-called a WEB camera, a smartphone, a camera attached to a mobile phone.

The patient photographing camera 1009a detects pain and suffering for example, by measuring facial color and/or facial expressions, and measures the physiological condition of the patient.

Reference sign 1009b denotes a skin humidity photographing camera, which includes a near infrared camera equipped with a filter for passing around 1.45 $\mu$m, for example, mainly for measuring the humidity of the skin such as the face, arms, legs and the like, and constitutes a photographing unit like the photography camera for the face.

Reference sign 1009c denotes a thermographical photographing unit, which includes an infrared camera module, and is a camera unit for inputting photographing while discriminating the skin temperature etc. of a patient by color combination.

Reference signs 1000a, 1000b, 1000c, and 1000d denote sensor units, which have the same configuration and include an irradiating section for irradiating laser light and a light receiving part for receiving reflected light reflected from the living tissue, and four sensor units are fixedly connected to the side face of dialysis machine (dialyzer) 1011 at a predetermined distance.

In the sensor unit, for example, the optical fiber end faces of the laser light output part and the light receiving part respectively in contact and connected fixedly with the side face of the dialyzer 1011.

The sensor units are connected to the dialyzer information input unit 1008 via light guides 1008a to 1008d formed of optical fibers and the like.

Reference sign 1011 denotes a dialysis machine (dialyzer), in which several thousands to several hundreds of thousands of porous hollow fiber bundles are arranged. Blood passes through each interior of the hollow fiber. Bodily fluid, waste matter, etc. are taken out to the outside from porous portion on the porous side.

Outside of the hollow fiber, blood dialysate flows in the opposite direction from the direction of flow of blood and, and the bodily fluid, waste matter of interior of the hollow fiber are transported and dialysate is supplied into the interior of the hollow fiber by back filtration.

Reference numeral 1011a denotes a blood input unit, and reference sign 1011b denotes a blood output unit, which are connected respectively to the interior of the hollow fiber.

Reference sign 1012 designates a blood driving pump for generating a flow in the blood taken out of the living body via the shunt part S, and usually produces a flow of 200 ml/sec. However, the rotation speed is changed manually or automatically by an external electric signal or an input by a switch or the like, thereby making the flow of blood regulatable.

Reference sign 1013 denotes a dialysate driving section, which is equipped with an electric pump, a dehydration regulating part, a waste reservoir, etc., and performs removal and dehydration of waste matter while regulating the flow of dialysate. However, in this example, the pump function will be mainly described.

Reference sign 1014 denotes a replacement fluid supply section, which includes a replacement fluid reservoir and is a portion for supplying missing bodily fluid and supplying essential ingredients in the body, and may be provided with a configuration for automatically supplying the regulated amount as needed in order to lower the blood pressure and stabilize the blood pressure.

Reference numeral 1015 designates a blood flow input unit, which is a band for fixing the sensor on the wrist to measure the blood flow at the wrist with the laser blood flow unit. Note that when attaching to the fingertip or the earlobe, it may be a cap or a clamping member instead of the band.

Reference sign 1016 denotes a pulse wave sensor of a type to be attached to the fingertip of the foot or an infrared reflection type sensor to be attached to the earlobe, and is electrically connected to a pulse wave information processing unit 1005 with a wireless or wired connecting body 1016a.

Reference sign 1017 denotes a conduit for a dialysate circuit, such as a catheter tube for circulating dialysate via a dialysis machine 1011 and the like. Reference sign 1017a denotes a dialysate outlet port for extracting the dialysate flowing downward from the dialysis machine 1011 to the outside.

Reference sign 1017b denotes a dialysate input unit for supplying dialysate supplied through the dialysate circuit conduit 1017 into the dialysis machine 1011.

Reference signs 1018a to 1018d denote conduits for blood circuit, which include, for example, catheter tubes for connecting the shunt part S and the dialysis machine 1011.

Next, an operation of the example illustrated in FIG. 10 will be described.

A blood circuit BC is formed by a blood driving pump 1012, a conduit for blood circuit 1018c, a dialysis machine 1011, a conduit for blood circuit 1018d, and a replacement fluid supply section 1014 by forming a shunt part S on upper arm A of a patient and connecting conduits for blood circuit 1018a and 1018b.

The face of patient F and other skin are photographed in advance by the photographing unit 1009, the body temperature distribution, the state of the facial color, and the humidity distribution are photographed and the images are output to the image processing unit 1006 via the connecting body 1009d.

After setting the state of facial color, temperature condition, and humidity condition have been set as the initial state from the image data of life time, the data is transmitted to the individual difference information management unit 1001, and the individual difference information management unit 1001 stores it as patient data.

It is preferable to record the initial individual difference information of the patient before starting the dialysis treatment from waking up during start of dialysis treatment, from waking time to bedtime and while sleeping to enable various comparisons with changes during dialysis treatment.

A pulse wave sensor 1016 is attached to the earlobe by clamping, but it may be unnecessary if it is possible to detect a volumetric pulse wave which enables measurement of the reflected wave by using the blood flow input unit 1015.

The blood flow input unit 1015 is attached on the wrist or the blood flow sensor band 1015a for the fingertip is attached.

The blood flow sensor in the blood flow sensor band is a sensor for laser blood flow meter sensor and includes a pair of optical fiber ends constituting a laser output part and a light receiving part or a combination of a laser light output semiconductor chip and a light receiving semiconductor chip. In the former case, the transfer section 1015b is composed of a light guide such as optical fiber, and in the latter case, the transfer section 1015b is composed of an electrical lead wire.

The light receiving signal or electric signal obtained by the blood flow input unit 1015 (1015a) is transmitted to the blood flow information processing section 1007 via a transfer section 1015b including a light guide or an electrical lead wire.

In the blood flow information processing section 1007, when the transfer section 1015b is a light guide, the light receiving signal is converted into an electric signal via the photoelectric conversion means, and when the transfer section 1015b is the electrical lead wire, it is directly input to the filter and the amplification circuit, and then the blood flow amount is calculated.

Similarly to the pulse wave signal, when the reflected light is detected from the light receiving signal, the ejection wave high value P1 and the reflected wave high value P2 are detected and the ratio P2/P1 is obtained. The blood flow amount and P2/P1 is input to individual difference information managing means 1001 and stored temporarily or continuously in the memory as blood pressure related data.

The blood pressure related data is always measured and transmitted to the individual difference information management unit 1001 during dialysis treatment. The image processing unit 1006 further transmits the patient state signal such as the temperature image signal, the humidity image signal, the facial color and the like to the individual difference information management unit 1001.

While photographing the face of patient F with patient photographing camera 1009a, change in face color is detected.

As a method of changing the facial color, for example, a method described in the document "Extraction and Application of Physiological Information from Face Color Change, the 298th meeting of the Society of Instrument and Control Engineers, Tohoku Chapter (2015.11.18), Document Number 298-4" after measurement of the facial color using the described method, the change is recorded as data.

For example, the term "change" is understood as change in color (primary color) between frames at a predetermined time intervals at the time of photographing. When the difference becomes a predetermined value or larger, the fact that a change in facial color occurs to the individual difference information management unit 1001.

Whether this change is a common change as the patient's individual difference, and whether a common change is warning sign to interrupt dialysis treatment is collated against the patient's record.

If there is no change similar to patient data, for example, the data of the external dialysis treatment agency is searched via network NET and if there is no similar change, it is assumed that there is a possibility of distress, is displayed on the display unit 1003.

In addition to facial color, for example, changes in facial expressions due to yawning, nausea, vomiting, headache, palpitations, cold sweats, and tinnitus occurring at the time of decrease in blood pressure are detected by comparison with data previously recorded for each individual difference.

The change of patient does not necessarily include the face but also hands and feet such as the number of turns, state of the body, and the like.

Further, if necessary, the drive control unit 1002 outputs a regulates signal for driving the blood driving pump 1012, a dialysate driving section 1013, and a replacement fluid supply section 1014.

The skin humidity photographing camera 1009b for the skin moisture and the thermographical photographing camera 1009c preferably detect humidity and temperature at the same time and used as data for judging whether it is a sign of decrease in blood pressure from the relationship with the facial color.

In addition, recognition of facial expressions is captured by camera and detected, facial expression recognition using Intel Perceptual Computing (PerC) SDK and Creative Intaractive Gesture Camera of made by Intel Corporation, and other Microsoft company's kinect for Windows etc. detects facial expressions, and it is available as an element of the means for obtaining decrease in blood pressure or prediction data before decline.

Measurement of the skin humidity by the skin humidity photographing camera 1009b is described, for example, in the literature "Mutsuko Nakamura et al., Moisture retention effect of cosmetics as seen in near infrared spectroscopic images, Optics, Vol. 39 No. 11 (2010), 529-533" irradiates the face with near infrared light and measures the humidity of the face.

The photographing unit 1009 is provided with a lighting unit, and according to a chronological order, a method of taking an image while switching the lighting unit is exemplified. In one camera, a configuration in which a plurality of light sources of near infrared light, a white light source and the like are used by switching.

In photographing a patient with these cameras, since the face direction of the patient may change due to turning or the like during dialysis, a configuration in which a plurality of camera units are prepared and placed in the direction the face faces, the computer is made to recognize the face outline and the like, and a photographing unit 1009 having a robot arm attached to a distal end is moved to truck it is also applicable.

Since the blood flow amount decreases when the temperature drops due to poor circulation or the like, the thermographical photographing camera 1009c may constantly photograph the peripheral part in which the blood flow is measured and detect the change in temperature.

The four blood flow sensors 100a to 1000d arranged on the side surface of the dialysis machine 1011 respectively measure the blood flow in the hollow fiber in the dialysis machine 1011 and supply it to the dialyzer information input unit 1008 via a light guide.

The dialyzer information input unit 1008 outputs, from these blood flow values, values related to the dehydration amount such as filtration flow rate to the information processing unit for dialyzer 1004.

Based on these values, the information processing unit for dialyzer 1004 obtains the minimum filtration flow rate value and the like by using the chart shown in FIG. 9.

The flow rate value is directly related to the dehydration amount, although it changes in the direction of gradually decreasing the filtration flow rate value. Also, the blood flow amount obtained from the blood flow input unit 1015 (1015a) indicates the blood flow after plasma refilling.

Therefore, when individual difference information management unit 1001 monitors the difference between them, and when the difference is maintained for a predetermined time at a predetermined value or higher, the individual difference information management unit 1001 determines that it indicates the possibility of the decrease in blood pressure, and displays an alarm to notify the same to on the display unit 1003. Alternatively, an operation to issue an instruction to increase the amount of replacement fluid in the replacement fluid supply section 1014 to the drive control unit 1002.

An abnormal increase in concentration is detected from peripheral blood flow amount and amount of change

**EP 3 511 033 A1**

over time of filtration amount by dehydration, the possibility of blood pressure variation is predictively detected.

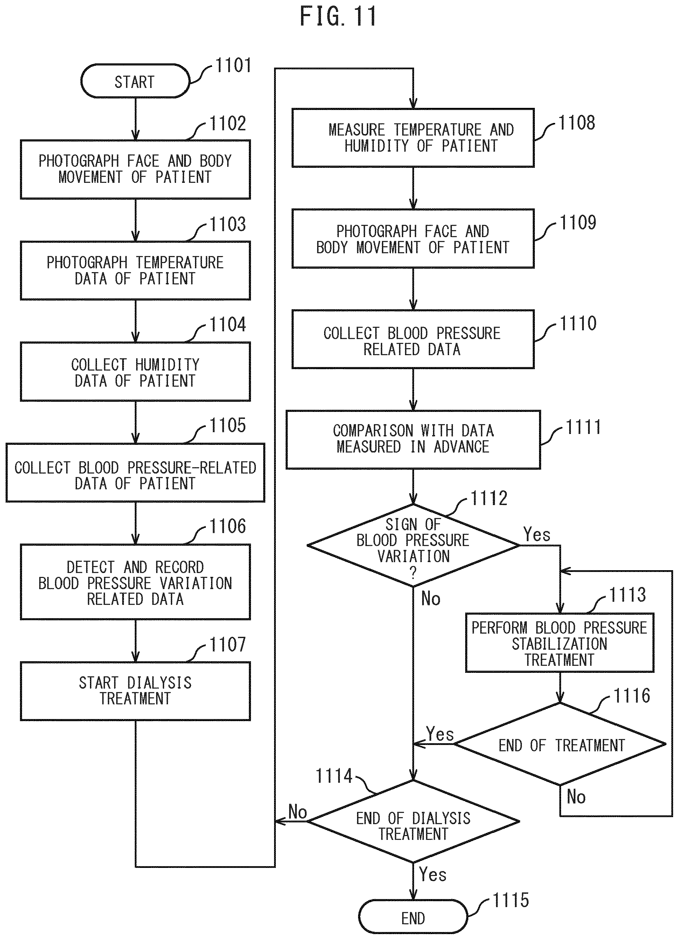

Note that Figure 10 illustrates the entire unit for obtaining individual difference information. However, as an example, not all of these are required, but it may be formed only of a unit that can detect at least individual difference information of patients. The example is also illustrated in FIG. 11 and will be described.

The example illustrated in FIG. 11 has a configuration that does not require the sensor units 1000a, 1000b, 1000c and 1000d, the dialyzer information input unit 1008, and the information processing unit for dialyzer 1004.

In FIG. 11, reference sign 1101 denotes a step of starting the first data collection. The first data collection is exemplified by the state before the first dialysis treatment or the state other than the dialysis treatment day.

In Step 1102, for example, photographing of patient's normal face and body movement with a patient photographing camera 1009a. The patient photographing camera 1009a may be separated from the photographing unit 1009 and used alone.

From hidden images, habits, motions and facial expressions that characterize patients are extracted. It is preferable that a part corresponding to a part to be photographed during dialysis treatment is photographed, such as the face, the hand, the feet, the whole body or the like. Particularly, expression of the face as a habit, a specific color and the like are exemplified.

Also, examples of the storage place to store images of the change in face such as yawning or the change in body movement which may occur at low blood pressure, which are photographed in advance as digital still images or digital movies, include mass storage media such hard disk, of computer of individual difference information management unit 1001, SSD, USB memory and the like.

Step 1103 is a step of photographing and measuring the normal temperature data of the patient. The usual temperature data of a patient is a body temperature, which indicates not only the body temperature data continuously measured by a thermometer, but also temperatures of the patient's face, arms, hands and legs measured by a thermographical photographing unit illustrated at 1009c in FIG. 10 and stored as digital data as described above.

This is effective as individual difference data of patients who originally have relatively low limb temperature such as cold constitution.

That is, in this example, it is expected to be able to detect signs of lowering blood pressure, in particular rapid signs of decline. Since blood pressure is indicated by the product of blood flow and peripheral vascular resistance, blood pressure decreases due to a decrease in blood flow, but according to the homeostasis-like intracorporeal manipulation of a person, there are cases where the blood flow narrowing operation, that is, the peripheral vascular resistance is increased in order to keep the blood pressure constant. At that time, since the blood flow of the hands and feet decreases, the temperature may be lowered, but depending on the patient, the temperature of the limbs may be low in the first place. Therefore, the temperature of the peripheral part such as hands and feet is measured in advance, so that it is possible to grasp the decrease in blood pressure according to the individual difference of patients from the change amount of body temperature.

Reference sign 1104 denotes a step of collecting patient's usual humidity data. Humidity data may be measured with the humidity sensor in contact with the skin, but it may be measured in advance by using the skin humidity photographing camera 1009b as described above. The individual difference data of sweating or the state of dry skin may be measured and, alternatively, the face may be photographed with a normal camera and the reference state of humidity may be measured from the color of the face.

Step 1105 is a step of collecting patient's normal blood pressure related data and storing the same on a digital recording medium. The blood pressure related data indicates a combination of one or more of blood pressure value, peripheral vascular resistance value, blood flow value, and the like, but it is preferable to provide a blood pressure measurement device including at least a blood pressure sensor. Also, the value of blood pressure related data is recorded together with the change amount.

For example, the change amount indicates the change amount of the blood pressure value, the peripheral vascular resistance value, and the blood flow value in the predetermined time.

Among them, the peripheral vascular resistance value can obtain a parametric value from the volumetric pulse wave. By using this value, the value corresponding to the resistance value, and the change amount can be obtained on a real time basis.

That is, from the volumetric pulse wave, the ratio of the peak value of the ejection wave and the peak value of the reflected wave, that is, the value of AIX is obtained, and by obtaining this change amount over time, change in peripheral vascular resistance is obtained.

Although volumetric pulse wave may be detected from earlobe, and fingertip, it is preferable to detect volumetric pulse wave from the fingertip of the foot because the patient during dialysis treatment is equal to sleeping state.

A step 1106 is a step of detecting and recording blood pressure variation related data, for example, to have a facial expression from a patient such as a yawning appearance, a facial expression at a time of difficulty, etc. appearing when the blood pressure is lowered, It is a step to record.

Note that it is necessary to record expressions corresponding to representations of common faces such as emotions and sorrows and painful expressions close thereto as data in such a manner that computer performs arithmetic processing and detects its expression (for example, in the case of Intel Perceptual Computing (PerC) SDK and Creative Intaractive Gesture Camera of Intel Corporation made), it may be unnecessary, and on the other hand, there are also good case using this automatic detection in combination.

Step 1107 is a dialysis treatment start step, in which measurement of the volumetric pulse wave and measurement of the change amount of the peripheral vascular resistance value, intermittent and continuous measurement of the blood pressure at the fingertip, wrist, upper arm are performed with the camera unit 1009, and measurement of the change amount and the like thereof is started.

Step 1108 is a step of measuring the temperature and humidity of the patient, and photographing a patient with the skin humidity photographing camera 1009b and the thermographical photographing unit 1009c of the photographing unit 1009 shown in FIG. 10.

Regarding the photographing screen, since the patient may be lying sideways for example, photographing from multiple directions is preferable. However, photographing following the orientation of the face and the like may be performed by a reflecting mirror, for example.

Step 1109 is a step of photographing a face and a body movement. In a patient photographing camera 1009a of a photographing unit 1009, the face color, the expression, the body movement such as turning of the dialysis patient is measured, and extracted as an image or by extracting the characteristic point by the image processing unit 1006, and are stored in the digital storage device.

Step 1111 is a step of comparing normal data with data obtained on a real time basis at the time of dialysis therapy. Comparison with the predetermined normal data is performed on the temperature, the humidity, the facial expression, The state of the body movement in the individual difference information processing section 1001, respectively. In the next Step 1112, if it is similar or match the facial color, the body movement, the expression of the face at low blood pressure stored in advance, and if there is an expression different from the patient image data measured in a normal state (YES), the procedure goes to Step 1113.

Step 1113 is a step which carries out the blood pressure stabilization treatment, in which decrease of driving amount of blood pump, decrease of drive amount of dialysate driving pump, supply of replacement fluid, and the like are performed to compensate for decrease of blood flow due to dehydration.

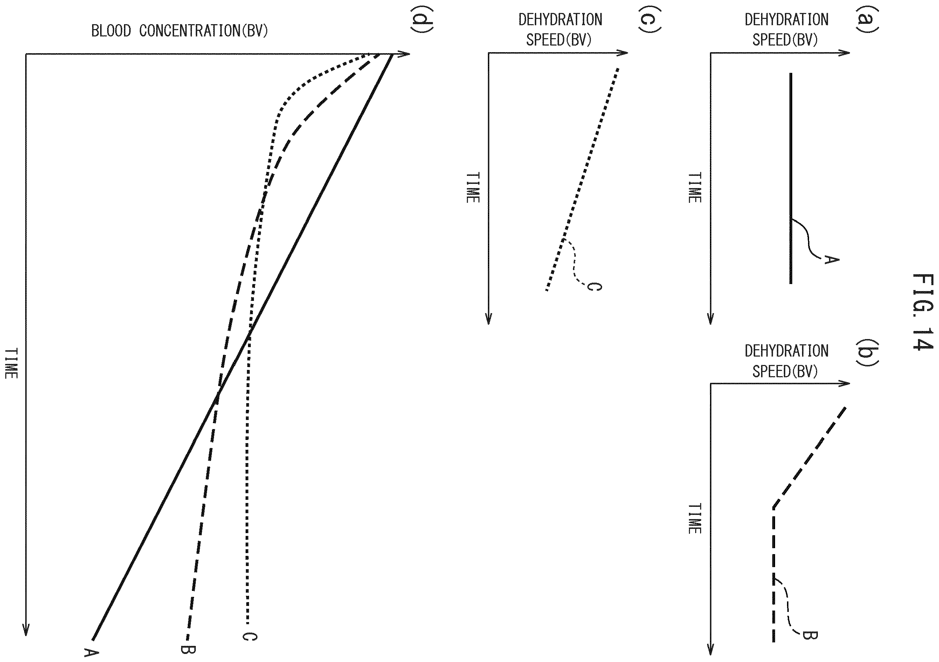

An example of the relationship between dehydration speed and blood concentration (BV) will now be described with reference to FIGS. 14(a) to 14(d).

FIG. 14(d) is a graph plotting an example of the relationship between dehydration speed and blood concentration (BV), and the straight line A in the graph indicates a relationship of a blood concentration value in a case where the dehydration speed shown in FIG. 14(a) is set to be constant (which is normally performed even dehydration), and illustrates a state in which the blood concentration decreases with time.

Further, a curve B illustrates a change in blood concentration when the dehydration speed is initially made high as in FIG. 14(b), the dehydration speed is decreased at a constant rate until a predetermined time is elapsed, and then the dehydration speed is kept constant.

Further, a curve C illustrates a change in blood concentration when the initial dehydration speed illustrated in FIG. 14(c) is increased and then the speed is lowered.

As illustrated in FIG. 14(b) and FIG. 14(c), while the dehydration speed is rather high at the beginning, the decrease in blood concentration may be suppressed by decreasing the dehydration speed at a constant rate or by making dehydration speed constant, dehydration with less decrease of the blood concentration is achieved, and the blood pressure value of the patient can be stabilized and thus a tendency to perform gentle dialysis treatment is achieved.

In order to end a single dialysis treatment within a predetermined time, one dialysis treatment may be completed within a given time by increasing the dehydration amount larger than the normal initial amount, and then regulating the dehydration speed to decrease depending on the individual difference of the patient. In this manner, gentle dialysis treatment for the patient is achieved even the one dialysis treatment is completed within a given time.

Accordingly, dehydration treatment with stabilized blood concentration may be carried out by starting the dehydration amount by selecting one of patterns of A to C according to individual differences of patients when setting the dehydration speed and dehydration amount in advance by calculation, or by increasing or decreasing the dehydration speed in the middle. In that case the change in dehydration speed is preferably based on the individual differences for each patient (for example, a value determined by organizing the relationship such as the relationship between the repeated dehydration operation and the change in blood pressure at that time, or a value determined by using, for example, machine learning algorithm).

Referring again to FIG. 11, Step 1116 is a step in which treatment is performed until the symptom is settled while confirming whether symptoms have been improved.

Step 1114 is a step of determining whether or not the dialysis treatment ends when a predetermined time elapses. When the predetermined time has elapsed (YES), the procedure goes to step 1115 for ending, and if not, the procedure goes back to step 1108, where photographing of the patient by the photographing unit 1009 is continued.

Step 1115 is an end step, which is a step of ending the dialysis treatment, but in that case, patient data in the case of a decrease in blood pressure or the like may be stored in electronic medical records or the like.

Unlike the variation of the blood flow, a phenomenon similar to homeostasis trying to keep constant appears in the variation of the blood pressure value. However, for example, due to the change in peripheral vascular resistance value or the like, the operation state with constant blood pressure against decrease in blood flow may be achieved. In addition, the operating condition with constant blood pressure may appear by facial expressions, colors, and body movements depending on the person. Therefore, when a decrease based on the individual difference, abnormal decrease in the blood pressure value is prevented and a gentle dialysis treatment is achieved by performing an operation for stabilizing the blood pressure even if the blood value is not changed.

Next, an operation of the example of present invention shown in FIG. 11 will be described.

When starting this example, facial expressions, face and body movements are photographed and stored in advance before the dialysis treatment in order to obtain expressions and body movements of at low blood pressure based on the individual difference of the patient, for example, at low blood pressure (1101).

The facial expression and body movement of the patient are photographed in advance and recorded (1102).

The photographing may be a photographing camera having a resolution of about smartphone, WEB camera or the like, or may be a camera with higher resolution. Furthermore, one or a plurality of cameras capable of taking 3D images may be used.

The subject to be imaged is, for example, symptoms occurring at low blood pressure, including the patient's condition at the time of occurrence of yawning, nausea, headache, cold sweat, palpitations, and tinnitus.

Specifically, for example, a state yawning or before yawning, a shape yawn specific to the patient and the like are photographed and stored as digital images, as moving images, or as still images via a computer via a computer in mass storage media such as a hard disk and a USB memory.

Since the timing at which yawning comes out is unspecified and patient's habits are present, it is also possible to use a state obtained by photographing a patient during dialysis treatment, for example.

In addition, the patient may be made create a situation similar to a yawn, and this may be photographed it and stored as an image.

Furthermore, since the characteristic of yawn has a common characteristic point on the digital image such as opening the mouth widely for a long time, it may be recognized to be yawning from the face change by characteristic point extraction without recording in advance.

Further, temperature data of the patient is stored (1103). In addition to measuring the body temperature data and deep body temperature data in the thermometer and the deep thermometer as the temperature data, photograph is taken of the face, hands, feet, fingertips, neck, etc. in the thermographical photographing camera 1009a shown in FIG. 10.

It is preferable to measure the imaging time from several hours to 24 hours to detect the site where patient specific temperature change etc. appears, and to photograph until patient specific temperature distribution is obtained.

It is preferable that the obtained temperature data is measured and stored in a state in which a unique site is specified and temperature change, temperature, etc. at that site can be specified. In addition to temperature value, as temperature data, temporal change amount of temperature is preferably calculated and stored.

Furthermore, humidity data of patient is stored (1104). For example, humidity data is calculated from the image obtained by photographing the face, limbs and the like by the skin humidity photographing camera 1009b such as a near infrared camera, and the amount of perspiration, its degree, and the like are calculated. However, depending on the image of the thermography camera, humidity may also be measured from temperature change due to heat of vaporization. In addition, temporal change information of color is detected and recorded from an image by a usual camera 1009a.

Further, blood pressure related data of the patient is measured (1105). Blood pressure related data is not only blood pressure value but also peripheral vascular resistance value and blood flow value. It is preferable to measure and record in a state corresponding to each part of the body, and in this case as well, the rate of change per unit time is calculated and recorded.

The blood pressure related data further preferably records the ratio of the amplitude value in the reflected wave time phase and the amplitude value in the ejection wave time phase in the pulse waveform, the other AIx value and its temporal change amount.

It is preferable to measure these values in various cases such as in normal state, during dialysis treatment, at low blood pressure, and the like. Also, as described above, the patient may be asked to express expression and body movement virtually, and this state may be stored in the image.

The above data may be stored in the form of patient expression, body movement, temperature image, humidity image, as well as the characteristic point extracted from the image and may be stored together with the state of the patient.

As the characteristic point, for example, in the case of a face, there are both side portions of the mouth, the vicinity of the upper middle portion of the lips and the like. Yawn may be detected by a change of this characteristic point.

Further, detection and recording of blood pressure variation related data is performed (1106).

This may be achieved by detecting from images photographed in steps from 1102 to 1105, or by making the patient, for example yawn in order to act as if the patient is in a hypotensive condition, and recording this condition.

As described above, the facial expression, body movement, temperature, humidity data of patient are recorded and prepared in advance for comparison.

As mentioned above, for example, using the Intel Perceptual Computing (PerC) SDK and the Creative Intaractive Gesture Camera of manufactured by Intel Corporation, like a facial expression detected even without comparative data, there are cases in which the above data is unnecessary when the movement having a common typical characteristic point or the like is present.

Note that data measured during dialysis treatment and compared with the obtained value include comparison with the obtained value as well as the temporal change amount.

A dialysis treatment is started (1107).

Immediately before the start of dialysis treatment, photographing from step 1108 to 1110 is performed.

The temperature and humidity data of patient obtained in step 1108 are measured over time and the change amount is obtained.

Further, the amount of change over time obtained by comparing images, which is to obtain the face and body movements obtained in step 1109 and the amount of change over time of facial color, for example, is obtained by decomposing the facial color into primary colors of RGB or CYMK. In case that the characteristic point is a tip part of the jaw, this characteristic point is tracked and the change amount is obtained.

In step 1110, a temperature change amount or the like of a specific part, for example, the back of the hand, the palm of the hand, and the fingertip, etc. is photographed as an image by thermography and imaging with a near infrared camera.

Further, in step 1110, blood pressure value, peripheral vascular resistance value or related value, blood flow value or related value is measured over time and its change amount is measured.

In step 1102, compared with the pre-stored face and body movements and the change over time of the facial color, a comparison check is made as to whether or not it matches or approximates hypotensive symptoms (1111).

Then, for example, if it can be confirmed that the yaw occurred plural times, an image close to nausea could be confirmed, and furthermore, for example, if the change amount exceeds a certain threshold value when the change amount of the peripheral vascular resistance related value, the change of the temperature of the fingertip in the thermographic image, and the like are collated. Even if the change in blood pressure value is not seen, if the change amount exceeds a certain threshold or the like, it is determined that there is possibility of decrease in blood pressure (1112), and in step 1113, treatment for blood pressure stabilization (supply of replacement fluid, operation to decrease blood pump rotation speed) is performed. When facial expression, body movement, temperature data, humidity data, etc. are measured, and the symptoms are stabilized (1116), it is confirmed whether the dialysis treatment has ended (1114).

At the end, image data in association with decrease in blood pressure is recorded from the obtained image data in the storage device with an index corresponding to the symptom attached (1115).

INDUSTRIAL APPLICABILITY

Present invention can perform optimized hemodialysis such as suppressing the variation of blood pressure according to individual differences of patients and is used for treatment with reduced risk such as complications related to various heart and blood vessels in hemodialysis.

REFERENCE SIGNS LIST

| | |
|---|---|
| 200 | Blood circuit |
| 201 | Blood removing part |
| 202a | Blood driving means |
| 202b | Regulating means |
| 203 | Blood processing means |
| 204 | Blood returning part |
| 205 | Individual difference information management processing means |
| 206 | Blood flowing means |
| 207 | Dialysate treatment section |
| 207a | Waste reservoir |
| 207b | Wastewater reservoir |

208 Dialysis information acquiring means
209 Input/output storage means
210 Biological information input means

**Claims**

1. A individual difference information management system in dialysis treatment comprising:

   blood related information acquiring means configured to acquire blood flow information and patient biological information in a blood circuit for hemodialysis;
   individual difference information managing means configured to acquire and manage patient individual difference information from the blood related information; and
   optimized dialysis means configured to perform optimized dialysis treatment for patients on the basis of the patient individual difference information.

2. The individual difference information management system according to claim 1, wherein the patient biological information is at least one information selected from a group including peripheral vascular resistance information, blood pressure information, face information, body movement information, temperature information, and humidity information.

3. The individual difference information management system according to claim 1, further including individual difference information managing means configured to acquire and manage patient individual difference information from change amount acquiring means configured to obtain a change amount of information acquired by the blood related information acquiring means and the blood related information and/or the change amount.

4. The individual difference information management system according to claim 1, wherein the optimized dialysis means includes means configured to stabilize a blood pressure value.

5. The individual difference information management system according to claim 1, wherein the blood related information acquiring means includes a pulse wave information detecting unit configured to detect pulse wave information relating to an ejection wave and a reflected wave for acquiring peripheral vascular resistance information.

6. The individual difference information management system according to claim 2, wherein the face information includes a color, an expression, a movement, and a deformation of the face, and the body movement information includes movements of a body of a hand, a leg, a neck, a waist, and the like.

7. The individual difference information management system according to claim 1, comprising:

   blood flow information acquiring means configured to acquire blood flow information in a hollow fiber filter of a dialysis machine in a blood circuit for hemodialysis;
   individual difference information managing means configured to compare the blood flow information acquired from the blood flow information acquiring means and a blood flow amount set based on a driving amount of a blood driving section of the blood circuit and acquire individual difference information for regulating the driving amount of the blood driving section; and
   optimized dialysis means configured to regulate the driving amount of the driving section for performing optimal dialysis based on the individual difference information acquired by the individual difference information managing means.

8. The individual difference information management system according to claim 7, wherein the blood flow information acquiring means includes a plurality of laser blood flow meter sensors, wherein blood flow information in the hollow fiber filter of the dialysis machine is acquired by providing the sensors at predetermined positions on a surface of the dialysis machine.

9. The individual difference information management system according to claim 1, wherein the optimized dialysis means comprises: blood driving amount regulating means configured to regulate a blood driving amount of the blood circuit for hemodialysis; a second regulating section configured to supply a concentration regulating solution for regulating a blood concentration in the blood circuit for hemodialysis; and dialysate circulation amount regulating means configured to regulate a circulation amount of dialysate.

**10.** The individual difference information management system according to claim 1 or 7, wherein the individual difference information managing means calculates a dehydration speed and plasma refilling rate (PRR) data, and outputs an optimization signal configured to regulate a driving amount of the blood pump so as to obtain the blood flow information in such a manner that a difference between the dehydration speed and PRR data falls within a predetermined range.

**11.** The individual difference information management system according to claim 1 or 7, wherein, using singular data detecting means configured to detect a singular data in a change over time from the blood flow information acquiring means, the individual difference information managing means acquires a blood pressure change predicting data and an individual difference date from a singular value and a change value of the singular data detecting means.

**12.** The individual difference information management system according to claim 1 or 7, comprising: blood pressure detection means configured to detect a blood pressure consecutively or intermittently before and after the dialysis treatment; body temperature detecting means configured to detect a body temperature consecutively or intermittently before and after the dialysis treatment; humidity detection means configured to detect a moisture of a skin consecutively or intermittently before and after the dialysis treatment; blood flow detecting means configured to detect a blood flow consecutively or intermittently before and after the dialysis treatment; and blood pressure stabilizing means configured to stabilize the blood pressure based on one or a plurality of items of information acquired from the blood pressure detecting means, the body temperature detecting means, the humidity detection means, and the blood flow detecting means.

**13.** The individual difference information management system according to claim 12, wherein the body temperature detecting means, the humidity detection means, and the blood flow detecting means are non-contact types with respect to a biological body.

## FIG. 1

ELIMINATION OF SUFFERING SUCH AS PAIN ELIMINATION OF GANGRENE OF DISTAL PORTION OF EXTREMITIES DECREASE OF RISK OF CARDIOVASCULAR COMPLICATION GOOD LIFE PROGNOSIS GENTLE TREATMENT WITH LESS BLOOD PRESSURE VARIATION

EP 3 511 033 A1

EP 3 511 033 A1

# FIG. 2

46

EP 3 511 033 A1

FIG. 3

47

# FIG. 4

(a)

(b)

□— UPPER HOLLOW FIBER (Pu)    △— LOWER HOLLOW FIBER (Pd)    ●— BLOOD FLOW RATE OF PUMP (Qb)

# FIG. 5

START

501 — DIALYSIS FOR FIRST TIME?

No →

Yes ↓

502 — DIALYSIS TREATMENT

503 — DETECT INDIVIDUAL DIFFERENT INFORMATION

504 — CREATE DIALYSIS SETTING DATA FOR EACH INDIVIDUAL DIFFERENCE

505 — REGISTER FOR EACH INDIVIDUAL DIFFERENCE INTO DATABASE

506 — EXPLAIN DIALYSIS TREATMENT SUITABLE FOR PATIENT

507 — PATIENT REQUESTED FOR TREATMENT?

No

Yes ↓

508 — ADJUST DATABASE

END

509 — CALL OUT DATA OF PATIENT FROM DATABASE

510 — DIALYSIS TREATMENT

511 — COMPARE INDIVIDUAL DIFFERENT INFORMATION

512 — ADJUSTMENT BASED ON INDIVIDUAL DIFFERENCE INFORMATION?

No

Yes ↓

513 — ADJUST DATABASE

# FIG. 6

## (a)

## (b)

# FIG. 7

START

COLLECT BIOLOGICAL INFORMATION DATA DURING LIFETIME — 701

COLLATE WITH PATIENT DATA DATABASE (TREATED PATIENT) — 702

PATIENT DATA HAVING SIMILAR CHARACTERISTIC? — 703
No → / Yes

SET ALLOWABLE RANGE OF CORRESPONDING GROUP — 704

REGISTER IN CORRESPONDING GROUP — 709

COLLATE WITH PATIENT DATABASE (OTHER MEDICAL INSTITUTION) — 705

PATIENT DATA WITH SIMILAR CHARACTERISTICS? — 706
Yes / No

SET ALLOWABLE RANGE OF PATIENT DATA — 707

REGISTER AS SPECIFIC PATIENT DATA — 708

DIALYSIS THERAPY — 710

MEASURE RESPECTIVE DATA AT THE TIME OF DIALYSIS THERAPY — 711

COMPARE INDIVIDUAL DIFFERENCE RANGE OF PATIENT WITH RESPECTIVE DATA — 712

WITHIN ALLOWABLE RANGE? — 713
No → Yes

ADJUST DIALYSIS THERAPY — 714

RECORD PATIENT DATA — 715

STABLE LEVEL VARIED? — 716
Yes → No

ADJUST THERAPY AND ALLOWABLE RANGE — 717

RECORD PATIENT DATA — 718

DIALYSIS THERAPY COMPLETED? — 719
No / Yes

END

EP 3 511 033 A1

# FIG. 8

801 — TEMPERATURE/HUMIDITY DETECTOR → TEMPERATURE/HUMIDITY CHANGE AMOUNT DETECTION UNIT

812

802 — PULSE WAVE DETECTION UNIT → PULSE WAVE VALUE CHANGE AMOUNT DETECTING SECTION

813

803 — BLOOD FLOW/ BLOOD PRESSURE DETECTING SECTION → BLOOD FLOW/BLOOD PRESSURE CHANGE AMOUNT DETECTING SECTION

814

804 — IMAGE CHARACTERISTIC PORTION DETECTION UNIT → IMAGE CHARACTERISTIC PORTION CHANGE AMOUNT DETECTING SECTION

815

MIXING MEANS → MODULATING MEANS → TRANSMISSION MEANS

805          806          811          807

808 — RECEPTION MEANS → DEMODULATING MEANS

809

SEPARATING MEANS → PROCESSING MEANS → OUTPUT MEANS ○

810          816          817          800

52

# FIG. 9

# FIG. 10

# FIG. 11

START —1101

PHOTOGRAPH FACE AND BODY MOVEMENT OF PATIENT —1102

PHOTOGRAPH TEMPERATURE DATA OF PATIENT —1103

COLLECT HUMIDITY DATA OF PATIENT —1104

COLLECT BLOOD PRESSURE-RELATED DATA OF PATIENT —1105

DETECT AND RECORD BLOOD PRESSURE VARIATION RELATED DATA —1106

START DIALYSIS TREATMENT —1107

MEASURE TEMPERATURE AND HUMIDITY OF PATIENT —1108

PHOTOGRAPH FACE AND BODY MOVEMENT OF PATIENT —1109

COLLECT BLOOD PRESSURE RELATED DATA —1110

COMPARISON WITH DATA MEASURED IN ADVANCE —1111

SIGN OF BLOOD PRESSURE VARIATION ? —1112
Yes
No

PERFORM BLOOD PRESSURE STABILIZATION TREATMENT —1113

END OF TREATMENT —1116
Yes
No

END OF DIALYSIS TREATMENT —1114
No
Yes

END —1115

FIG. 12

FIG. 13

# FIG. 14

(a)

(b)

(c)

(d)

| INTERNATIONAL SEARCH REPORT | International application No. |
|---|---|
| | PCT/JP2017/032551 |

**A. CLASSIFICATION OF SUBJECT MATTER**
Int.Cl. A61M1/14 (2006. 01)i, A61M1/16 (2006. 01)i, A61M1/36 (2006. 01)i

According to International Patent Classification (IPC) or to both national classification and IPC

**B. FIELDS SEARCHED**

Minimum documentation searched (classification system followed by classification symbols)
Int.Cl. A61M1/14, A61M1/16, A61M1/34, A61M1/36

Documentation searched other than minimum documentation to the extent that such documents are included in the fields searched
| | |
|---|---|
| Japanese Published Examined Utility Model Applications | 1922–1996 |
| Japanese Published Unexamined Utility Model Applications | 1971–2017 |
| Japanese Examined Utility Model Registrations | 1996–2017 |
| Japanese Registered Utility Model Specifications | 1994–2017 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 58-155864 A (TOYOTA CENTRAL RESEARCH AND DEVELOPMENT LABORATORIES, INC.) 16 September 1983, page 2, upper right column, line 12 to page 8, upper right column, line 13, fig. 1-8 & US 4469593 A (column 4, line 58 to column 12, line 33, fig. 1-8) & EP 89003 A2 | 1-3<br>2, 4-6, 9-13<br>7-8 |
| Y<br>A | JP 2016-112277 A (SEIKO EPSON CORP.) 23 June 2016, paragraphs [0022]-[0062], fig. 1-7 & EP 3033990 A1 (paragraphs [0022]-[0065], fig. 1-7) & US 2016/0174854 A1 & CN 105708434 A | 2, 5, 12-13<br>7-8 |

☒ Further documents are listed in the continuation of Box C.  ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 17 November 2017 (17.11.2017) | 28 November 2017 (28.11.2017) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

| International application No. |
| --- |
| PCT/JP2017/032551 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y<br>A | JP 2008-284229 A (TAKANO, Hideo) 27 November 2008, paragraphs [0008]-[0072], fig. 1-4 (Family: none) | 2, 6, 12-13<br>7-8 |
| Y<br>A | JP 2014-530672 A (FRESENIUS MEDICAL CARE DEUTSCHLAND GMBH) 20 November 2014, paragraphs [0050]-[0095], fig. 1-4 & US 2013/0267883 A1 (paragraphs [0054]-[0099], fig. 1-4) & WO 2013/045448 A1 & CN 103917258 A & KR 10-2014-0067150 A & EP 2760499 A1 | 2, 6, 12-13<br>7-8 |
| Y<br>A | JP 2014-512880 A (FRESENIUS MEDICAL CARE DEUTSCHLAND GMBH) 29 May 2014, paragraphs [0050]-[0067], fig. 1-2 & US 2012/0212455 A1 (paragraphs [0051]-[0068], fig. 1-2) & WO 2012/110251 A1 & CN 103354751 A & KR 10-2014-0024858 A & EP 2675495 A1 | 2, 12-13<br>7-8 |
| Y<br>A | JP 2014-113423 A (GOLDWELL LTD.) 26 June 2014, paragraphs [0015]-[0027], fig. 1-7 (Family: none) | 2, 4, 12-13<br>7-8 |
| Y<br>A | WO 2013/005320 A1 (PIONEER CORP.) 10 January 2013, paragraphs [0011]-[0125], fig. 1-11 & EP 2730302 A1 (paragraphs [0010]-[0126], fig. 1-11) | 4, 11-13<br>7-8 |
| Y<br>A | JP 2014-518687 A (MEDTRONIC, INC.) 07 August 2014, paragraphs [0015]-[0100], fig. 1-20 & WO 2013/101292 A2 (paragraphs [0023]-[00106], fig. 1-20) | 4, 11-13<br>7-8 |
| Y<br>A | JP 2015-047349 A (NIKKISO CO., LTD.) 16 March 2015, claim 4 & EP 3042672 A1 (claim 4) & US 2016/0175508 A1 & CN 105473170 A & WO 2015/030233 A1 | 9<br>7-8 |
| Y<br>A | JP 2004-049493 A (JMS CO., LTD.) 19 February 2004, claim 11 paragraph [0038] (Family: none) | 10<br>7-8 |
| A | US 5928180 A (NIKOLAI M. Krivitski) 27 July 1999, whole document (Family: none) | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008023269 A
- JP 2004357784 A
- JP 2012526569 A
- JP 2014532475 A
- JP 2015029882 A
- JP 2005334527 A

- JP 2003190277 A
- JP 2006112277 A
- JP 2008 A
- JP 534214 A
- JP 2008534214 A

### Non-patent literature cited in the description

- *Yu Dialysis Clinic- Material of 7th Study Session of Patients, http://yutoseki.jp/swfu/d/study7.pdf*
- **SUGURU OBUNAI ; SATOAKI MATOBA.** A case of finger gangrene that could be rescued by multidisciplinary treatment: Subject Number. *Annual Meeting of Japanese Society for Dialysis Therapy: Archived Abstracts of the General Assembly, http:// /www.my-schedule.jp/jsdt_archive/detail.php?sess_id=17048*
- **KEI EGUCHI.** Invention of a New Actual Blood Flow Amount Measurement Method (CRIT-2 point method). *Journal of Japanese Society for Dialysis Therapy,* 2008, vol. 41, 127-131
- **KAZUYUKI SUZUKI et al.** Dialysis Conditions/Dialysis Volume and Life Prognosis. *Journal of Japanese Society for Dialysis Therapy,* 2012, vol. 45 (2), 143-155
- **KAZUYUKI SUZUKI et al.** Hemodialysis Condition /Dialysis Volume and Life Prognosis. *Journal of Japanese Society for Dialysis Therapy,* 2010, vol. 43 (7), 551-559
- **SUSUMU OOKAWARA et al.** Mathematical Analysis of the Change in Circulating Blood Volume during Hemodialysis by Continuous Hematocrit Measurement. *Journal of Japanese Society for Dialysis Therapy,* 1998, vol. 31 (6), 1001-1005

- **SUSUMU OOKAWARA.** Mathematical Analysis of the Change in Circulating Blood Volume during Hemodialysis by Continuous Hematocrit Measurement. *Journal of Japanese Society for Dialysis Therapy,* 1998, vol. 31 (6), 1001-1005
- *Yu Dialysis Clinic-Material of 7th Study Session of Patients, http://yu-toseki.jp/swfu/d/study7.pdf*
- the extraction and application of physiological information from the color change of the face. the 298th meeting of the Society of Instrument and Control Engineers. 18 November 2015
- **MARIKO EGAWA.** *Med Imag Tech,* January 2012, vol. 30 (1
- **YAMANAKA et al.** *Journal of Japanese Society for Dialysis Therapy,* 2002, vol. 35 (2), 97-107
- **KIHARA et al.** *Journal of Japanese Society for Dialysis Therapy,* 2007, vol. 40 (3), 241-245
- **SHIBATA et al.** *Journal of Japanese Society for Dialysis Therapy,* 2002, vol. 35 (10), 1337-1342
- *NTT Technical Journal,* 2005, vol. 11, 24-27
- Extraction and Application of Physiological Information from Face Color Change. the 298th meeting of the Society of Instrument and Control Engineers. 18 November 2015
- **MUTSUKO NAKAMURA et al.** Moisture retention effect of cosmetics as seen in near infrared spectroscopic images. *Optics,* 2010, vol. 39 (11), 529-533